# EUROPEAN PATENT APPLICATION

(11) **EP 1 642 980 A1**
(43) Date of publication of application: **05.04.2006**
(21) Application number: 05076460.4
(22) Date of filing: 19.06.1996
(51) Int. Cl.: C12N 15/62, C07K 1/107, C12N 9/12

(54) **Modified proteins and methods of their production**

(30) Priority: 28.06.1995 US 496297; 29.12.1995 US 580555
(62) Divisional of application: 96922498.9
(71) Applicant: NEW ENGLAND BIOLABS, INC., Beverly Massachusetts 01915 (US)
(72) Inventor: Comb, Donald G., Manchester, MA 01944 (US); Hodges, Robert A., Norcross, GA 30092 (US); Perler, Francine B., Brookline, MA 02146 (US); Noren, Christopher J., Boxford, MA 01921 (US); Jack, William E., Wenham, MA 01984 (US); Chong, Shaorong S.C., Somerville, MA 02144 (US); Xu, Ming-Qun, Hamilton, MA 01982 (US)
(74) Representative: Froud, Clive

(57) **Abstract**

The present invention is directed to modified proteins and methods of their production. The modified proteins comprise a controllable intervening protein sequence (CIVPS) inserted into a target protein, the CIVPS being capable of excision from or cleavage of the modified protein under predetermined conditions in *cis* or in *trans*, i.e., increase in temperature, exposure to light, unblocking of amino acid residues by dephosphorylation, treatment with chemical reagents or deglycosylation. If desired, the modified protein can be subjected to these conditions. The CIVPS may also be inserted into a region that substantially inactivates target protein activity.

## Description

### CROSS-REFERENCE TO RELATED APPLICATION

This Application is a Continuation-In-Part Application of co-pending Application Serial No. 08/496,247, Filed June 28, 1995 which is a Continuation-In-Part Application of co-pending Application Serial No. 08/146,885, filed November 3, 1993 which is a Continuation-In-Part of co-pending Application Serial No. 08/004,139, filed December 9, 1992.

### BACKGROUND OF THE INVENTION

The present invention is directed to modified proteins and methods of producing the same. More specifically, the modified protein of the present invention comprises a target protein and a controllable intervening protein sequence (CIVPS), the CIVPS being capable of excision or cleavage under predetermined conditions.

Production of mature proteins involves the flow of information from DNA to RNA to protein. Precise excision of DNA and RNA elements which interrupt that information has been previously described (M. Belfort, *Annu. Rev. Genet.* 24:363 (1990); T.R. Cech, *Annu. Rev. Biochem.* 59:543 (1990); Hunter et al., *Genes Dev.* 3:2101 (1989)). More recently, evidence for the precise excision of intervening protein sequences has also been described for the *TFPl* allele from *Saccharomyces cerevisiae* (Hirata et al., *J. Biol. Chem. 265:6726* (1990); Kane et al., *Science* 250:651 (1990)) and the *rec A* gene from *Mycobacterium tuberculosis* (Davis et al., *J. Bact.* 173:5653 (1991); Davis et al., Cell 71:1 (1992)). Each contains internal in-frame peptide segments which must be removed to produce the mature protein. Expression of Tfp1 and Rec A each results in two peptides: one representing the intervening protein sequence (IVPS) and the other the ligated product of the external protein sequences (EPS). This post-translational processing event has been termed "protein splicing". Similarly, the Vent DNA polymerase gene from the hyperthermophilic archaea *Thermococcus litoralis* contains two in-frame IVPSs (Perler, et al., *PNAS* 89:5577 (1992)).

A major impediment to the development of methods of using IVPSs or protein splicing in other than research applications has been the inability to control the activity of the IVPS and thus the splicing event.

Thus, it would be desirable to have a method which provides a ready means to modify a target protein using an IVPS, particularly where the activity of the IVPS is controllable. It would also be desirable to have a method which can specifically modify target proteins such that their activity is substantially inactivated. It would be desirable to have a method which can be used to restore the activity of an inactivated modified protein.

### SUMMARY OF THE INVENTION

The present invention relates to modified proteins comprising an IVPS and a target protein, the IVPS being capable of excision by protein splicing, or cleavage in the absence of splicing, under predetermined conditions in either *cis* or in *trans.* Such predetermined conditions depend on the IVPS used and can include, for example, increase in temperature, changes in pH conditions, exposure to light, dephosphorylation, or deglycosylation of amino acid residues or exposure to chemical reagents which induce cleavage/splicing. The IVPS may be joined with the target protein either by inserting the IVPS into the target protein or fusing the IVPS with the target protein at either the amino or carboxy terminal end of the target protein. These IVPS, referred to as controllable intervening protein sequences (CIVPS), are therefore useful in controlling the splicing or cleavage reaction. The present invention further relates to methods for producing, selecting and testing CIVPSs.

In one preferred embodiment, a DNA sequence encoding a CIVPS is inserted into, or joined with, a DNA sequence encoding a target protein such that both coding sequences form a continuous open reading frame. Thereafter, expression of this fusion DNA is utilized to produce the modified target protein. In another embodiment, the modified protein so produced is subjected to predetermined conditions under which the CIVPS will be excised or cleaved. In certain embodiments, the CIVPS is inserted into a region of the target protein which renders the target protein substantially inactive and excision of the CIVPS restores the activity of the target protein.

Preferred CIVPSs include CIVPS1 and 2 obtainable from *T. litoralis* (also sometimes referred to as Vent IVPS 1 and 2 or IVS1 and 2) and CIVPS 3 obtainable from *Pyrococcus* sp.(also sometimes referred to as Deep Vent IVPS1 or IVS1). These CIVPSs are capable of excision, i.e., removal via protein splicing, from modified proteins upon an increase in temperature. Other preferred CIVPSs include those obtainable from yeast such as *Saccharomyces cerevisiae*.

In accordance with the present invention, it has also been found that certain CIVPS amino acid residues and at least the first downstream amino acid residue modulate the splicing reaction and that modification of these residues decreases or stops the splicing reaction. These residues have been shown to be conserved in other IVPSs. Modification of such residues can be used to convert a IVPS to a CIVPS.

In accordance with the present invention, it has been found that in certain situations, the complete splicing reaction is not necessary or desirable. In such situations, the CIVPS can be modified to allow cleavage in the absence of splicing, thus allowing for controlled separation or cleavage of the CIVPS from the target protein.

The potential uses for the modified proteins and CIVPSs of the present invention are manifold. These include, for example, control of a target protein's enzymatic activity, purification of modified proteins using antibodies specific to the CIVPS by affinity chromatography and production of proteins that are toxic to host cells.

The CIVPSs of the present invention may further be used in a method of protein purification in which a modified protein comprising a target protein fused to a CIVPS is produced. If desired, a three-part fusion can be produced in which the CIVPS is between the target protein and a protein having affinity for a substrate (binding protein), e.g., MBP. The modified protein is then contacted with a substrate to which the CIVPS or binding protein has specific affinity, e.g., using affinity chromatography. The highly purified target protein can be liberated from the column by subjecting the CIVPS to predetermined conditions under which cleavage, for example, between the CIVPS and the target protein is initiated. Alternatively, the fusion protein can be purified as above and then the target protein released from the fusion by subjecting the CIVPS to predetermined conditions.

### BRIEF DESCRIPTION OF THE DRAWINGS

Figure 1 depicts the amino acid sequence (SEQ ID NO:30, SEQ ID NO:31, SEQ ID NO:32, SEO ID NO:33, SEQ ID NO:34, SEQ ID NO:36, SEQ ID NO:37, SEQ ID NO:38 and SEQ ID NO:39) of proposed protein splice junctions. Amino-terminal (top) and carboxy-terminal (bottom) splice junctions are shown with splice sites indicated by arrows and conserved or similar amino acids boxed.
Figure 2 illustrates insertion of IVPS into the EcoRV site of the ß-galactasidase gene. PCR products of either Deep Vent IVPS1 (CIVPS3) or Vent IVPS2 (CIVPS2) are ligated to *Eco*RV digested pAHO5 between the Asp and Ile residues of ß-galactosidase to produce a modified ß-galactosidase product.
Figure 3A and 3B are graphs showing that splicing of modified ß-galactosidase yields active ß-galactosidase. Incubation of crude extracts from hosts expressing the indicated IVPS-ß-galactosidase fusion proteins at 42°C yields an increase in enzyme activity with time, whereas incubation at 42°C with the host alone (RR1) or an unmodified ß-galactosidase construct (pAHO5) shows no increase in enzyme activity.

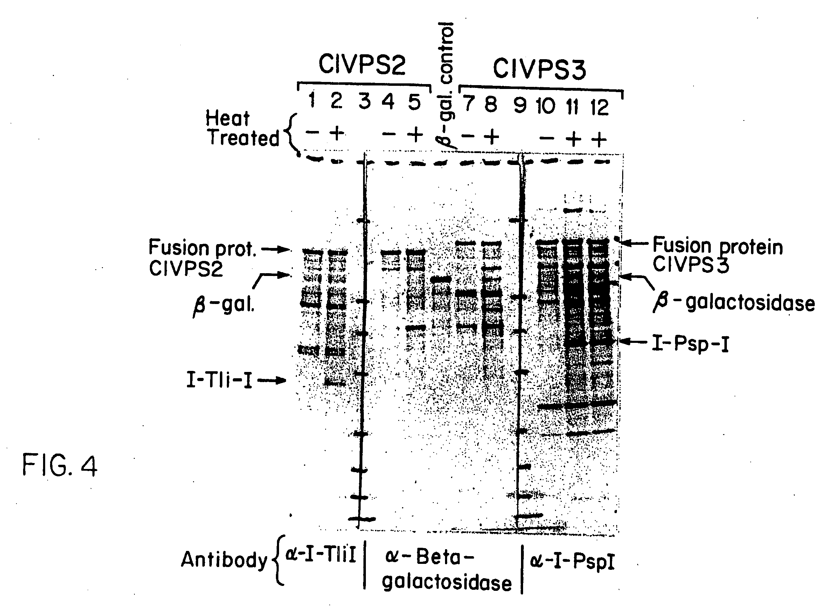

Figure 4 is a western blot showing the results of temperature controlled protein splicing experiments. CIVPS2 and CIVPS3 were cloned into the *Eco*RV site of ß-galactosidase. Western blot examination of cell extracts with sera directed against ß-galactosidase or the CIVPS protein (I-*Tli*l and I-*Psp*I, respectively) detects modified ß-galactosidase fusion protein (Lanes 1,4,7,10). Treatment of extracts at 42°C (Lanes 2,5,8,11) or 50°C (Lane 12) for 6 hours results in splicing and the production of free CIVPS proteins and unmodified ß-galactosidase (except for retained serine or threonine residue, see text example 2 & 3). Unmodified ß-galactosidase from pAHO5 is in lane 6. Lanes 3 & 9 contain size markers.
Figure 5 shows by western blot examination of cell extracts with sera directed against ß-agarase, the detection of modified ß-agarase fusion protein. Lanes 1 & 4: size markers; Lanes 2 & 5: ß-agarase standard; lane 3: CIVPS2-ß-agarase fusion; lane 6: CIVPS3-ß-agarase fusion.
Figure 6 illustrates insertion of IVPS2 (CIVPS2) into the ß-galactasidase gene by creation of new restriction sites (*Bsp*EI and *Spe*l) within the IVPS by silent mutations.
Figure 7 illustrates insertion of either Deep Vent IVPS1 (CIVPS3) or Vent IVPS2 (CIVPS2) into the ß-galactasidase gene by creation of new restriction sites (*Xba*l and *Sal*I) by silent mutations within the target gene.
Figure 8 is a plasmid map of pANG5.
Figure 9 is an autoradiogram of SDS-PAGE showing suppressor tRNA-mediated incorporation of a chemically blocked serine at the upstream junction of CIVPS2.
Figure 10 is an autoradiogram of SDS-PAGE showing the splicing reaction of CIVPS2 initiated by visible light irradiation of a chemically blocked precursor protein.

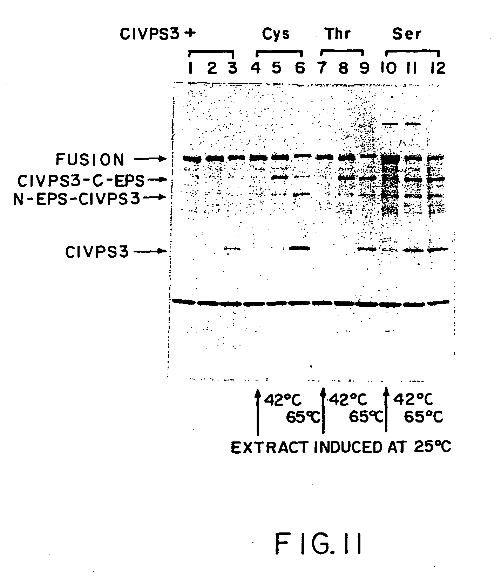

Figure 11 is a gel showing temperature controlled protein splicing and cleavage. Deep Vent IVPS1 (CIVPS3) cassettes were cloned into the *Eco*RV site of ß-galactosidase. Western blot analysis was used to examine cell extracts of pDV7 (CVPS3 cassette, lanes 1-3), pDVC302 (CIVPS3/Cys cassette, lanes 4-6), pDVT321 (CIVPS3/Thr cassette, lanes 7-9) and pDVS712 (CIVPS3/Ser cassette, lanes 10-12). Antibody directed against the CIVPS3 protein (I-*Psp*I) (NEB) detects fusion proteins and cleavage products including free CIVPS3, N-EPS-CIVPS3 and CIVPS3-C-EPS (from cleavage at one of the splice junctions). The untreated extracts were in lanes 1, 4, 7, and 10. Treatment of extracts at 42°C (lanes 2, 5, 8, and 11) or 65°C (lanes 3, 6, 9, and 12) for 2 hours results in increased splicing and/or cleavage activity at different efficiency.

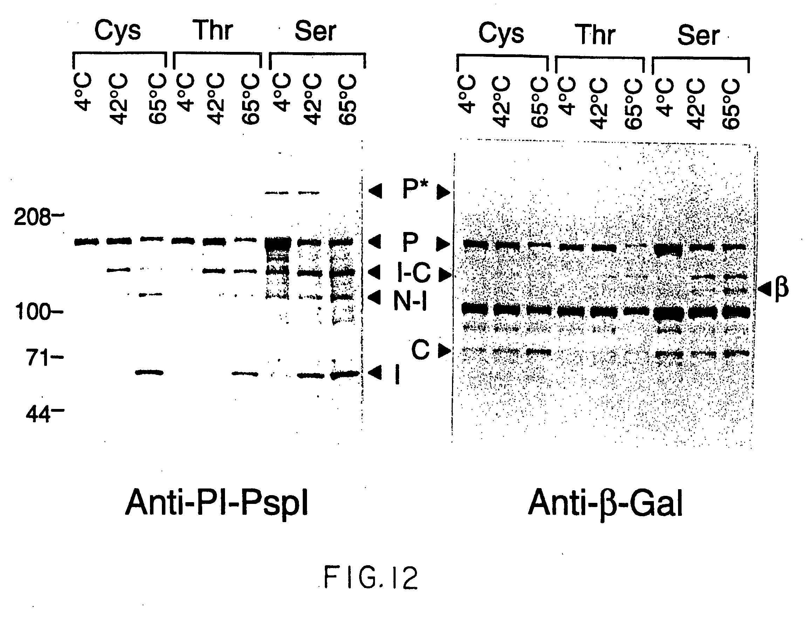

Figure 12 is a Western blot showing temperature controlled protein splicing and cleavage. Western blot analysis using antibody directed against I-PspI and ß-galactosidase (C-EPS domain) (Promega) were used to examine fusion constructs pDVC302 (lanes 1-3), pDVT321 (lanes 4-6) and pDVS712 (lanes 7-9). Treatment of extracts at 42°C (lanes 2, 5, and 8) or 65°C (lanes 3, 6, and 9) for 2 hours results in splicing (in pDVS712) or cleavage. Protein splicing in pDVS712 extract produced free CIVPS3 protein, I-PspI and unmodified ß-galactosidase (except for retained serine). Lane 1 contains size markers.

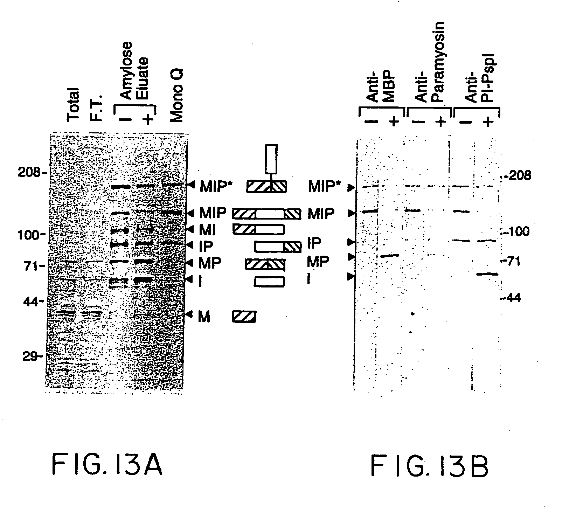

Figure 13A and 13B show the purification of MIP precursor on amylose and MonoQ columns examined by Coomassie blue staining and immunoblot. The diagram between Parts A and B represents the proposed structure of each band, including the branched molecule MIP*. The black boxes represent the MBP domain, the white boxes the IVPS doman and the gray boxes the paramyosin ΔSal domain. The pluses (+) indicate that the sample was heat treated at 37°C for 120 min., minuses (-) indicate that the sample was not heat treated. Part A: Coomassie blue stained gel. Total, crude supernatants from MIP cultures. F.T., amylose resin flow through. Amylose eluate (-), amylose resin purified MIP preparations. Amylose eluate (+), the amylose eluate in lane 4 was treated at 37°C for 120 min. to induce splicing. MonoQ, MonoQ purified sample. After chromatography on MonoQ, recovery of MBP-CIVPS3 (MI) was variable and generally low. Symbols are as follows: MIP*, 180 kDa apparent molecular mass branched molecule; MIP, 132 kDa precursor; single splice junction cleavage products (MI, MBP-CIVPS3; IP, CIVPS3-paramyosin ΔSal; M, MBP); and spliced products (MP, MBP-paramyosin ΔSal and I, CIVPS3 = PI-*Psp*I). Part B: Immunoblots. The MonoQ sample from Part A was heat treated as above and electrophoresed in triplicate. MIP-related proteins were identified by immune reactivity with anti-MBP sera, anti-paramyosin sera and anti-P(-*Psp*I (anti-CIVPS3) sera.
Figure 14 illustrates the replaceable splice junction cassettes in MIP21 fusion. pMIP21 contains two unique restriction sites flanking each splice junction. Splice junctions are indicated by arrows. Amino acid residues around the splice junctions are shown. Splice junctions can be changed by replacing either the amino terminal *Xho*I*-Kpn*I cassette or the carboxyl terminal *Bam*HI-*Stu*I cassette with another DNA cassette.
Figure 15A and 15B is a gel showing thermal inducible cleavage at a single splice junction from modified MIP fusions. Fusion proteins were purified using amylose resin columns.
Figure 15A shows cleavage at the C-terminal splice junction from MIP23 fusion. Purified fusion protein samples were incubated at 4°C, 37°C, 50°C or 65°C for 1 hour. Products were analyzed by a 4/20% SDS-PAGE followed by Coomassie blue staining. Cleavage of the C-terminal splice junction of the MIP23 fusion protein (MIP) yielded MBP-CIVPS (MI) and paramyosin ΔSal (P).

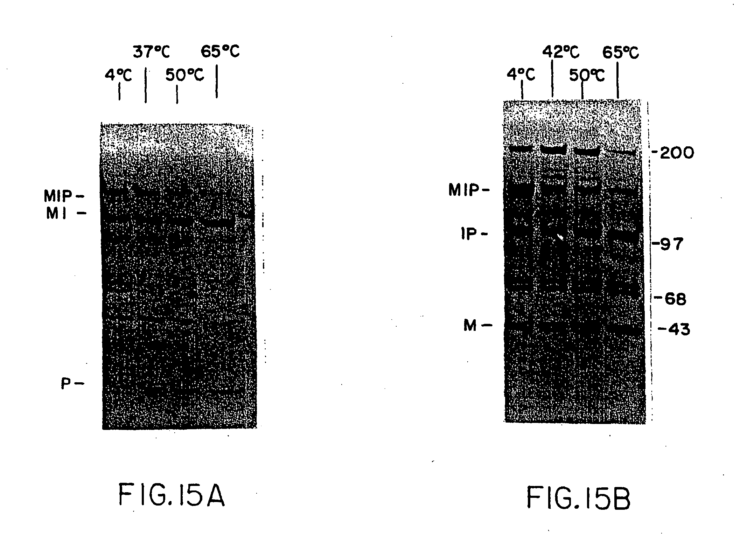

Figure 15B shows cleavage at the N-terminal splice junction from MIP28 fusion. Purified protein samples were incubated at 4°C, 42°C, 50°C or 65°C for 1 hour. Products were analyzed by a 4/20% SDS-PAGE followed by Coomassie blue staining. Cleavage of the N-terminal splice junction of the MIP28 fusion protein (MIP) yielded MBP (M) and CIVPS-paramyosin ΔSal (IP). Size standards ( in kilodaltons) are shown on the left side.

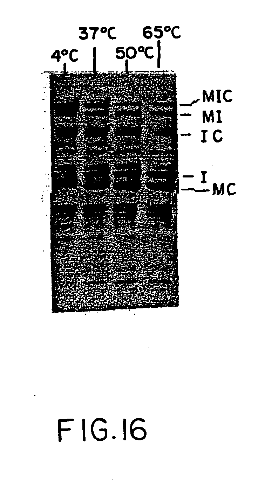

Figure 16 is a gel showing thermal inducible cleavage of MIC fusion. Purified fusion protein samples were incubated at 4°C, 37°C, 50°C or 65°C for 1 hour. Products were analyzed by a 4/20% SDS-PAGE followed by Coomassie blue staining. Incubation of MIC fusion protein (MIC) yielded formation of ligated product, MBP-CBD(MC), and excised product, Deep-Vent IVPS1 (I=I-Psp I). Also, cleavage products, MBP-Deep-Vent IVPS1 (MI) and Deep-Vent IVPS1-CBD(IC), are present in all samples and do not change with this heat treatment.

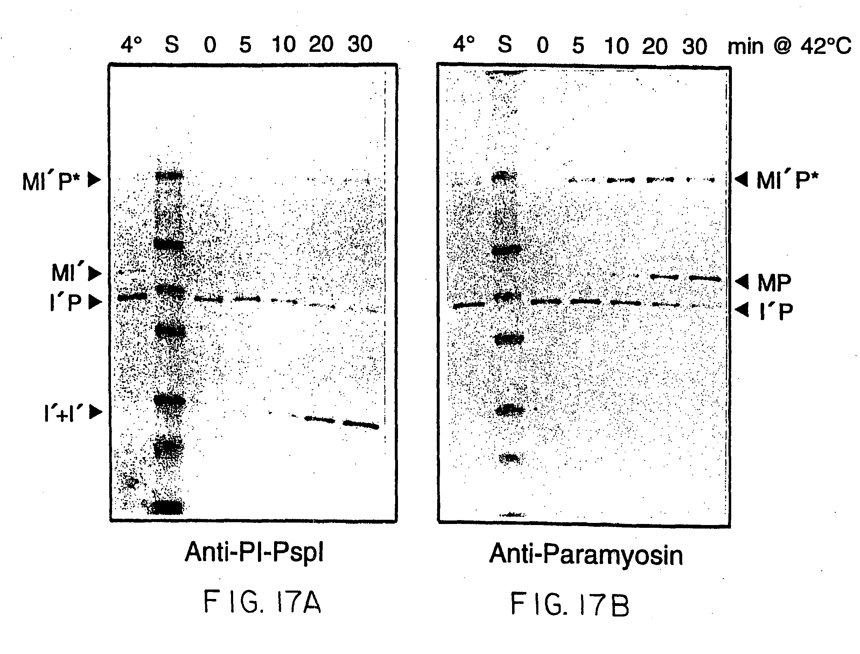

Figure 17A and 17B show the Western blot of a *trans*-splicing reaction with MI' and I'P. I'P and MI' were treated as described in the text to induce *trans*-splicing as observed by the accumulation of MP and I' products. Western blots with either anti-CIVPS3 (Anti-Pi-Pspl) sera or anti-Paramyosin sera were performed as described in the text. Lanes marked '4°' contain control I'P and MI' samples incubated at 4°C. Lanes 3-7 contain cleavage reaction samples after incubation for 0, 5, 10, 20, and 30 minutes at 42°C, respectively. Lane S contains size markers (NEB broad range prestained protein markers).

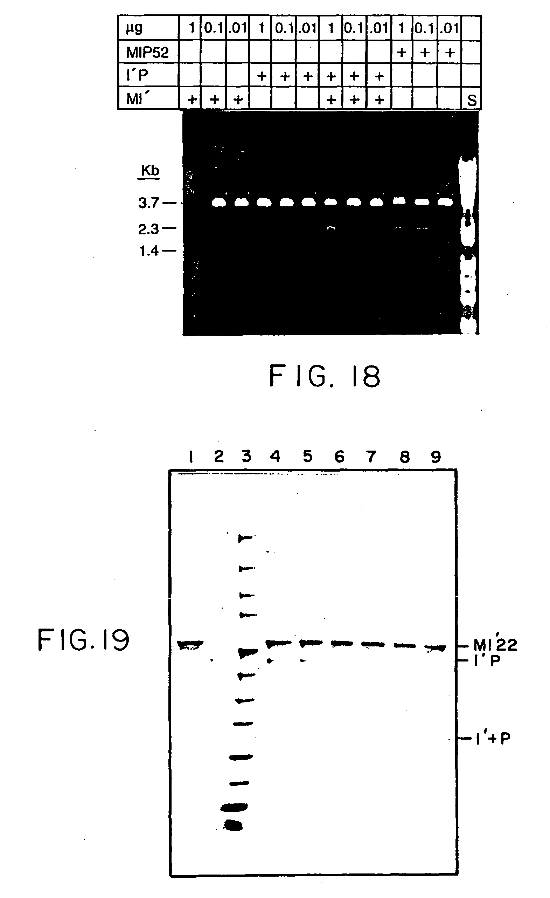

Figure 18 shows that *trans*-splicing re-establishes I-PspI endonuclease activity. Xmnl linearized pAKR7 DNA was digested with 0.01, 0.1 or 1 µg of either MI', I'P, the *trans*-splicing reaction products (indicated by a plus in both the I'P and MI' rows) or *cis*-spliced MIP52. I-PspI activity was only present in MIP52 and the *trans* -spliced mixture. Lane S contains size markers (a mixture of lambda DNA digested with HindIII and PhiX174 DNA digested with HaeIII).
Figure 19 shows the *trans*-cleavage of I'P by MI'22. I'P and MI'22 were treated as described in the text to induce *trans*-cleavage. Lanes 1 and 2 contain the starting samples MI'22 and I'P, respectively. Lane 3 contains size markers (NEB broad range protein markers). Lanes 4-9 contain cleavage reaction samples from 0, 5, 10, 20, 40 and 90 minutes at 42°C, respectively.

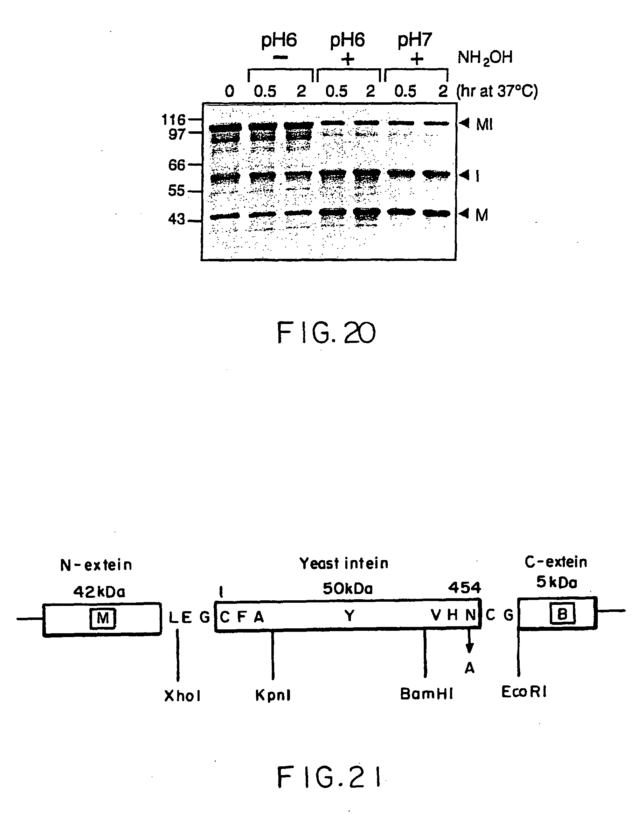

Figure 20 illustrates the chemical activation of cleavage at the N-terminal splice junction from the MI94 fusion containing the Ser1Cys substitution. Purified protein samples were incubated at 37°C with (+) or without (-) 0.25 M hydroxylamine. Products were analyzed by a 4-12% SDS-PAGE followed by Coomassie blue staining. Cleavage at the N-terminal splice junction of the MI94 fusion protein (MI) yielded MBP (M) and CIVPS3 (I). Size standards (in kilodaltons) are shown on the left side.

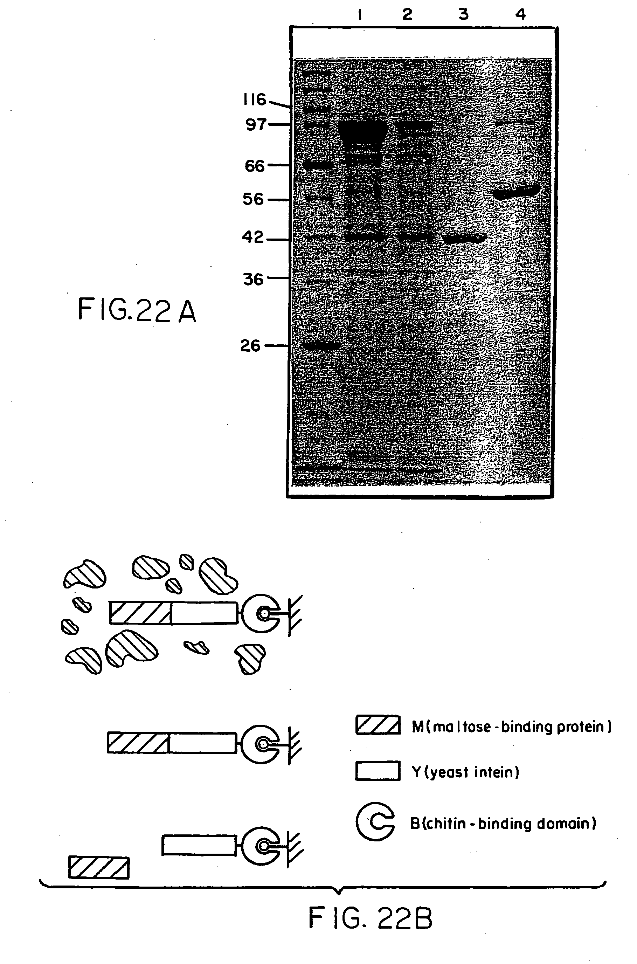

Figure 21 illustrates pMYB129 fusion construct carrying N454A substitution.
Figure 22A and 22B illustrates one-step purification of the target protein (MBP) by chitin. Cleavage is induced by 30 mM DTT at pH 7.6 at 4°C at 16 hours. Size markers (NEB) (on the left); lane 1: cell lysate; lane 2: flow-through lysate; lane 3: DTT-induced cleavage product, MBP (M); lane 4: 6M guanidine wash.

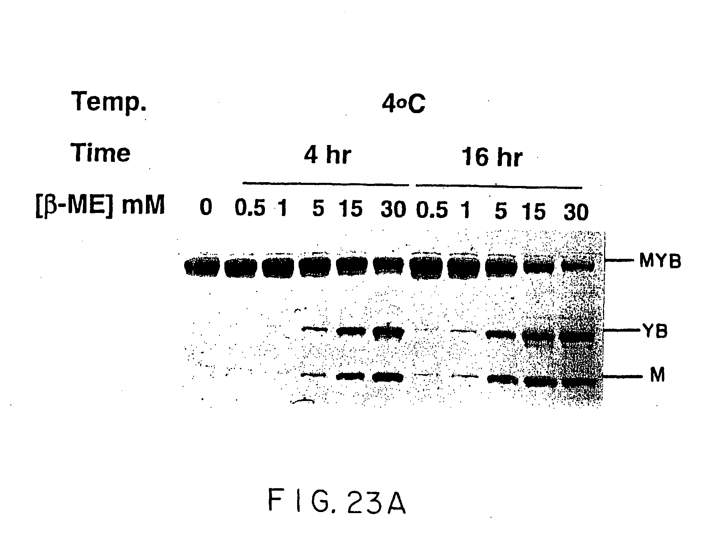

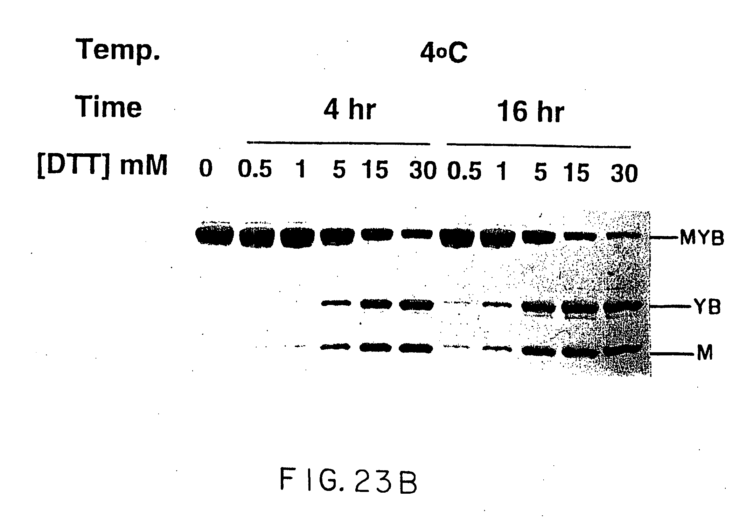

Figure 23A and 23B show activation of cleavage of MYB fusion protein by ß-mercaptoethanol (ß-ME) (Figure 23A) and DTT (Figure 23B).
Figure 24 illustrates the reaction vessel dimensions and set up used in the preparation of chitin beads.

### DETAILED DESCRIPTION OF THE INVENTION

The present invention is directed to modified proteins and methods of their production. The modified proteins comprise a controllable intervening protein sequence (CIVPS) and a target protein, the CIVPS being capable of excision by protein splicing, or cleavage in the absence of splicing, under predetermined conditions, e.g., increase in temperature, changes in pH conditions, unblocking of amino acid residues by photolysis, dephosphorylation, deglycosylation, treatment with chemical reagents or other means. If desired, the modified protein can be subjected to these conditions. The CIVPS may also be inserted into a region that substantially inactivates target protein activity.

Intervening protein sequences (IVPS) are internal in-frame peptide segments found within a precursor protein which are removed or excised via protein splicing to form the native protein. IVPSs have been described in the *TFPI* allele from *Saccharomyces cerevisiae* (Hirata et al., *supra;* Kane et al., *supra)* and *rec A* gene from *Mycobacterium tuberculosis* (Davis et al., *supra* (*1991*); Davis et al., *supra* (1992)). The disclosure of these references are herein incorporated by reference.

CIVPSs of the present invention include any intervening protein sequence in which excision or cleavage can be controlled, either by inherent properties of the native IVPS, such as an increase in temperature, or by modifications made to an IVPS that allow the reaction to be controlled.

The Vent DNA polymerase gene from the hyperthermophilic archaea *Thermococcus litoralis* contains two in-frame IVPSs, IVPS1 (CIVPS1) and IVPS2 (CIVPS2), (Perler, et al. *supra*) that can be deleted at the DNA level without affecting the kinetic and biochemical properties of the expressed polymerase. Correct processing of the Vent DNA polymerase gene containing both IVPSs occurs in the native archaea, *T. litoralis.* In addition, correct processing of expression constructs lacking IVPS1 has been observed in eubacterial *E. coli* (Perler, et al., *supra*), in eukaryotic baculovirus-infected insect cell and *in vitro* transcription/translation systems (Hodges, et al., *Nucleic Acids Research,* 20:6153 (1992)). Furthermore, rabbit reticulocyte and *E. coli* in vitro transcription/translation systems correctly remove IVPS2 sequences to produce the mature polymerase. While not wishing to be bound by theory, it is believed that the Vent and Deep Vent IVPSs are self splicing.

The nucleotide sequence for the Vent DNA polymerase gene is set out in the Sequencing Listing as SEQ ID NO:1. The nucleotide sequence for CIVPS1 is from nucleotide 1773 to 3386. The nucleotide sequence for CIVPS2 is from nucleotide 3534 to 4703. CIVPS1 and CIVPS2 can be obtained from phage NEB 619, which was deposited with the American Type Culture Collection (ATCC) on April 24, 1990 and received ATCC accession number 40795.

A third IVPS (CIVPS3 or DV IVPS1), has been found by the present inventors in the DNA polymerase gene of the thermophilic archaebacteria, *Pyrococcus* species (isolate GB-D). The Pyrococcus DNA polymerase is sometimes referred to as Deep Vent DNA polymerase. The nucleotide sequence of the Deep Vent DNA polymerase is set out in the Sequence Listing as SEQ ID NO: 2. The nucleotide sequence for CIVPS3 is from 1839 to 3449. CIVPS3 can be obtained from plasmid pNEB #720 which was deposited with the ATCC on October 1, 1991 and received ATCC accession number 68723.

In accordance with the present invention, it has been found that the above CIVPS1, CIVPS2 and CIVPS3 are capable of excision from modified proteins upon an increase in temperature. For example, the CIVPSs are excised at reduced rates at temperatures from 37°C and below, but undergo excision more efficiently at temperatures from about 42°C to 80°C. Preferred excision temperatures are between about 42°C and 60°C. Most preferably, predetermined excision conditions are experimentally determined taking into consideration temperatures at which the target protein will not denature or undergo thermal inactivation. The modified proteins can be subjected to the predetermined temperatures for a period of time ranging from less than one minute to several hours. In certain situations, depending on the thermal sensitivity of the target protein, it may be desirable to increase the incubation time period while decreasing the temperature.

Additionally, different modified proteins may exhibit differences in splicing efficiency at various temperatures. If necessary, the optimum temperatures for isolation and splicing of each modified protein can be experimentally determined. If the CIVPS splices at too low a temperature for a proposed purpose, the CIVPS can be modified, or its position in the target protein changed such that the optimum splicing temperature is increased. If the optimum splicing temperature for a particular modified protein is about 37°C, in order to insure that the modified protein does not splice *in vivo,* and thus increase the yield of intact modified proteins, host cells can be grown and the modified protein purified at lower temperatures, e.g., 12°C-30°C. This can also be accomplished by mutating the splicing element to shift the splicing temperature optimum from, for example, 30°C-37°C to 42°C-50°C, and thus resulting in a reduced level of splicing at physiological temperature.

Other IVPSs can be isolated, for example, by identifying genes in which the coding capacity is significantly larger than the observed protein and that encodes a protein sequence not present in the mature protein. A protein containing an IVPS can be distinguished from a protein having a "pre-pro" precursor in that the mature protein will still have the N-terminal and C-terminal sequences of the IVPS containing precursor. Additionally, IVPSs can be detected by the absence of motifs that are conserved in certain protein families, e.g., DNA polymerases. The absence of such a motif may indicate that an IVPS is interrupting that motif (Perler et al., *supra*). Suspected IVPSs can be screened by inserting the suspected protein sequence into a marker protein, e.g., ß-galactosidase, such that the insertion decreases marker protein activity. The resulting modified protein can then be evaluated at periodic intervals for an increase in marker protein activity. See, Example 1-3. Once identified, the DNA encoding the IVPS can be isolated and manipulated using standard DNA manipulation techniques.

Chemical activation of splicing or cleavage may be accomplished by reacting the CIVPS of interest with a chemical reagent which enhances or induces splicing or cleavage. In one preferred embodiment, splicing or cleavage is controlled by employing one or more chemical reagents in a two-step process which first inactivates cleavage or splicing by mutation of the CIVPS or any other means, and then activates cleavage or splicing by addition of a chemical reagent, such as hydroxylamine, ß-mercaptoethanol or dithiothreitol, for example. Control of cleavage or splicing by chemical reagents can be applied to both *cis* and *trans* CIVPS reactions.

The chemical reagent employed depends, in part, on whether cleavage is occurring at the N-terminus or the C-terminus. While not wishing to be bound by theory, N-terminal cleavage is believed to involve an ester or thioester formation between the N-terminal domain and the IVPS. Accordingly, any chemical reagent which facilitates cleavage of the ester or thioester such as hydroxylamine (Bruice and Benkovic, *Bioorganic Mechanisms,* W.A. Benjamin, Inc., New York, (1966), the disclosure of which is hereby incorporated by reference herein) ß-mercaptoethanol or dithiothreitol may be used to induce N-terminal cleavage. C- terminal cleavage is believed to involve cyclization of the IVPS C-terminal conserved asparagine. Accordingly, any reagent which increases the rate of cyclization of asparagine could be used to facilitate C-terminal cleavage. In a process referred to as noncovalent chemical rescue, an enzyme can be mutated, resulting in an inactive form of the enzyme. The activity can then be restored by adding a chemical reagent to the reaction mixture. See, for example, Toney and Kirsch (*Science,* 243:1485-1488 (1989), the disclosure of which is hereby incorporated by reference herein). This process of noncovalent chemical rescue of cleavage activity in CIVPS3 is described in Example 14. Noncovalent chemical rescue of enzyme activity by a chemical reagent can be potentially applied to CIVPS cleavage or splicing mutants at the primary mutation or after introduction of a second mutation at many different possible amino acid residues in the CIVPS using the appropriate chemical reagents for each type of mutation (Toney and Kirsch, *supra*).

While not wishing to be bound by theory, C-terminal cleavage is believed to involve cyclization of the IVPS C-terminal conserved asparagine. Accordingly, any reagent which increases the rate of cyclization of asparagine could be used to facilitate C-terminal cleavage.

IVPSs may also be identified by a larger open reading frame than observed in the mature protein and the presence of a region which has some of the following properties: (1) similarity to HO endonuclease or other homing endonucleases, (2) the amino acid sequence (Ala/Val) His Asn (Ser/Cys/Thr) (SEQ ID NO:45).

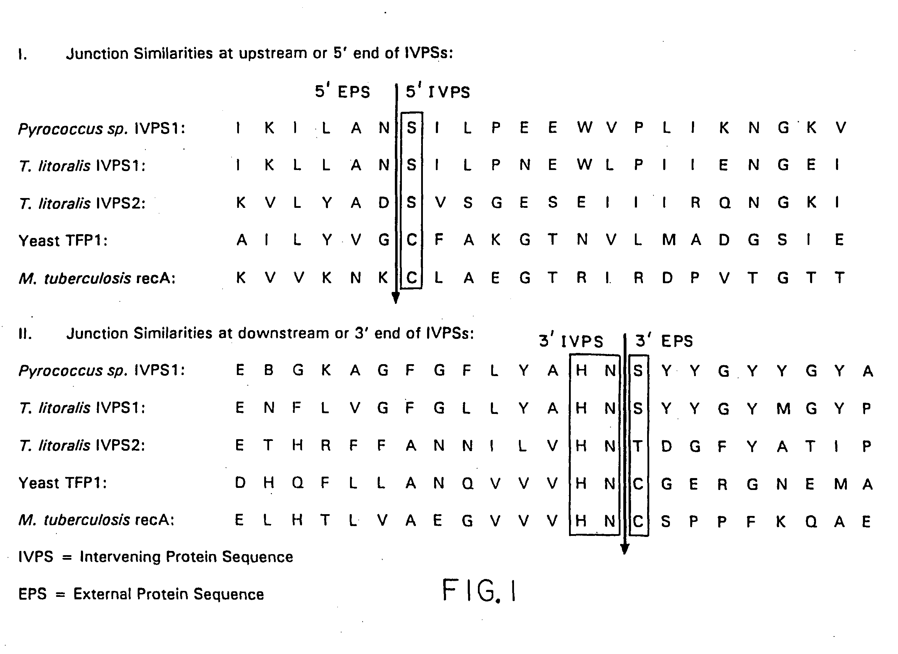

CIVPSs of the present invention also include IVPSs which have been modified such that the splicing reaction can be controlled. As shown in Figure 1 (SEQ ID NO:30, SEQ ID NO:31, SEQ ID NO:32, SEQ ID NO:33, SEQ ID NO:34, SEQ ID NO:35, SEQ ID NO:36, SEQ ID NO:37, SEQ ID NO:38, and SEQ ID NO:39), the aligned splice junctions of known protein splicing IVPSs reveal several similarities. In particular, -OH and -SH side chains are found on residues at the C-terminal side of both splice junctions, preceded by the dipeptide His-Asn at the downstream splice junction.

While not wishing to be bound by theory, it is believed that hydroxyl/sulfydryl groups participate in the splicing reaction and thus modification of these residues modulate the splicing reaction. Such modifications can be evaluated by inserting the modified CIVPS into a marker protein, e.g., ß-galactosidase, such that the insertion decreases marker protein activity. The resulting modified protein can then be evaluated at periodic intervals and under controlled conditions for an increase in marker protein activity. See, Example 1-3. In addition, Western blot analysis can be used to evaluate splicing and cleavage products. See, Example 8. Once identified, the DNA encoding the CIVPS can be isolated and manipulated using standard DNA manipulation techniques.

In accordance with the present invention, it has been found that single amino acid changes at the serine 1082 of CIVPS2 slowed or blocked the protein splicing reaction. Specifically, the threonine substitution mutant displayed 10% of the polymerase activity of the wild-type enzyme, while the cysteine and alanine substitution mutants gave no detectable activity. However, a reaction product corresponding to cleavage at the altered splice junction was observed. This species accumulated in a mutant which replaced the serine at the splice junction with cysteine, but was unaltered when serine was replaced with either threonine or alanine. Wild-type CIVPS2 showed accumulation of a species of the size expected for cleavage at the carboxy terminal splice junction during the splicing reaction, although accumulation of this product decreased, but was still observed, when serine 1082 was changed to threonine, cysteine, or alanine. The S1082A variant showed no evidence of protein splicing, but still produced this product.

Mutagenesis at the carboxy-terminal splice junction, namely amino acid substitutions for the threonine 1472 (T1472) residue with serine produced patterns of splicing identical to the wild-type. Replacement of T1472 with alanine, glycine, or isoleucine gave no detectable splicing. When asparagine 1471 was replaced with alanine, no splicing was observed, but evidence of cleavage at the amino splice junction was observed. Table 1, set forth below, summarizes the effects of amino acid substitutions on splicing and cleavage in CIVPS2.

Accordingly, cleavage at the CIVPS splice junctions can be accomplished in the absence of protein splicing, thus allowing for controlled separation of the CIVPS from the target protein. In certain situations, such activity is desired. In these situations, the CIVPSs of the present invention may also encompass autoproteolytic proteins, such as autoproteolytic proteases, for example, retroviral proteases such as the

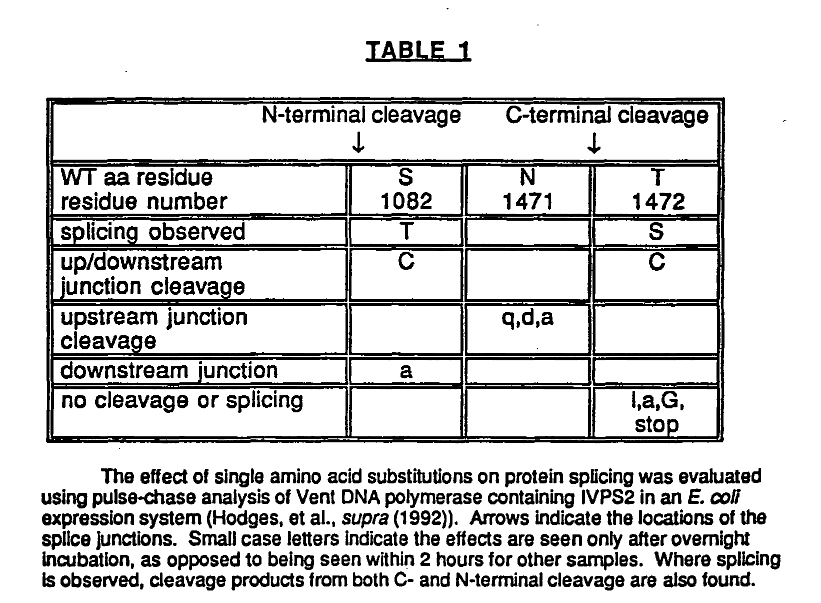

HIV-1 protease (Louis, et al., *Eur. J. Biochem.,* 199:361 (1991)) and Debouck, et al., *Proc. Natl. Acad. Sci. USA,* 84:8903-8906 (1987)). The skilled artisan is familiar with other such proteins. See, Kräusslich, et al., *Ann. Rev. Biochem.,* 701-754 (1988). Such proteins can be modified, in accordance with the disclosed methodology, such that the proteolytic activity is inducible under predetermined conditions.

Modification of the CIVPS amino acids, including splice junction amino acids, can be accomplished in a number of ways. For example, the sequence surrounding the amino acid residue to be modified may be altered to create a biological phosphorylation site allowing it to be a substrate for specific kinases and phosphatases. Examples of protein kinase include, for example, casein kinase II, cAMP-dependent protein kinase, cdc2, and pp60^{c-src} (Pearson and Kemp, *Methods in Enzymology* 200:62 (1991)). Examples of phosphatases include, for example, protein phosphatase 2A, lambda phosphatase, and the yop phosphatase from Yersinia (Tonks, *Current Opinion in Cell Biology,* 2:1114 (1990)).

Using CIVPS2 as an example, as set forth in Example 6C, an arginine residue was placed at position 1079 to create a consensus Calmodulin-dependent protein kinase II site (XRXXS*; Pearson et al., *supra*) The protein splicing reaction may then be regulated by the degree of phosphorylation, using a kinase to create phosphoserine and block the splicing, and phosphatases to remove the phosphate, restoring the wild type serine and, consequently, protein splicing.

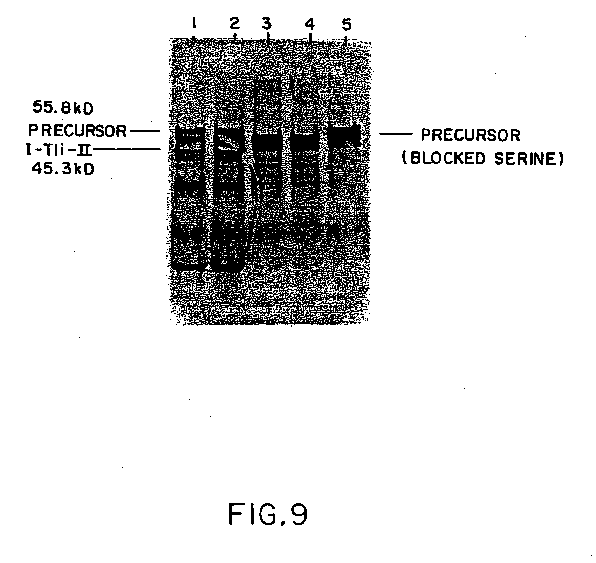

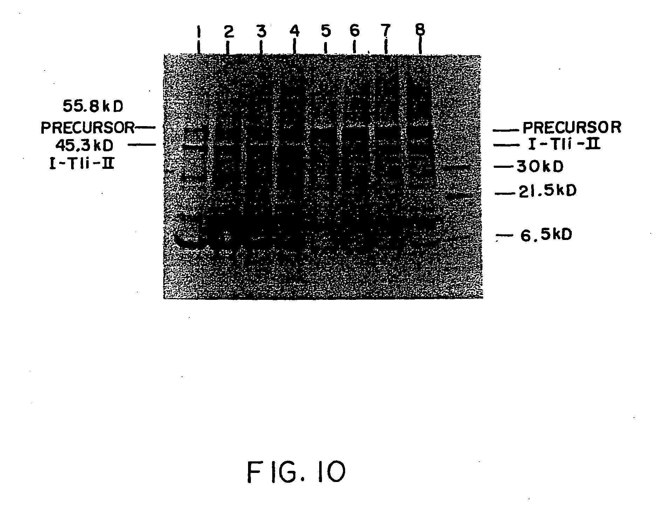

Additionally, critical splice junction residues can be modified chemically such that the splicing reaction is blocked until the modification is reversed. This can be accomplished by using, for example, unnatural amino acid mutagenesis (Noren, et al., *Science* 244:182 (1989); Eliman, et al., *Methods in Enzymology* 202:301 (1991)). Using this method, one of the amino acids involved in the splicing reaction can be replaced, during translation, by a synthetic derivative in which the side chain functionality of the side chain is "masked" by a chemically or photolytically removable group. For example, as set forth in Example 7, serine 1082 of CIVPS2 was modified by this method as follows: An amber stop codon was introduced into the Vent polymerase gene at the position corresponding to serine 1082 (see Example 6D). This gene was then added to an *in vitro* transcription/translation system (Ellman, et al., *supra*) that had previously been demonstrated to support protein splicing of the wild-type gene. In the absence of a tRNA to read through this codon, only truncated product was expected. When an amber suppressor tRNA that had been chemically aminoacylated with 0-(o-nitrobenzyl) serine was added to the system, translation was able to continue past this codon, resulting in site-specific incorporation of the modified serine. As expected, only full-length precursor was observed, indicating that the splicing reaction was blocked (Figure 9). The o-nitrobenzyl group is removable by brief irradiation at 350 nm (Pillai, *Synthesis* 1 (1990)), so the blocked precursor would be expected to splice normally following irradiation. When the blocked precursor was exposed to visible light to free the serine and then incubated to allow the splicing reaction to occur, spliced product was clearly seen (Figure 10).

This strategy could also be applied to threonine 1472, which is found at the downstream splice junction of CIVPS2, as well as any other residue in which either the chemical functionality of the side chain is required for splicing, or introduction of a bulky group at that position would interfere with splicing sterically. Blocking groups can be chosen not only on the basis of the chemistry of the side chain to be protected, but also on the desired method of deblocking (chemically or photolytically). For example, the cysteine groups present in other examples of protein splicing (Figure 1) have thiol side chains that could be blocked using, for example, disulfide exchange (e.g., with dithiodipyridine) or complexation with transition metal ions (e.g., Hg²⁺). See, Corey and Schultz, *J. Biological Chemistry* 264:3666 (1989). The resulting blocked precursors could then be activated for splicing by mild reduction or addition of metal chelators, respectively.

It has been shown that IVPS1 and IVPS2 each encodes an endonuclease, I-Tli-II and I-Til-I, respectively. In addition, DV IVPS1 also encodes an endonuclease, I-PspI, which is inserted at the same position in the DV DNA polymerase gene as IVPS1 is in the Vent DNA polymerase gene and is 62% identical to the Vent IVPS1 gene. It has been found that the IVPS open reading frames in *Tfp1, M. tuberculosis rec A,* Vent and Deep Vent DNA polymerase have protein sequence similarity to homing endonucleases, a class of intron-encoded proteins capable of cleaving alleles which lack the intron. (Hirata et al., *supra,* Kane et al., *supra,* Davis et al., *supra,* Perler et al., *supra*)

Certain host cells may not be able to tolerate the gene product of the CIVPS and thus, in some embodiments it may be preferable to inactivate the endonuclease function. In accordance with the present invention it has been shown that protein splicing can occur when the CIVPS endonuclease function has been inactivated. Such inactivation can be accomplished in a variety of ways, including for example, random mutagenesis, deletion or insertional inactivation, or site directed mutagenesis. Preferably, the endonuclease function is inactivated by site directed mutagenesis. I-Tli-I shares sequence similarity with other "homing endonucleases" in the pair of characteristic dodecapeptide motifs (Cummings et al., *Curr. Gent.* 16:381 (1989)). As shown in Example 6B, endonuclease activity was inactivated by oligonucleotide-directed mutagenesis of a single residue (aspartate 1236 to alanine) within one of these motifs. Substitution of alternative residues could also reduce or abolish endonuclease activity without affecting protein splicing. Inactivation of endonuclease function has been shown to increase the stability of constructs carrying the modified proteins.

Target proteins which can be used in accordance with the present invention include, for example, enzymes, toxins, cytokines, glycoproteins and growth factors. Many such proteins are well known to the skilled artisan. The amino acid and nucleotide sequence of such proteins are easily available through many computer data bases, for example, GenBank, EMBL and Swiss-Prot. Alternatively, the nucleotide or amino acid sequence of a target protein can be determined using routine procedures in the art.

If it is desirable to substantially inactivate target protein activity, the CIVPS is inserted into a region(s) that will inactivate such activity. Such regions are well known to the skilled artisan and include, for example, binding sites, enzyme active sites, the conserved motifs of proteins, e.g., DNA polymerases, and dimerization or multimerization sites. Alternatively, the CIVPS may be inserted randomly and the activity of each modified protein measured until the desired level of activity is obtained. Preferably, such a modified protein has about a 50% reduced level of activity compared to the native protein. More preferably about 75%. Still more preferably greater than 99%.

The CIVPS may be inserted into the target gene by any number of means. Preferably, to assure proper protein splicing if the CIVPS is excised, it is important to insert the CIVPS immediately before a proper splice junction residue because excision of the CIVPS leaves that amino acid at the splice junction. This can be accomplished by either inserting the CIVPS immediately before the appropriate splice junction amino acid or by modifying the CIVPS such that it "brings" the appropriate amino acid with it.

For example, CIVPS1, 2 or 3 can be inserted immediately before the appropriate splice junction amino acids, for example, serine, threonine or cysteine residues, most preferably before serine or threonine. See, Figure 1. Such sites are readily available in most target proteins.

In certain situations, such as when the target protein is a toxin, it may be desirable to further control protein splicing by adding a secondary control. This may be accomplished by inserting the CIVPS before a less optimal amino acid, for example, one that the CIVPS does not normally precede and thus may slow down the splicing reaction.

As set forth above, insertion can be at any site within the target protein if the CIVPS "brings" the appropriate downstream amino acid with it. This can be accomplished by creation of CIVPS DNA having a codon for the desired downstream amino acid. Methods for producing such DNA are set out in detail below. This DNA can then be inserted at any site within the target DNA. Upon protein splicing of the resulting modified protein, the extra residue brought by the CIVPS will be left behind. Thus, if activity of the final product is important, the skilled artisan must takes steps to assure that the extra residue will not be left in an area of the target protein that will adversely affect activity.

The CIVPS may be inserted into the target protein, or fused to the target protein, by chemically synthesizing the primary amino acid sequence of the target protein, including the CIVPS, inserted at any desired site, using standard methods (e.g., see Hunkapiller, et al., *Nature* 310:105 (1984)) and a commercially available protein synthesizer.

Alternatively, a DNA sequence encoding a CIVPS is inserted in, or fused to, a DNA sequence encoding for a target protein such that both coding sequences form a continuous reading frame. This can be accomplished using a variety of methods known to the skilled artisan, several of which are set out below.

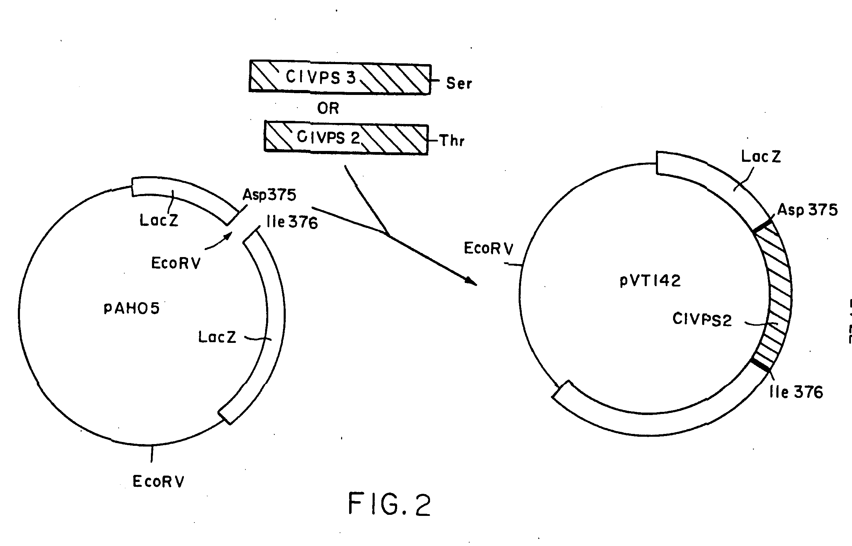

For example, the CIVPS DNA is inserted into any restriction enzyme site that makes a blunt cut in the target gene and which is in frame. This can be accompanied by first, synthesizing an CIVPS DNA fragment with a threonine codon (for Vent IVPS2) or a serine codon (for Deep Vent IVPS1 or Vent IVPS 1) at its 3' end. This fragment is then ligated in-frame to a linear plasmid cut to blunt ends by the restriction endonuclease. Using the *lacZ* DNA sequence, for example, an *Eco*RV site can be used to insert Vent IVPS2 or Deep Vent IVPS1 between residue 375 (aspartic acid) and 376 (isoleucine). See, Figure 2. However, as discussed above, using this method, if the CIVPS is excised an extra residue is expected to remain at the splice junction and therefore depending on where the CIVPS is inserted, the resulting protein may not have the same function or structure as the native protein.

The CIVPS DNA could also be inserted by making silent mutations (preserving the amino acid residue) near one end or both ends of the CIVPS to create restriction sites compatible with the target gene. Using CIVPS2 as an example, a *Bsp*EI restriction site can be made near the 5' end and a *Spe*l restriction site near its 3' end, by silent mutations. Using PCR primers overlapping the new restriction sites and continuing through the beginning of the *lac*Z target gene at either asp 594 or thr 595, one can generate a *lac*Z fragment with compatible *Bsp*E1 and *Spe*I restriction sites. Then, the CIVPS is inserted between an aspartic acid codon (residue 594) and a threonine codon (residue 595) within the *lac*Z coding region. DNA fragment(s) can be synthesized from both the CIVPS and the target gene by PCR with their ends at the insertion site overlapping with the termini of the CIVPS, therefore, including the same restriction sites. After appropriate restriction endonuclease treatment. DNA fragments with compatible ends can then be ligated to create a fusion gene. Since no extra residue would be left after excision of the CIVPS, native polypeptide will form when splicing occurs. Preferably, the restriction site being created is unique within the CIVPS and within the target gene to avoid ligation of multiple fragments and thus, complicated screening procedures.

If the plasmid vector carrying the target gene sequence is relatively small, for example, less than about 5Kb, a linear form of the plasmid can be generated using PCR, and then the linear plasmid can be ligated to the CIVPS gene. Using this method the CIVPS gene can be inserted at any location in the target gene as follows: First, plasmid DNA containing the target gene can be synthesized by PCR using a pair of primers starting at the insertion site, for example, serine or threonine codons for CIVPS1, 2 and 3, or any codon if the CIVPS also brings the appropriate downstream amino acid. Next, the CIVPS gene (with or without serine or threonine) can be ligated to the linear plasmid DNA (without the serine or threonine codon). The required splice junction amino acids (serine or threonine) can be positioned on either the CIVPS fragment or on the target gene. The advantage of having the required amino acid on the CIVPS fragment when placing upstream of an endogenous serine or threonine is that the self-ligated vector DNA (without the CIVPS insert) may only express a deficient product of the target gene because of the deletion of the serine or threonine in the coding region. This may aid in phenotype selection for the fusion construct if the fusion protein can splice to produce a functional product.

The fusion DNA encoding the modified protein can be inserted into an appropriate expression vector, i.e., a vector which contains the necessary elements for the transcription and translation of the inserted protein-coding sequence. A variety of host-vector systems may be utilized to express the protein-coding sequence. These include mammalian cell systems infected with virus (e.g., vaccinia virus, adenovirus, etc.); insect cell systems infected with virus (e.g., baculovirus); microorganisms such as yeast containing yeast vectors, or bacteria transformed with bacteriophage DNA, plasmid DNA or cosmid DNA. Depending on the host-vector system utilized, any one of a number of suitable transcription and translation elements may be used. For instance, when expressing a modified eukaryotic protein, it may be advantageous to use appropriate eukaryotic vectors and host cells. Expression of the fusion DNA results in the production of the modified proteins of the present invention.

Once obtained, the modified proteins can be separated and purified by appropriate combination of known techniques. These methods include, for example, methods utilizing solubility such as salt precipitation and solvent precipitation, methods utilizing the difference in molecular weight such as dialysis, ultra-filtration, gel-filtration, and SDS-polyacrylamide gel electrophoresis, methods utilizing a difference in electrical charge such as ion-exchange column chromatography, methods utilizing specific affinity such as affinity chromatography, methods utilizing a difference in hydrophobicity such as reverse-phase high performance liquid chromatography and methods utilizing a difference in isoelectric point, such as isoelectric focusing electrophoresis.

If desired, the modified proteins can be subjected to predetermined conditions under which the CIVPS is excised. Such conditions depend on the CIVPS used. For example, CIVPS 1, 2 and 3 are capable of excision by subjecting the modified protein to increased temperature, 42°C - 80°C, most preferably, 42°C - 60°C. This can be accomplished using any known means, for example a water bath or a heat generating laser. The time period for incubation can range from less than one minute to greater than several hours. As discussed above, in certain situations, depending on the thermal sensitivity of the target protein, it may be desirable to increase the incubation period while decreasing the temperature. In addition, if *in vivo* splicing is desired, temperatures compatible with the growth of the host organism are preferred.

The present invention may be used to produce proteins that are highly toxic to the host cells by using the CIVPS to modifying a toxic target protein such that the modified protein is non-toxic. This can be accomplished, for example, by inserting the CIVPS into a region(s) responsible for toxicity. After isolation, the non-toxic modified protein can then be subject to predetermined condition under which the CIVPS will excise and the resulting toxin can be isolated.

If a protein is extremely toxic to a host cell it may be desirable to produce that protein using a method referred to as "transplicing". Using this method the toxic protein is produced in two or more pieces in separate host cells, each piece being modified by insertion of a CIVPS. For example, a first modified protein can be produced comprising an amino portion of a target protein to which is inserted at its carboxy terminus an amino terminal fragment of a CIVPS, thereafter a second modified protein comprising the remaining portion of the target protein into which is inserted at its amino terminus the remaining fragment of CIVPSs. Alternatively, overlapping CIVPS fragments can be used. Each modified protein is then isolated from the host cells and incubated together under appropriate conditions for splicing of the CIVPS. This results in a ligated target protein. By dividing the target protein in two different hosts, there is no possibility that even a minute fraction will splice *in vivo,* adversely affecting the host. In addition, the entire CIVPS may be inserted on either side of the splice junction of the first modified protein and the remaining target protein fragment added to the splicing mixture.

Accordingly, *trans* -splicing may allow expression of highly toxic genes in *E. coli* by expressing only an inactive portion of the target protein in each of two different hosts. The two complementary fragments can then be purified in large amounts and ligated together by *in vitro trans* -splicing. By dividing the CIVPS into 2 parts, its splicing activity is effectively controlled until the two parts are brought together. Therefore, any IVPS becomes a CIVPS when divided into 2 parts which are purified from different hosts and kept separate until splicing is required.

*Trans*-cleavage combines the properties of *trans*-splicing, CIVPS cleavage and the three part affinity-cleavage vector systems. In *trans*-cleavage, the CIVPS is separated into 2 fragments, which, when combined and activated, result in cleavage between the protein of interest and the CIVPS. In one envisioned application similar to that described for *cis*-cleavage in Example 9, *trans*-cleavage can be used for affinity purification of a protein of interest. In this application, one or both fragments of the CIVPS has an affinity tag for purification and a cloning site to make an in-frame fusion with the protein of interest. Each of the two constructs are grown and induced separately as described for *trans*-splicing. Protein from each of the two constructs is then purified either by standard chromatographic or affinity techniques. The two protein fragments are then combined under conditions which allow the two parts of the CIVPS to come together to form an active CIVPS. Cleavage is then induced by temperature, pH, chemical reagents or other means, releasing the purified protein of interest.

In one embodiment, the combination of the two parts of the CIVPS can occur while one part is bound to a solid matrix; in this case, after activation of cleavage, the protein of interest is released from the solid matrix while the CIVPS and any affinity tag remain on the solid matrix. Under some conditions, the two CIVPS fragments will remain associated after the cleavage reaction, allowing both to remain bound to the solid support even though only one fragment has an affinity tag. One might also have affinity tags on both fragments of the CIVPS to allow separation of the protein of interest from the CIVPS fragments after cleavage. As in the case of the 3 part fusion described in Example 9, the order of the binding domain, the CIVPS and the protein of interest can be varied. All variations described for CIVPS purification and cleavage schemes can be applied to *trans*-cleavage systems also.

By using the same information obtained for *cis*-cleavage on CIVPS fusions or by using new mutations, cleavage may be programmed to occur at either the N-terminal or the C-terminal of the CIVPS. In the present example, the starting point for the CIVPS fragments are those described in Example 12. These CIVPS3 fragments were converted to *trans*-cleavage reagents by cassette replacement as described in Example 10. Some of the many possible mutations which we have shown result in *trans*-cleavage at the C-terminal of CIVPS3 are Ala535 of CIVPS3 to Lys, Ser1 of CIVPS3 to Ala and Ile2 of CIVPS3 to Lys. Asn537 of CIVPS to Ala resulted in *trans*-cleavage at the N-terminal of CIVPS3.

The IVPSs of the present invention may be used in a "protein ligation" to add unnatural amino acid residues, structural probes, identifying epitopes or tags, or other determinants to a target protein. For example, the target protein can be fused to the amino terminus of the IVPS. A stop codon can be placed immediately following the carboxy terminus of the IVPS. The peptide to be fused can then be added to the mixture. If necessary, in order to more closely mimic the native splicing mechanism, the amino terminus of this peptide may be serine, threonine, or cysteine. The splicing reaction may then proceed, pushed by mass action towards splicing of the product.

The above reaction could also be adapted to occur with a starting material composed of the IVPS fused at the carboxy terminus to the amino terminus of the target protein. Initiation at a methionine engineered to precede the serine residue which begins in certain CIVPS would allow translation to occur which would likely be processed off in *E*. *coli* leaving an amino terminal serine residue. The peptide to be fused to the amino terminus of this target protein could then be added, and splicing allowed to proceed. Such an approach may be favored since there is no known requirement for the carboxy terminal residue on the peptide being added. Additionally, current experimental evidence suggests that cleavage of the upstream splice junction precedes the ligation reaction, indicating this approach more closely approximates the native reaction mechanism. Targeting peptides could also be added to the peptide to facilitate translocation of the fusion protein.

The present invention can also be used to study the effect of a target protein during a specific part of the cell cycle or under specific conditions such as induction of another protein or differentiation. For example, the chromosomal copy of a gene encoding a particular protein can be replaced with a version containing a CIVPS. At a specific point in the cell cycle, differentiation or other desired point, the cells are heated causing the precursor to splice, and thus the active target protein is present only at this point.

The CIVPs of the present invention can also be used to isolate modified proteins by use of affinity chromatography with antibodies specific to the CIVPS. For example, monoclonal or polyclonal antibodies can be generated having binding affinity to a CIVPS using standard techniques. These antibodies can then be utilized in affinity chromatography purification procedures to isolate a modified protein. After purification, if desired, the modified proteins can be subjected to predetermined conditions under which the CIVPS will undergo excision.

As discussed above, cleavage at the CIVPS splice junction can be accomplished in the absence of protein splicing, thus allowing for controlled separation of the CIVPS from the target protein. Such CIVPSs can therefore be used in a fusion protein purification system.

Fusion protein purification systems are well known to the skilled artisan. See, EPO 0 286 239 and N.M. Sassenfeld, *TIBTECH,* 8:88-93 (1990). Typically, in such systems, a binding protein and a target protein are joined by a linker having a protease recognition site. The fusion is then purified by affinity chromatography on a substrate having affinity for the binding protein. The binding protein and the target protein are then separated by contact with a protease, e.g., factor Xa. In these systems, in order to obtain a highly purified target protein, the protease must be separated from the target protein, thus adding an additional purification step, as well as the potential for contamination. The method of the present invention, by using a CIVPS, instead of a protease, avoids these and other problems encountered in currently used protein fusion purification systems.

In the method of the present invention, a modified protein comprising a fusion protein in which a CIVPS is between the target protein and a protein having affinity for a substrate (binding protein) is formed. Techniques for forming such fusion proteins are well known to the skilled artisan. See, EPO 0 286 239 and J. Sambrook, et al., Molecular Cloning: A Laboratory Manual (1989), Cold Spring Harbor Laboratory Press, Cold Spring Harbor, NY, p. 17.29-17.33.

Binding proteins which may be employed in the method of the present invention include, for example, sugar binding proteins, such as maltose or arabinose binding protein, receptor binding proteins, amino acids binding proteins and metal binding proteins. Other binding proteins are well known to the skilled artisan. See, EPO 0 286 239 and N.M. Sassenfeld, *TIBTECH, supra.*

The modified protein is then contacted with a substrate to which the binding protein has specific affinity, e.g., using affinity chromatography.

The highly purified target protein can be liberated from the column by subjecting the CIVPS to predetermined conditions under which cleavage is initiated, for example, between the CIVPS and the target protein. Alternatively, the purified fusion protein can be eluted from the column and liberated as above.

The present invention is further illustrated by the following examples. These examples are provided to aid in the understanding of the invention and are not construed as a limitation thereof.

All references cited above and below are herein incorporated by reference.

### EXAMPLE 1

### SYNTHESIS OF IVPS CASSETTES FOR INSERTION INTO BLUNT SITES BETWEEN TARGET GENE CODONS

DNA fragments or cassettes for in-frame insertion of IVPSs into the *lacZ* coding region or any other target gene can be prepared by polymerase chain reaction (PCR) with or without the first downstream external protein sequence (EPS) codon. The native downstream residues are serine for Deep Vent IVPS1 and Vent IVPS1 or threonine for Vent IVPS2. It has been found that IVPS2 can splice if it precedes a threonine or cysteine, although at reduced levels. Although not wishing to be bound by theory, it is believed that all the IVPSs can splice to some extent when preceding either serine, threonine or cysteine. Cassettes including the downstream serine or threonine can be inserted at any desired location in the target gene including preceding a serine or threonine. In the latter constructions, one may delete the serine or threonine from the target gene and substitute it with the incoming residue on the cassette. Cassettes lacking downstream serines, threonines or cysteines may be inserted prior to a serine, threonine or cysteine in the target gene.

The following protocol describes the production of cassettes for Deep Vent IVPS1 (CIVP3) and Vent IVPS2 (CIVPS2) (endo⁺ and endo-versions), including the first downstream EPS codon.

The PCR mixture contains Vent DNA polymerase buffer (NEB), supplemented with 2 mM Magnesium sulfate, 400 µM of each dNTP, 0.9 µM of each primer and 40 ng plasmid DNA and 2 units of Vent DNA polymerase in 100 µl. Amplification was carried out by using a Perkin-Elmer/Cetus thermal cycler at 94°C for 30 sec, 48°C for 30 sec and 72°C for 2 min for 30 cycles. Deep Vent IVPS1 was synthesized from pNEB #720 (ATCC No. 68723) which has a 4.8 Kb BamHI fragment containing the *Pyrococcus sp.* DNA polymerase gene inserted into the BamHI site of pUC19. Vent IVPS2 was synthesized from pV153-2 which has a 1.9 kb EcoR1 fragment (2851-4766) of the Vent DNA polymerase gene sequence in the vector Bluescribe SK- (Stratagene). Alternatively, pNEB671 (ATCC No. 68447) can also be used for IVPS2. pAMQ29 is an endonuclease-deficient derivative of pV153-2, carrying an amino acid substitution (aspartic acid 1236 to alanine) within the Vent IVPS2 coding region. Primers 5'-AGTGTCTCCGGAGAAAGTGAGA T-3' (SEQ ID NO:3) (Vent IVPS2 forward, 3534-3556, a substitution of A3542 to C) and 5'-AGTATT GTGTACCAGGATGTTG-3' (SEQ ID NO:4) (Vent IVPS2/Thr reverse, 4685-4706) were used to synthesize endo⁺ or endo⁻ Vent IVPS2 fragment (1173 bp) with a threonine codon at its 3' terminus. Primers 5'-AGCATTTTACCGGAAGAATGGGTT-3' (SEQ ID NO:5) (DV IVPS1 forward, 1839-1862) and 5'-GCTATTATGTGCATAGA GGAATCCA-3'(SEQ ID NO:6) (DV IVPS1/Ser reverse, 3428-3452) were used to synthesize the *Pyrococcus sp.* (or Deep Vent) IVPS1 fragment (1614 bp) with a serine codon at its 3' end. Reverse primers lacking the final three nucleotides could be used to generate IVPS fragments lacking the C-terminal serine or threonine.

The PCR samples were extracted with phenol and chloroform, precipitated in 0.3 M NaAc and 70% ethanol at -20°C for overnight, recovered by spinning at 10 K for 10 min in a microfuge, dried and each resuspended in 30 µl of distilled water, loaded on a 1 % agarose gel for electrophoresis at 60 volts for 15 hours. The gel slices that contain the PCR-amplified fragments were placed in a 1% low melting agarose gel for electrophoresis at 80 volts for 2 hours. DNA fragments were recovered from the low melting agarose gel by incubation in 0.5 ml of TE buffer (10 mM Tris-HCl/0.1 mM EDTA, pH8.0) at 65°C for 30 min, extractions with phenol, phenol-chloroform (1:1 mixture) and chloroform, precipitation in 0.6 M NaAc (pH5.2) and 50% isopropanol at -20°C for overnight. DNA was spun down, washed with 70% ethanol, dried and resuspended in 15.5 µl distilled water.

Phosphorylation of the IVPS DNA fragments was performed at 37°C for 60 min with 2 µl of 10 x polynucleotide kinase buffer (NEB), 15.5 µl of purified DNA , 2 µl 10 mM ATP, and 5 units of T4 Polynucleotide kinase (NEB) in 20 µl. The samples were heated in a 65°C water bath for 10 min. After addition of 80 µl of TE buffer (10 mM Tris-HCl/0.1 mM EDTA, pH8.0), the samples were sequentially extracted with phenol, phenol-chloroform (1:1 mixture) and chloroform. DNA was precipitated in 2.5 M NH₄Ac and 70% ethanol at -70°C for 3.5 hours, pelleted by spinning at 10 K for 10 min in a microfuge, washed with cold 70% ethanol, dried and resuspended in distilled water (20 µl for Vent IVPS2 or Deep Vent IVPS1 DNA, 10 µl for Vent IVPS endo- DNA).

### EXAMPLE 2

### IN-FRAME INSERTION OF IVPS IN A RESTRICTION ENZYME LINEARIZED PLASMID, SUCH AS ONE ENCODING β-GALACTOSIDASE

In this example, we describe how the IVPS cassettes can be cloned into a target gene by inserting the cassette at a restriction enzyme site which makes a blunt cut in the target gene between 2 codons. The cassette can carry a C-terminal serine, cysteine or threonine if necessary. This protocol works best if the restriction enzyme cuts the target gene vector once or twice. As an example, we describe insertion into the EcoRV site of the *lac*Z gene (Figure 2).

### PREPARATION OF ECORV-LINEARIZED PAHO5

pAHO5 carries the entire *lac*Z gene sequence on a 3.1 kb BamHI-*Dral* fragment from pRS415 (Simons, et al., *Gene* 53:85-96 (1987)) inserted between *Bam*HI and *Sma*I sites in the polylinker of pAGR3 downstream of a tac promoter. The tac promoter is a transcription control element which can be repressed by the product of the *lac*lq gene and be induced by isopropyl ß-D-thiogalactoside (IPTG). The 5.9 Kb vector pAHO5 (NEB) also has a transcription terminator sequence upstream of the tac promoter and the polylinker, and the *E. coli lac*l*q* gene. pAHO5 contains two *Eco*RV recognition sequences. *Eco*RV leaves blunt ends at its cleavage site. One of the *Eco*RV cleavage sites cuts within the *lacZ* coding region between the 375th codon (aspartic acid) and the 376th codon (isoleucine) and is planned as the site for in-frame insertion of the IVPS fragments. The other site is located 3.2 Kb downstream within the *E. coli lac*Iq gene. The plasmid is cut partially to produce some molecules in which only one of the *Eco*RV sites has been cleaved. These linear plasmids are purified. The IVPS cassettes will be randomly cloned into either *Eco*RV site. Therefore, the resultant recombinants must be screened for orientation and insertion into the proper *Eco*RV site. DNA was partially digested by incubation of 15 µg of pAHO5 DNA with 40 units of *Eco*RV (NEB) in 100 µl of 1 × NEB buffer 2 at 37°C for 60 min. 20 µl agarose gel loading dye was added to the sample after the sample was heated to 65°C for 10 min to inactivate *Eco*RV. DNA fragments were separated by electrophoresis on a 1% low melting agarose gel. Linearized pAHO5 plasmid DNA was recovered from the low melting agarose gel as described in example 1 and resuspended in 44.6 µl of distilled water.

Dephosphorylation of *Eco*RV-linearized pAHO5 was carried out in 50 µl of 1 X NEB buffer 2 at 50°C for 60 min. in the presence of 2 µg DNA and 4 units of Calf Intestinal Alkaline Phosphotase (NEB). The sample was heated in a 65°C water bath for 30 min after addition of 0.5 µl of 0.5 M EDTA (pH8.0) and extracted with phenol, phenol-chloroform (1:1 mixture), and chloroform. DNA was precipitated in 0.75 M NH4Ac and 70% ethanol for 2 hours, recovered as described in Example 1, and resuspended in 20 µl of distilled water.

### CONSTRUCTION OF IVPS-lacZ FUSION GENES

Ligation of dephosphrylated pAHO5 DNA with phosphorylated IVPS fragments was carried out at 16°C for 15 hours in 20 µl volume with addition of 8.6 µl distilled water, 2µl of 10 X T4 DNA ligase buffer (NEB), 4 µl of 0.1 µg/µl dephosphorylated pAHO5 DNA, 5 µl IVPS DNA prepared as described above (0.25 µg of Vent IVPS2, 0.4 µg Deep Vent IVPS1 or 0.25 µg of Vent IVPS2 endo⁻) and 160 units of T4 DNA ligase (NEB).

*E. coli* strain RR1 was transformed by mixing 100 µl of competent RR1 cells with 10 µl of ligation sample on ice for 30 min., heating at 42°C for 2 min., chilling on ice for 5 min., adding 0.8 ml LB media (10 grams/liter tryptone, 5 grams/liter yeast extract, 10 grams/liter NaCl, 1 gram/liter Dextrose, 1 gram/liter MgCl₂.6H₂O, pH7.2 at 25°C) and incubating at 30°C for 45 min. The samples were plated onto LB plates, supplemented with 100 µg/ml ampicillin. After incubation overnight at 30°C, about 150-300 colonies per plate were observed.

Colony hybridization was utilized to screen for clones that carry recombinant plasmids. The Vent IVPS2 forward primer and the Deep vent IVPS1 forward primer, described in example 1, were radio-labeled with (_^{-32P}) ATP using T4 polynucleotide kinase and used as hybridization probes. Colonies were lifted onto nitrocellulose and treated for 5 min. in each of the following solutions: 10% SDS, 0.5 M NaOH/1.5 M NaCl, 0.5 M Tris-HCl (pH7.5)/0.5 M NaCl (twice) and 2XSSC (twice). The nitrocellulose filters were dried at room temperature for 1 hour, baked in vacuum at 80°C for 2 hours, soaked in 6 x SSC for 5 min and washed in a solution of 50 mM Tris-Cl (pH8.0), 1 M NaCl, 1 mM EDTA and 0.1% SDS at 42°C for 2 hours. After treatment at 42°C for 4 hours in 6 X NET, 5 X Denhardt's, 0.5% SDS and 25 µg/ml of denatured salmon sperm DNA, the filters were incubated with the radiolabeled oligomer probe under the same conditions for 16 hours and then washed in 6 x SSC at room temperature three times for 15 min, twice at 42°C for 2 min and twice at 50°C for two min, followed by autoradiogram. 36 clones were found to hybridize to the corresponding oligomer probes.

The positive clones were further analyzed to determine insert location by PCR amplification of plasmid DNA extracted from these clones, using the Vent IVPS2 forward primer (or the Deep Vent IVPS1 forward primer) described in Example 1, and a *lacZ* reverse primer (5'-AGGGTCGACAGATTTGATCCAGCG-3- (SEQ ID NO:7)) complementary to the *lacZ* coding sequence (1417-1440, with a G:T mismatch at 1437) 392 nt downstream of the insertion site. PCR reactions from 14 clones produced the corresponding DNA fragments. Clones pVT133, 138, 139, 141, 142, and 144 contain the 1.4 Kb Vent IVPS2 insert, and pVTE 834, 836, 839 and 841 contain the Vent IVPS2 (endo⁻) insert, all yielding DNA fragments of approximately 1.1 kb. Clones pDVS 712, 742, 745 and 746 carry the 1.6 Kb Deep Vent IVPS1 insert, producing DNA fragments of about 2.0 Kb.

### EXPRESSION OF THE IVPS-lacZ FUSION GENES

The clones were further examined by their ability to express fusion (modified) proteins with inducer IPTG.

The clones were cultured in LB medium supplemented with 100 µg/ml ampicillin at 30°C until OD₆₀₀ₙₘ reached 0.5. To prepare lysate from uninduced cells, 1.5 ml of culture was pelleted and resuspended in 100 µl of urea lysis buffer, followed by boiling for 10 min. After addition of IPTG to a final concentration of 0.3 mM, the cultures were grown at 30°C for 4 additional hours. Cells from 1.5 ml culture were pelleted and then lysed with 250 µl of the urea lysis buffer after induction for 2 hours and 4 hours. Protein products were analyzed by Coomassie Blue stained gels. Three of the Vent IVPS2-*lac*Z fusion constructs (pVT139, 142 and 144) and all four Vent IVPS2 (endo⁻)-*lac*Z fusion constructs showed a major product of about 162-165 KDa, the expected size for a Vent IVPS2-ß-galactosidase fusion protein. All four Deep Vent IVPS1-*lacZ* fusion clones expressed a larger product of 173-178 KDa, the expected size for the Deep Vent IVPS1-ß-galactosidase fusion protein.

The identity of the Vent IVPS2 fusion proteins from pVT142 and 144, and pVTE836 and 839 was further analyzed by western blots using antibody raised against I-Tli-I (NEB) or ß-galactosidase (Promega). Samples were electrophoresed on 4-20% SDS gels (ISS, Daichi, Tokyo, Japan) with prestained markers (BRL), transferred to nitrocellulose, probed with antisera (from mouse), and detected using alkaline phosphate-linked anti-mouse secondary antibody as described by the manufacturer (Promega). A band of approximately 160 KDa from all four clones being examined reacts with both sera and migrates at the same location as the Coomassie Blue stained band. Deep Vent IVPS1 fusions were also examined. Western blot analysis of pDVS712 and 742 using sera against ß-galactosidase and I*-Psp*l (the protein product of Deep Vent IVPS1) yielded the predicted major band at about 168-175 KDa, identical to the Coomassie Blue stained band.

### EXAMPLE 3

### THERMAL CONTROL OF PROTEIN SPLICING IN β-GALACTOSIDASE-IVPS FUSIONS

The constructs described above (IVPSs inserted into the *lacZ Eco*RV site) yield fusion (modified) proteins after induction. The IVPS protein can be excised from the fusion protein to generate a ligated target protein (active ß-galactosidase) and free IVPS endonuclease by incubation at elevated temperatures.

### SPLICING IS CONTROLLABLE BY TEMPERATURE INDUCTION: ß-GALACTOSIDASE ACTIVITY IN CRUDE EXTRACTS INCREASESIN RESPONSE TO TEMPERATURE SHIFT

Crude extracts were prepared from cultures of RR1 (the *E. coli* host) and RR1 containing pAHO5 (the non-fusion ß-galactosidase parent plasmid described in Example 2) or the fusion constructs, pVT142 (Vent IVPS2 or CIVPS2), pVTE836 (Vent IVPS1 endo-) or pDVS712 (Deep Vent IVPS1 or CIVPS 3) by the following steps. A single colony was inoculated in 10 ml LB medium supplemented with 100 µg/ml ampicillin, incubated at 30°C overnight, subcultured in 1 liter LB medium (100 µg/ml ampicillin) at 30°C to OD₆₀₀ₙₘ about 0.5 and induced with IPTG at 0.3 mM at 30°C for 2 hours. Cells were spun down and resuspended in 100 ml of LB, sonicated for 3 min at 4°C and spun at 7000 rpm for 15 min. The supernatants were recovered and stored at -20°C.

7.5 ml aliquots of crude extracts were incubated in 42°C or 50°C water baths; 1 ml aliquots were taken at 1, 2 and 12 hours for pVT142 and pVTE836 extracts or 0.5, 1, 2, 4 and 16 hours for pDVS712, pAHO5 or RR1 extract.

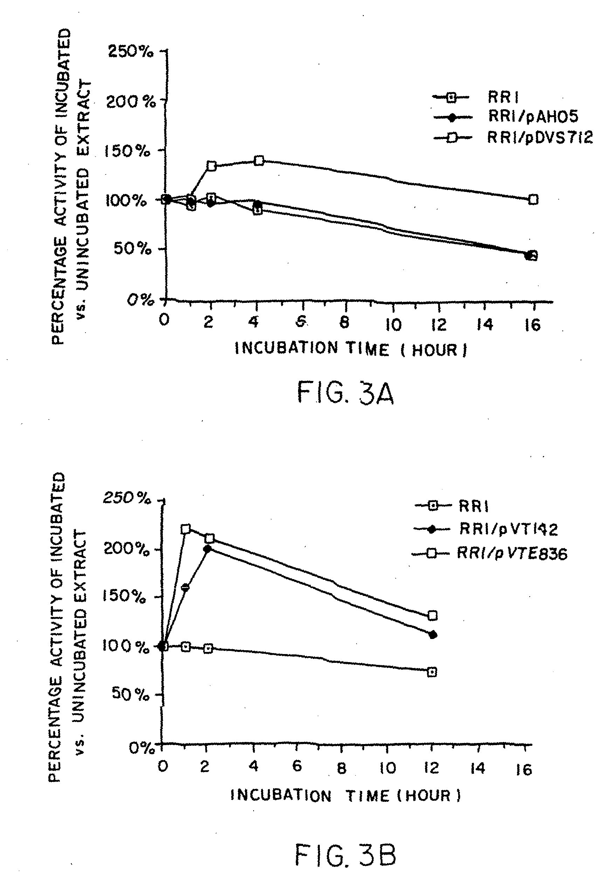

ß-galactosidase activity was measured according to Miller et al. (Experiments in Molecular Genetics (1972), Cold Spring Harbor, New York, Cold Spring Harbor Laboratory). Assay buffer was prepared by mixing Z buffer with 2.7 µl/ml of 2-mercaptoethanol. Substrate o-nitrophenyl-ß-D-galactopyranoside (ONPG) was dissolved in the assay buffer at 4 mg/ml. 0.1 ml of treated or untreated extract was transferred into a test tube containing 0.9 ml of assay buffer and 1 drop of 0.1%SDS and incubated for 5 min at 28°C. 0.1 ml LB medium was used for blank. 0.2 ml of 4 mg/ml ONPG was added to start an assay reaction. When adequate yellow color developed, the reaction was stopped by addition of 0.5 ml of 1 M Na₂CO₃. The incubation time was recorded and activity was measured on a spectrophotometer at OD₄₂₀ₙₘ and OD₅₅₀ₙₘ. The enzymatic activity from the heat-treated extract was calculated as follows. The activity after incubation was divided by the activity of the zero time point; the ratio was then multiplied by 100 to yield a percentage. Comparison of enzymatic activity indicated that while heat treatment had no effect on activity from RR1 or RR1/pAHO5 extract in the first two hours of incubation, all three IVPS-LacZ fusion constructs, pVT142, pVTE836 and pDVS712, exhibited an increase in enzymatic activity in response to the temperature shift to 42°C from 143% to 221 % of untreated samples (Figure 3A and 3B). This increase in ß-galactosidase activity was due to excision of the IVPS and ligation of the two halves of ß-galactosidase, forming more enzyme which was active. The splicing was confirmed by Western blot analysis. ß-galactosidase activity in RR1 cells comes from expression of the chromosomal gene. The overnight incubation resulted in lower enzymatic activity from all samples, probably due to thermal inactivation of ß-galactosidase (Figure 3A and 3B).

### SPLICING IS CONTROLLABLE BY TEMPERATURE INDUCTION: ANALYSIS OF PROTEINS BY COOMASSIE BLUE STAINING AND WESTERN BLOTS

Analysis of IVPS-*lacZ* fusion protein synthesis in RR1 cells is complicated by chromosomal expression of ß-galactosidase. Therefore, for ease of analysis, all the constructs were transferred to an *E. coli* host which did not synthesize ß-galactosidase.

Preparation of crude cell extracts from the IVPS-*lacZ* fusion clones and western blot analysis of heat-treated samples were performed as followings.

The fusion constructs and the *lacZ* expression vector pAOH5 were introduced into a *lacZ*-deletion *E. coli* strain ER2267 (NEB) by the standard transformation procedure as previously described.

The cultures of ER2267 (50 ml), ER2267/pAHO5 (50 ml), pVT142 or pDVS712 plasmid (each in 1 liter) were grown at 30°C in LB media, supplemented with ampicillin at 100 µg/ml for plasmid-containing cells. When OD₆₀₀ₙₘ reached between 0.48 and 0.55, inducer IPTG was added into the cultures to 0.3 mM final concentration and the cultures were incubated at 23°C for 3 additional hours. Cells were spun down, resuspended in 50 ml (for ER2267 or pAHO5-bearing ER2267) or 100 ml (for pVT142- or pDVS712-bearing ER2267) LB media, sonicated for 3 min at 4°C and spun at 7000 rpm for 10 min. The supernatants were stored at -20°C. Three 5 ml aliquots of each extract were incubated and sampled at 23°C, 42°C or 50°C for 16 hours. Aliquots of 0.9 ml were transferred into 1.5 ml microfuge tubes after incubation for 1, 2, 3, 4, 6 hours. 5 µl of untreated or treated extract was mixed with 10 µl of water and 5 µl of 5 × sample buffer (0.31 M Tris-Cl, pH6.8/10%SDS /25% 2-mercaptoethanol /50% glycerol/0.005% Bromophenol blue) and boiled for 10 min.

5 µl of each sample was loaded on a 4/20% SDS polyacrylamide and electrophoresed at 100 volts for 3-4 hours. Western blots, using antibody raised against ß-galactosidase (Promega) and antibody raised against endonuclease I-Tli-I or I-*Psp*l (NEB), were carried out according to the procedure of Promega. The results showed barely trace amounts of endonuclease present in cells after IPTG induction at 23°C from both pVT142 and pDVS712 constructs, indicating inefficient excision activity, if any. However, after shifting the ER2267/pVT142 extract to higher temperatures, 42°C or 50°C, abundant IVPS2 product (I-Tli-I about 42 KDa), identical to the excised endonuclease from the Vent DNA polymerase precursor, was accumulated (Figure 4). A similar pattern was observed for pDVS712/ER2267 extract treated at 42°C or 50°C (Figure 4), resulting in accumulation of a product of about 60 KDa, expected for the Deep Vent IVPS1 product, I-PspI.

Western blot analysis using antibody against ß-galactosidase indicated that excision of the IVPS domains was coupled with ligation or rejoining of the N-domain and the C-domain of the interrupted ß-galactosidase. The heat-treated samples of both fusion constructs contained a product of 114 KDa, identical in size to full-length ß-galactosidase (Figure 4). However, this product was only accumulated in small amount in the samples of pVT142, indicating that splicing from this fusion protein is inefficient under these conditions.

The fusion proteins were further tested for their ability to splice at higher temperatures, up to 80°C. The initial reaction rates at different temperatures were compared. The extracts were incubated in 300 µl aliquots in 1.5 ml-microfuge tubes at 42°C, 50°C, 65°C or 80°C. 20 µl were taken from each heated extract sample at 15 and 30 min and 1, 2, and 4 hours, and mixed with 40 µl of water and 20 µl of 5 × sample buffer and boiled for 10 min. Western blot analysis showed that Deep Vent IVPS-ß-galactosidase fusion protein was able to splice at 65°C and at 80°C, although splicing seems more efficient at 65°C as measured by the accumulation of the 114 KD product. Excision of the Vent IVPS2 was efficient at 65°C but seems blocked at 80°C. Lack of accumulation may be due to thermal denaturation and precipitation of ß-galactosidase at 80°C with time.

### EXAMPLE 4

### IN-FRAME INSERTION OF IVPS IN A PCR GENERATED LINEAR PLASMID. SUCH AS ONE ENCODING ß-AGARASE I

In Example 2, we described inserting the IVPS cassettes from Example 1 into a restriction enzyme linearized plasmid. This method is limited by the availability of appropriate restriction enzyme sites in a target gene. PCR amplification using opposing primers on a circular plasmid allows linearization of any plasmid at any position, limited only by the capacity of the PCR reaction. Once the target plasmid is linear, the process is essentially the same as described in Example 2 for restriction enzyme generated linear plasmids.

As described in Example 2, insertion of an IVPS cassette into a target gene can be accomplished by ligation of an IVPS fragment with linear plasmid. In this example, PCR primers are used to generate plasmids linearized just prior to a serine or threonine codon. Thus, when the IVPS is excised and the two halves of the target protein are ligated, no extra amino acid is left behind in the target protein. The serine or threonine at the insertion site can be positioned on either the IVPS fragment or on the target gene fragment. If the serine or threonine is present on the IVPS cassette, then the target gene PCR primer can be constructed with a deletion of the 3 nucleotides encoding the first residue of the downstream EPS. If the IVPS cassette lacks the serine or threonine codon, then PCR with opposing, abutting PCR primers is used to synthesize target plasmid linearized at the serine or threonine codons.

This example describes cloning two IVPS elements, Vent IVPS2 and Deep Vent IVPS1, into a gene encoding ß-agarase I (Yaphe, W., *Can. J. Microbiol.* 3:987-993 (1957)) by the procedure described in Example 2. The Deep Vent IVPS1 is inserted in front of a serine, the 108th codon, of the 290 amino acid ß-agarase I gene, while the Vent IVPS2 is inserted in front of a threonine, the 133th codon of the ß-agarase I gene.

The IVPS DNA fragments, including the serine codon (for Deep Vent IVPS1) or the threonine codon (for Vent IVPS2) at the 3' end, were prepared as described in Example 1. pAG6a1 (NEB), a 3.8 Kb recombinant plasmid containing the ß-a arase I gene sequence in vector pUC18 in the orientation of lac promoter, was used as a PCR template to synthesize linear plasmid DNA fragments. Primers agaS108.rv (5'-GAGAACTTTGTTCGTACCTG-3' (SEQ ID NO:8)) and agaS108.fw (5'-GGTATTATTTCTTCTAAAGCA-3' (SEQ ID NO:9)) are compementary to DNA sequence 5' and 3' of the 108th codon, respectively. Primers agaT133.rv (5'-GTTGTTTGTTGGTTTTACCA-3' (SEQ ID NO:10)) and agaT133.fw (5'-ATGGCAAATGCTGTATGGAT-3' (SEQ ID NO:11)) are complementary to sequence 5' and 3' of the 133th codon, respectively. Each pair of the primers was used to synthesize linear plasmid DNA fragments, lacking the serine or threonine codon. The PCR mixture contained Vent DNA polymerase buffer (NEB), supplemented with 2 mM Magnesium sulfate, 400 µM of each dNTP, 0.5 µM of each primer, 20 ng plasmid DNA and 2 units of Vent DNA polymerase in 100 µl. Amplification was carried out using a Perkin-Elmer/Cetus thermal cycler at 94°C for 30 sec, 45°C for 30 sec and 72°C for 5 min for 30 cycles. The PCR samples were extracted with phenol and chloroform, precipitated in 0.3 M NaAcetate and 50% isopropanol, recovered by spinning at 10 Krpm for 10 min in a microfuge, dried and resuspended in 100 µl of distilled water. The DNA samples were then electrophoresed on a 1% low melting agarose gel and PCR-synthesized fragments were recovered as described in Example 1.

Ligation of PCR-synthesized fragment with phosphorylated IVPS fragment (Example 1) was carried out at 16°C for 12 hours in 20 µl volume with addition of 9.5 µl distilled water, 2 µl of 10 X T4 DNA ligase buffer (NEB), 4 µl of 0.01 µg/µl PCR-synthesized plasmid DNA, 4 µl IVPS DNA (0.20 µg of Vent IVPS2 or 0.32 µg Deep Vent IVPS1) and 0.5 µl of 400,000 M/ml of T4 DNA ligase (NEB). Transformation of *E. coli* strain RR1 with the ligation samples was performed as described in Example 2. Transformants were cultured in LB medium, supplemented with 100 µg/ml ampicillin, for extraction of plasmid DNA using alkaline lysis method (Sambrook et al., Molecular Cloning; A Laboratory Manual (1989), Cold Spring Harbor Laboratory press, Cold Spring Harbor, New York). Plasmid DNAs were compared with pAG6a1 by electrophoresis on a 0.8% agarose gel followed by staining with ethidium bromide. Recombinant plasmid pAG108S18 contains the Deep Vent IVPS1 insert while pAG133T22, 26, 31 and 35 all contain the Vent IVPS2 insert.

### EXPRESSION OF THE IVPS-β-AGARASE I FUSION GENES

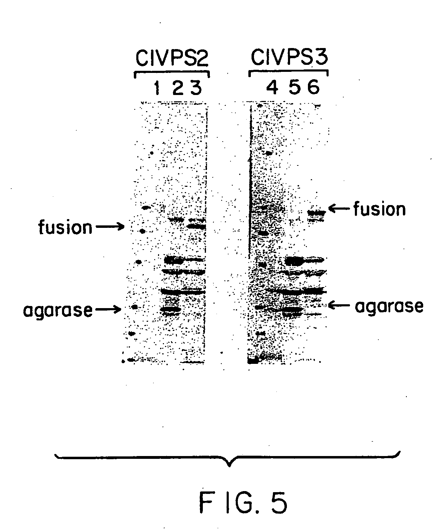

The clones were further examined by their ability to express fusion proteins. RR1 cells carrying pAG108S18 or pAG133t35 were cultured in 1 liter of a modified LB medium, lacking dextrose, supplemented with 100 ug/ml ampicillin, at 30°C until OD₆₀₀ₙₘ reached about 0.5. After addition of inducer IPTG to a final concentration of 0.3 mM, the cultures were cooled down and grown at 25°C for 4 additional hours. Cells were spun down and resuspended in 50 ml LB medium. Crude extracts were prepared as described in Example 3. Western blots using antibodies raised against I-Tli-I (NEB), I-*Psp*l (NEB) and ß-agarase I (NEB) were performed to detect fusion (modified) proteins expressed from these clones. Samples were electrophoresed on 4-20% SDS gels (ISS, Daichi, Tokyo, Japan) with prestained markers (BRL), transferred to nitrocellulose, probed with antisera (from mouse), and detected using alkaline phosphatase-linked anti-mouse secondary antibody as described by the manufacturer (Promega). Both anti-I-*Psp*I sera and anti-ß-agarase I sera reacted with a 90-95 KDa product expressed from pAG108S18/RR1, of the expected size for a Deep Vent IVPS1 (approximately 60 KDa) - ß-agarase I (approximately 30 KDa) fusion protein (Figure 5). Both anti-I-Tli-I sera and anti-ß-agarase I sera reacted with a 70-75 KDa product,from pAG108S18/RR1, approximately the size expected for a Vent IVPS2 (42KDa)- ß-agarase I fusion protein (Figure 5).

### EXAMPLE 5

### INSERTION OF IVPS INTO TARGET GENE BY CREATION OF NEW RESTRICTION ENZYMES SITES THROUGH SILENT SUBSTITUTIONS

In the previous examples, an IVPS cassette containing the entire IVPS sequence, with or without the first downstream EPS codon, was inserted into a blunt, linearized plasmid. It is also possible to create a restriction site by silent mutations (preserving the amino acid residue) near the ends of either the IVPS or the target gene.

### CREATION OF A RESTRICTION SITE NEAR THE END OF THE IVPS

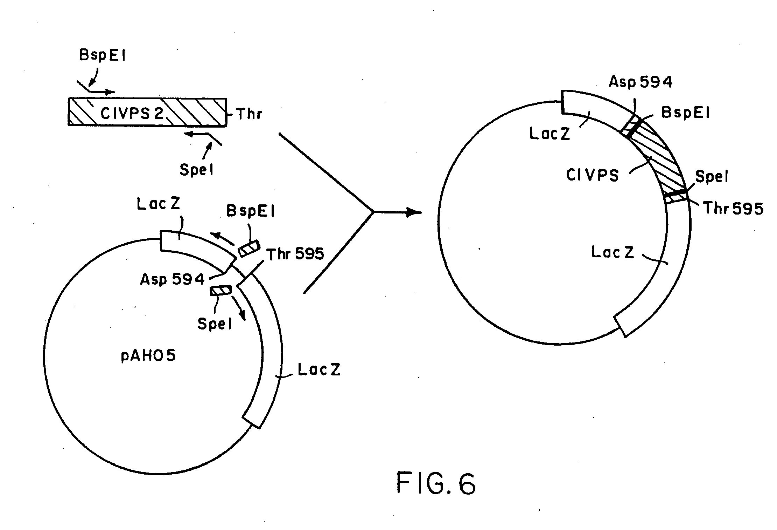

It is possible to create a restriction site by silent mutations (preserving the amino acid residue) at both ends of an IVPS to facilitate insertion of the IVPS at any position within the target gene. After creation of the new restriction sites, the IVPS is cut with these enzymes. The target gene plasmid is generated by PCR. Since the restriction sites are within the IVPS, one must include the missing IVPS sequences on the 5' end of the respective target gene PCR primers to complete the IVPS and to generate compatible cloning sites in the target gene (Figure 6).

For example, silent mutations in Vent IVPS2 can create a *Bsp*EI site at the 5' end using primer Vent IVS2 Forward BspEI (5'-AGTGTCTC CGGAGAAAGTGAGAT-3' (SEQ ID NO:12)) and a *Spe*l at its 3' end, by using primer, Vent IVS2 Reverse *Spe*l (5'-ATTGTGTACTAGTATGTTGTT TGCAA-3' (SEQ ID NO:13)). It can then be inserted, for example, between an aspartic acid codon (residue 594) and a threonine codon (residue 595) within the *lac*Z coding region. A linear target gene plasmid can be generated by PCR as described in Example 4 with primers which include the *Bsp*EI and *Spe*l sites, the remaining portion of the IVPS and a region with identity to *lac*Z using primer, *lac*Z1/*Bsp*E1 reverse (5'-GCCTCCGGAGACACTATCGCCAAAATCACCGCCGTAA-3' (SEQ ID NO:14)) and primer, *lac*Z2/*Spe*I forward (5'-GCCACTAGTACAC AATACGCCGAACGATCGCCAGTTCT-3'(SEQ ID NO:15)). DNA fragments are synthesized from both the IVPS and the target gene by PCR. Both IVPS and target gene primers contain the new restriction sites. After cutting with the appropriate restriction endonucleases, DNA fragments with compatible ends can then be ligated to create a fusion gene. Since no extra residue would be left after excision of the IVPS, native ß-galactosidase polypeptide would be expected to form if splicing occurs.

Insertion of IVPS at restriction sites near the insertion site.

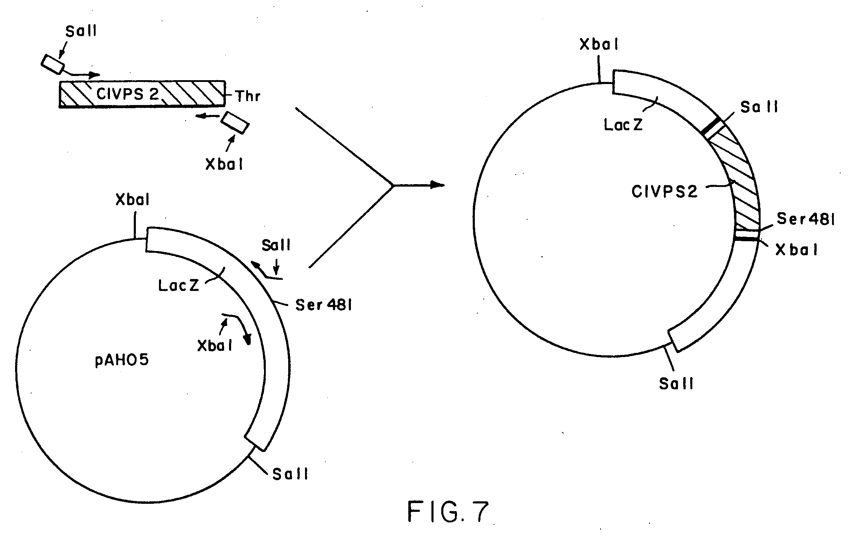

In another general approach (Figure 7), a restriction site near the insertion site in the target gene (for example, a threonine or a serine codon), can be used to insert an IVPS with ends compatible to the target gene. Restriction site(s) can be created by silent nucleotide substitution at or near the insertion site or native restriction sites can be used. A linear target gene plasmid is made by PCR as described in Example 4, beginning at the restriction sites near the insertion site. The IVPS is synthesized with primers containing the compatible restriction sites and the remainder of the target gene sequence (the sequence between the restriction site and the insertion site). The IVPS DNA fragment, with the ends overlapping the sequence at the insertion site, can be synthesized, cut with the appropriate enzyme(s), and then ligated to the vector that is cut by the same enzyme(s).

For example, IVPS elements can be inserted between residue 479 (aspartic acid) and 481 (serine) within the *lac*Z gene by creating a SalI site (residues 478-479) and a *Xba*l site (residues 481-482 serine-arginine) by silent mutations. This can be achieved by PCR of the target plasmid, pAHO5, described in example 2, using primers, *lac*Z3 Sal reverse (5'-AGGGTCGACAGATTTGATCCAGCG-3' (SEQ ID NO:7)) and *lac*Z4 Xba forward (5'-CCTTCTAGACCGGTGCAGTATGAAGG-3' (SEQ ID NO:16)). Next the IVPS2 fragment is generated by PCR using primers, Vent IVS2 Forward *Sal*I (5'-GCCGTCGACCCTAGTGTCTCAGGAGAAA GTGAGATC-3' (SEQ ID NO:17)) and Vent IVS2 reverse XbaI (5'-GCCTC TAGAATTGTGTACCAGGATGTTGTTTGC-3' (SEQ ID NO:18)). DNA fragments are synthesized from both the IVPS and the target gene by PCR. Both IVPS and target gene primers contain the new restriction sites. Unfortunately, this vector also contains single *Xba*l and *Sal*I sites (Figure 7). Therefore, the target gene vector PCR product must be cut under conditions which produce partial digestion. The required linear plasmid is then isolated from agarose gels. After cutting with the appropriate restriction endonucleases, DNA fragments with compatible ends can then be ligated to create a fusion gene. Since no extra residue would be left after excision of the IVPS, native ß-galactosidase polypeptide would be expected to form if splicing occurs. Generally, it is important to select or create an unique site within the target gene and vector to facilitate the cloning process as described above.

### EXAMPLE 6

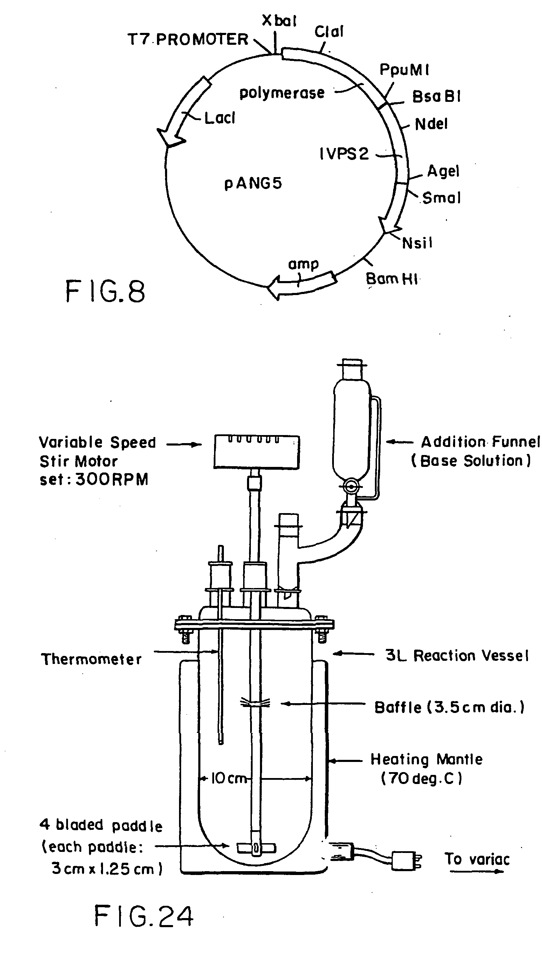

A. To facilitate experimentation on the splicing of IVPS2 in Vent DNA polymerase, a modified version of the T7 promoter construct pV174-1B1 was created. This modified version, pANG5 (Figure 8), encodes a Vent DNA polymerase precursor identical to that of pV174-1B1. Numerous silent mutations were introduced to simplify the generation of mutants as discussed in this application, particularly at the upstream and downstream splice junctions. Changes included:
   1. Destroying *Xma*l and *Ppu*MI sites in the vector backbone. The *Xmal* site was removed first by cutting the T7 expression vector pAII17 with *Xma*l, repairing the cohesive ends with the Klenow fragment of DNA polymerase I, and then religating the blunt termini. Plasmids were screened for resistance to cleavage by *Xma*l. The *Ppu*MI site was similarly removed from the resulting vector, screening this time for resistance to *Ppu*MI cleavage. The final vector was named pAML1. This vector allowed the use of unique *Xma*l and *Ppu*MI sites within the polymerase gene.
   2. Introduction of silent base changes to create restriction sites. Changes were introduced using oligonucleotide-directed mutagenesis as described by Kunkel (T.A. Kunkel, J.D. Roberts and R.A. Zakour, *Methods in Enzymology* 154:367-382 (1987)). Single-strand templates were created in two Bluescript SK-phagemid derivatives by superinfection with the f1 helper phage IR1 (Enea, et al., *Virology* 122:22-226 (1982)). The first contained a *Bsa*AI to BamHI fragment (representing nucleotides 3714-5837 of the Vent DNA polymerase sequence) from pV174-1B1 ligated into *Bam*HI/*Eco*RV cut Bluescript. The second fragment included a *Cla*I to *Ssp*I fragment (nucleotides 816-4408) ligated into *Clal*I/*Eco*RV cut Bluescript.
      The *Bsa*AI/*Bam*HI construct was mutagenized simultaneously with three oligonucleotides: 
5'-GCAAAGAACCGGTGCGTCTCTTC-3' (SEQ ID NO:19) (AgeI nt
4669-4674)

5'-AGCAACAGAGTTACCTCTTG-3' (SEQ ID NO:20)
(amber1703ochre)

5'-CAGTTTCCAGCTCCTACAATGAGACCTACGAGC-3' (SEQ ID
NO:21) (D1236A)
 where modified bases are underlined, and changes are indicated in parenthesis. The oligonucleotide to create D1236A also included silent base changes to create a BsaI site to assist in screening. The resulting isolate was named pAMN2.
      The *Cla*I/*Ssp*I construct was mutagenized simultaneously with four oligonucleotides: 
5'-GTAGTGTCGACCCCATGCGG-3' (SEQ ID NO:22) (SalI nt
3863-3468)

5'-CGTTTTGCCTGATTATTATCTCACTTTC-3' (SEQ ID NO:23)
(BsaBI nt 3554-3563))

5'-GTCCACCTTCGAAAAAGATCC-3' (SEQ ID NO:24) (BstBI nt
3608-3613)
 
5'-CCGCATAAAGGACCTTAAAGC-3' (SEQ ID NO:25) (PpuMI nt
3517-3523)
 where markings are as above. Screening was also as above, with the resulting construct was named pAM022.
      The *Bsa*AI/*Bam*HI construct was also mutagenized with the oligonucleotide: 
5'-GAGGAAGAGATCATCATCATAGC-3' (SEQ ID NO:26) (BsaBI
blocking nt 5641)
 and screened for resistance to *Bsa*BI cleavage due to the addition of a dam methylation site. The resulting construct was named pAMW3.
      Finally, the Ndel site at the initiation codon of pV174-1B1 was inactivated by partial *Nde*I cleavage, repairing the termini with Klenow, and recircularizing using T4 DNA ligase. Plasmids were screened for the loss of the appropriate *Nde*I site. One such construct was named pAKC4.
      The pANG5 construct was assembled from the above parts:
      *1. Xba*I/*Cla*I from pAKC4 (translation initiation and amino terminus of vent DNA polymerase)
      *2. Cla*I/*Nde*I from pAM022 (more amino terminal polymerase plus the amino terminal region of IVPS2)
      *3. NdeI*/*NsiI* from pAMN2 (carboxyl terminal region of IVPS2, carboxyl terminal region of vent DNA polymerase)
      *4. Nsi*I/*Bam*HI from pAMW3 (final 5 amino acids of the polymerase plus the downstream region)
      5. *Bam*HI/*Xba*I from pAML1 (T7 promoter, origin of replication, ampicillin resistance).
      Comparisons between pANG5 and the parent pV174-1 B1 show identical patterns of Vent DNA polymerase and I*- Tli*I production, with the exception of the greater viability of the pANG5 containing strains, as discussed below. This is as expected if splicing occurs at the protein level, as opposed to at the RNA or DNA level.
   B. During work on the expression of the Vent DNA polymerase gene in *E. coli* it was found that a large increase in expression and cell viability occurred after deletion of IVPS1 and IVPS2. This increase could either represent toxic effects of I-*Tli*II and I-*Tli*I, the gene products of IVPS1 and IVPS2, respectively, or toxic effects of the splicing reaction itself. It was reasoned that endonuclease and splicing activities could well be independent, allowing inactivation of the endonuclease without affecting splicing. A single amino acid substitution to A as described in the construction of pANG5 was made in a conserved residue within the amino-proximal dodecapeptide motif of I-*Tli*I (changed residue D1236). Although these constructs expressed Vent DNA polymerase, no I-*Tli*I activity was detected. Unlike pV174-1B1, T7 expression strains such as BL21 (DE3) tolerated pANG5 well, even at 37°C. Analysis of protein splicing by western blot and pulse-chase analysis showed no discernible differences in protein splicing between pANG5 and pV174-1B1, namely production of a full-length precursor and subsequent formation of the mature polymerase and a protein corresponding in size to I-*Tli*I.
   C. A consensus calmodulin-dependent protein kinase II site (XRXXS*; Pearson et al., *supra*) was constructed, replacing tyrosine 1079 with arginine using cassette replacement mutagenesis. In short, pANG5 was cut at the unique sites *Bsa*BI and *Ppu*MI and the duplex (SEQ ID NO:27) listed below was inserted, introducing the desired change. 
5'-GTCCTTCGTGCGGACAGTGTCTCAGGAGAAAGTGAGATAA-3'
3'-GAAGCACGCCTGTCACAGAGTCCTCTTTCACTCTATT-5'
 The correct construct was verified by DNA sequencing.
   D. Introduction of an amber stop codon for adding a blocked amino acid was accomplished by cassette replacement mutagenesis in pANG5. For example, serine 1082 was replaced by an amber codon using the following duplex (SEQ ID NO:28) inserted into pANG5 cut with *Ppu*MI and *Bsa*BI: 
5'-GTCCTTTATGCGGACTAGGTCTCAGGAGAAAGTGAGATAA-3'
3'-GAAATACGCCTGATCCAGAGTCCTCTTTCACTCTATT-5'.
 Similarly, tyrosine 1472 was replaced with an amber termination codon by placing the following duplex (SEQ ID NO:29) into pANG5 cut with *Agel* and *Smal*: 
5'-CCGGTTCTTTGCAAACAACATCCTGGTACACAATTAAGACGGC
3'-AAGAAACGTTTGTTGTAGGACCATGTGTTAATTCTGCCG
TTTTATGCCACAATACCC-3'
AAAATACGGTGTTATGGG-5'
 Finally, since the Vent DNA polymerase gene ends in an amber codon (TAG), that termination codon will be changed to an ochre codon (TAA) by inserting an appropriate restriction fragment from pAMN2 (described above) into the corresponding site in pANG5.

### EXAMPLE 7

### CONTROL OF PROTEIN SPLICING BY INCORPORATION OF 0-(O-NITROBENZYL) SERINE AT THE SPLICE JUNCTION OF CIVPS2

Two vectors were constructed using pV174.1B1 to demonstrate photoactivatable protein splicing. The first construct, pANY5 (also referred to as "wild-type"), can be described on the amino acid level as follows: pV174.1B1 Δ1-1063, Δ1544-1702, V1542M, V1541M, 1543opal(TGA). This construct is designed to give a 55.8 kDa precursor protein, which splices out the 45.3 kDa endonuclease (I-*Tli*l) and yields a 10.5 kDa ligation product, when translated in an *in vitro* transcription/translation system. The second construct, pAOD1 (also referred to as the "amber mutant"), can be described on the amino acid level as follows: pV174.1B1 Δ1-1063, Δ1544-1702, V1542M, V1541M, 1543opal(TGA), S1082amber(TAG). This construct is designed to give a 2.2 kDa amber fragment under standard *in vitro* transcription/translation conditions, but will incorporate a photoactivatible serine when the *in vitro* reaction is supplemented with an amber suppressor tRNA that has been chemically aminocylated with o-nitrobenzylserine. With the serine at position 1082 "blocked", the precursor is unable to splice. When irradiated with intense 350 nm light, the o-nitrobenzyl group is released (Pillai, *supra*), the nuceophilic hydroxyl side chain of serine is freed, and the protein is able to splice.

The amber suppresssor tRNA (lacking the 3' terminal CA residues) was synthesized on milligram scale by *in vitro* runoff transcription of *Fok*l-linearized pYPhe2 plasmid template with T7 RNA polymerase as described (Ellman, et al., *supra;* Noren, et al., *Nucleic Acids Res.* 18:83 (1990)). Serine derivatives protected at the a amine with functionalities like BPOC, CBZ, or BOC are available from commercial sources (Bachem, Sigma, Aldrich). N-blocked serine can be converted to N-blocked 0-(o-nitrobenzyl) serine by a standard alkyl halide substitution reaction with a reagent such as o-nitrobenzylbromide. The fully blocked serine was then coupled to 5'-phosphoxyribocytidylyl-(3'-5')-riboadenosine (pdCpA) as described (Ellman, et al., *supra*). The aminoacylated dimer was then ligated to the truncated suppressor tRNA with T4 RNA ligase (New England Biolabs, Inc.) to yield full-length aminoacylated suppressor tRNA.

*In vitro* transcription/translation of the "wild-type" construct was carried out by combining on ice: 3 µg cesium chloride-purified plasmid DNA, 3 µl 100 mM magnesium acetate, 1 µl 100 mM calcium acetate, 7.5 µl low molecular weight mix (Ellman, et al., *supra*) (no calcium or methionine), 1 µl (³⁵S)-methionine (10 µCi/µL, 1000 Ci/mmol), 1 µl 3 mg/ml rifampicin, and water to 30 µL. The reactions were incubated for 3 minutes at 37°C while an aliquot of S-30 extract prepared from *E. coli* D10 (Ellman, et al. *supra*) was thawed. 8.5 µl of S-30 extract was added, followed by 1.5 µl of T7 RNA polymerase (300 U/µL, New England Biolabs, Inc.), and the reactions were incubated 60 min. at 37°C. Samples were electrophoresed on a 10-20% tricine SDS-PAGE gel (NOVEX) and autoradiographed to visualize the proteins (Figure 9).

*In vitro* transcription/translation of the "amber mutant" was carried out as described for the "wild-type" except that the reactions were supplemented with 3.5 µl of chemically aminoaceylated o-nitrobenzylsserine-tRNA_{amber} at a concentration of approx. 3 µg/µl. The suppressor tRNA was added to the reaction immediately before addition of the S-30 extract.

Figure 9 shows a 10-20% tricine SDS-PAGE gel of *in vitro* transcription/translation reactions primed with either the "wild-type" (pANY5) or "amber mutant" (pAOD1) constructs. Lane 1 shows the 55.8 kDa precursor and excised 45.3 kDa I-T*li*l endonuclease expressed *in vitro* from the "wild-type" construct. Lane 2 shows the "wild-type" reaction supplemented with 13.5 µg of full length uncharged amber suppressor tRNA to demonstrate there is no inhibition of translation due to added tRNA. Lanes 3 and 4 show the result of *in vitro* expression of the "amber mutant" without and with full length unacylated supressor tRNA (10.5 µg) added. Neither of these reactions produce the full length precursor molecule, nor any splice products, as expected. This indicates that the suppressor tRNA is not aminoacylated by any of the endogenous aminoacyl-tRNA synthetases in the cell extract. The band of approximate molecular weight 52 kDa is apparently caused by a secondary translational initiation site just downstream from the amber mutation. Lane 5 shows the result of supplementing the "amber mutant" with the chemically aminoacylated O-nitrobenzylserine-tRNA_{amber}. Precursor protein is produced *in vitro,* but no splice products (i.e., I*-Tli*I) are visible.

Controlled splicing was achieved by photochemically removing the o-nitrobenzyl group from the serine which had been incorporated site-specifically at position 1082 of the precursor protein. A 6 µL aliquot of an *in vitro* reaction was treated with 0.51 µl of RNase A (10 µg/µl) to arrest translation, irradiated with intense (275 W) visible light from a GE model #RSK6B tanning lamp at 10 cm for 10 minutes, diluted with 4 µl of water, and then incubated at 37°C for 60 minutes to allow splicing to occur. The resulting splice products were visualized by electrophoresis on a 10-20% tricine SDS-PAGE gel followed by autoradiography (Figure 10).

Figure 10 illustrates the results of exposing the chemically blocked precursor (Lane 5, Figure 9) to 350 nm light. Lanes 1 through 4 are controls in which the "wild-type" reaction (Lane 1, Figure 9) was treated as follows. Lane 1, incubated 60 min. at 37°C; Lane 2 added 0.5 µl RNase (10 µg/µl) and incubated 60 min. at 37°C; Lane 3, irradiated 10 minutes with 350 nm light and incubated 60 min. at 37°C; Lane 4, treated with RNase as above, irradiated 10 min. with 350 nm light and incubated 60 min. at 37°C. Lanes 5-8 show the result of treating the "blocked" precursor (Lane 5, Figure 9) in the same way as for Lanes 1-4, respectively. Irradiated of the "blocked" precursor results in the excision of the I-*Tli*l (45.3 kDa) endonuclease encoded by IVPS2 (cf. Lanes 7-8 with Lanes 5-6).

### EXAMPLE 8

### IN-FRAME INSERTION OF MODIFIED IVPS INTO A TARGET GENE AND THERMAL CONTROL OF PEPTIDE BOND CLEAVAGE

In this example, we describe how an IVPS (CIVPS) cassette can be modified and inserted into a target gene. As an example, we describe modification of *Pyrococcus sp.* (or Deep Vent) IVPS1 (CIVPS3) by substitution or deletion of the first native downstream residue (serine), and in-frame insertion of the modified cassettes into the EcoRV site of the *E. coli lacZ* gene.

### MODIFICATION OF IVPS CASSETTES

In general, an IVPS cassette can be modified by substitution and deletion of residue(s) or addition of residue(s) to one or both ends of IVPS. The modified or fusion proteins using such modified IVPS cassettes may exhibit different catalytic activities, such as splicing (peptide ligation) or cleavage at a specific peptide bond.

As previously discussed, the first downstream residues at the carboxyl splice junction are serine for Deep Vent IVPS1 (CIVPS3) and Vent IVPS1 or threonine for Vent IVPS2. The first IVPS residue at the amino splice junction of CIVPS1, CIVPS2 and CIVPS3 is serine. Cysteine residues have been found at the splice junctions of the yeast TFP1 and M. tuberculosis RecA (See, Hirata, et al., *supra;* Kane, et al., *supra*; Davis, et al., *supra*). It is believed that serine, threonine or cysteine residues at splice junctions are essential for protein splicing and cleavage. The previous examples have shown that an IVPS with the first downstream residue is sufficient to contain information for protein splicing. However, these residues may function differently in various IVPS contexts. Substitutions of the native residue, for example, a serine by threonine or cysteine in the Vent IVPS2 (CIVPS2) resulted in reduced splicing and altered cleavage activity (see, Hodges, et al., *supra*).

### SYNTHESIS OF MODIFIED IVPS CASSETTES FOR IN-FRAME INSERTION INTO BLUNT SITES BETWEEN TARGET GENE CODON

IVPS cassettes for in-frame insertion into the *lacZ* coding region or any other target gene can be prepared by polymerase chain reaction (PCR). The following protocol describes the production of four Deep Vent IVPS1 cassettes without or with an additional carboxyl terminal codon, serine, threonine or cysteine, referred as CIVPS3, CIVPS3/Ser, CIVPS3/Thr and CIVPS3/Cys, respectively.

Primer 5'-AGCATTTTACCGGAAGAATGGG'TT-3' (SEQ ID NO:5) (DV IVPS1 forward, 1839-1862) and one of the four reverse primers described below were used to synthesize the cassettes from pNEB#720 (ATCC No. 68723). pNEB#720 used as template has a 4.8 Kb *Bam*HI fragment containing Deep Vent DNA polymerase gene inserted into the *Bam*HI site of pUC19. Reverse primers 5'-GCAATTATGTGCATAGAGG AATCCA-3' (SEQ ID NO:40) and 5'-GGTATTATGTGCATAGAGGAATCC A-3' (SEQ ID NO:41) (3428-3452) were used to generate CIVPS3/Thr and and CIVPS3/Cys fragments (1614 bp), respectively. The PCR mixture contains Vent DNA polymerase buffer (NEB), supplemented with 2 mM magnesium sulfate, 400 µM of each dNTP, 100 µg/ml BSA, 0.9 µM of each primer and 40 ng plasmid DNA and 2 units of Vent DNA polymerase in 100 µl. Amplification was carried out by using a Perkin-Elmer/Cetus thermal cycler at 94°C for 30 sec., 48°C for 30 sec. and 72°C for 2 min for 20 cycles. Primer 5'-ATTATGTGCATAGAGGAATCCA AAG-3' (SEQ ID NO:42) (3425-3449) was used to synthesize CIVPS3 fragment (1611 bp) by PCR as described above except the amplification was carried out for 30 cycles. Primer 5'-GCTATTATGTGCATAGAGGAAT CCA-3' (SEQ ID NO:6) (3428-3452) were used to synthesize IVPS1/Ser fragment (1614 bp) as previously described in Example 1.

The PCR samples were extracted with phenol and chloroform, and precipitated in 0.3 µM NaAc and 50% isopropanol at -20°C for 6 hours, recovered by spinning at 10 Krpm for 10 min. in a microfuge, dried and each resuspended in 20 µl of distilled water, loaded on a 1% low melting agarose gel for electrophoresis at 80 volts for 6 hours. DNA fragments were recovered from the low melting agarose gel by incubation in 0.4 ml of TE buffer (10 mM Tris-HCl/0.1 mM EDTA, pH 8.0) at 65°C for 30 min., extractions with phenol and chloroform, precipitation in 0.3 µM NaAc (pH5.2) and 50% isopropanol at -20°C for overnight. DNA was spun down, washed with 70% ethanol, dried and resspended in 10 µl distilled water.

Phosphorylation of the IVPS1 DNA fragments was performed at 37°C for 60 min. with 4 µl of 10 x polynucleotide kinase buffer (NEB), 31 µl of purified DNA, 4 µl 10 mM ATP, and 10 units of T4 polynucleotide kinase (NEB) in 40 µl. The samples were heated in a 65°C water bath for 10 min. After addition of 80 µl of TE bffer (10 mM Tris-HCl/0.1 mM EDTA, pH 8.0), the samples were sequentially extracted with phenol and chloroform. DNA was precipitated in 2.4 µM NH₄AC and 70% ethanol at -70°C overnight, pelleted by spinning at 10 Krpm for 10 min. in a microfge, washed with cold 70% ethanol, dried and resuspended in 20 µl distilled water. Phosphorylation of the CIVPS3/Ser fragment was as described above.

### IN-FRAME INSERTION OF CIVPS3 CASSETTES INTO THE ECORV SITED OF THE E. COLI lacZ GENE IN VECTOR pAH05

PCR-synthesized CIVPS cassettes can be inserted into a target coding region by ligation with linearized vector bearing the target gene. Linear plasmid vector can be prepared by restriction enzyme or PCR synthesis as previously described. pAH05 carries the entire *lacZ* gene sequence on a 3.1 kb *Bam*HI*-Dra*I fragment from pRS415 (Simons, et al., *Gene,* 53:85-96 (1987)) inserted between *Bam*HI and *Sma*l sites in the polylinker of pAGR3 (NEB) downstream of a tac promoter. The tac promoter is a transcription control element which can be repressed by the prodct of the *lacl*q gene and be induced by isopropyl ß-D-thiogalactoside (IPTG). pAH05 contains two *Eco*RV recognition seqences. *Eco*RV leaves blunt ends at its cleavage site. One of the *Eco*RV cleavage sites cuts within *lacZ* coding region between the 375th codon (aspartic acid) and the 376th codon (isoleucine).

DNA was partially digested by incubation of 15 µg of pAH05 DNA with 40 units of *Eco*RV (NEB) in 100 µl of 1 x NEB bffer 2 at 37°C for 60 min. 20 µl agarose gel loading dye was added to the sample after the sample was heated to 65°C for 10 min. to inactivate *Eco*RV. DNA fragments were separated by electrophoresis on a 1 % low melting agarose gel. Linearized pAH05 plasmid DNA was recovered from the low melting agarose gel as described in Example 8 and resuspended in distilled water.

### CONSTRUCTION OF CIVPS-lacZ FUSION GENES

Construction of CIVPS3/Ser-*lac*Z fsion was described in Example 2. CIVPS3-*lac*Z fusion was made by ligation of dephosphorylated pAH05 DNA to the phosphorylated IVPS1 fragment. The reaction was carried at 16°C for 5 hours in 20 µl volume with 1X T4 DNA ligase bffer (NEB), 0.1 µg pAH05 DNA, 0.5 µg IVPS1 DNA and 160 units of T4 DNA ligase (NEB). *E. coli* strain RR1 was transformed by mixing 100 µl of competent RR1 cells with 10 µl of ligation sample on ice for 30 min., heating at 42°C for 2 min., chilling on ice for 5 min., adding 0.8 ml LB media (10 grams/liter tryptone, 5 grams/liter yeast extract, 10 grams/liter NaCl, 1 gram/liter dextrose, 1 gram/liter MgCl₂6H₂O, pH 7.2 at 25°C) and incubating at 30°C for 45 min. The samples were plated onto LB plates, supplemented with 100 µg/ml ampicillin. After incubation overnight at 30°C, abot 150-300 colonies per plate were observed.

CIVPS3/Thr-*lac*Z and CIVPS3/Cys-*lac*Z fusions were made by ligation of 0.1 µg EcoRV-linearized pAH05 DNA with 0.7 g of CIVPS3/Thr or CIVPS3/Cys fragment. Transformation of *E. coli* strain ER2252 (NEB) was carried out by the same protocol as described above.

Colony hybridization was utilized to screen for clones that carry recombinant plasmids. The Deep Vent CIVPS3 forward primer, described above, was radio-labeled with T4 polynucleotide kinase and used as a hybridization probe. Colonies were lifted onto nitrocellulose and treated for 5 min. in each of the following soltions: 10% SDS, 0.5 M NaOH/1.5 M NaCl, 0.5 M Tris-HCl (pH 7.4)/0.5 M NaCl (twice) and 2XSSC (twice). The nitrocellulose filters were dried at room temperature for 1 hour, baked in vacuum at 80°C for 2 hours, soaked in 6 x SSC for 5 min. and washed in a solution of 50 mM Tris-HCl (pH 8.0), 1 M NaCl, 1 mM EDTA and 0.1% SDS at 42°C for 2 hours. After treatment at 42°C for 4 hors in 6 X NET, 5 X Denhardt's, and 0.5% SDS, the filters were incubated with the radiolabeled oligomer probe under the same conditions for overnight and then washed in 2 x SSC for times at 42°C for 15 min. and twice at 50°C for two min., followed by autoradiography.

The positive clones that hybridized to the oligomer probe were further examined by their ability to express fusion proteins with inducer IPTG. The clones were cultured in LB medium supplemented with 100 µg/ml ampicillin at 30°C until OD₆₀₀ₙₘ reached 0.5. After addition of IPTG to a final concentration of 0.3 mM, the cultures were grown at 30°C for 4 additional hours. Crude lysates were prepared by boiling 0.1 ml of cells with 0.1 ml of the urea lysis buffer for 10 min. The identity of the fusion proteins from the positive clones described above was analyzed by Western blots using antibody raised against ß-galactosidase (Promega) or I*-Psp*I (the protein product of Deep Vent CIVPS3, NEB). Samples were electrophoresed on 4-20% SDS gels (ISS, Daichi, Tokyo, Japan) with prestained markers (BRL), transferred to nitrocellulose, probed with antisera (from mouse), and detected using alkaline phosphate-linked anti-mouse secondary antibody as described by the manufacturer (Promega). Deep Vent CIVPS3-*lacZ* fusion clones expressed a product, reacting with both antibodies, of 173-178 KDa, the expected size for the CIVPS3-ß-galactosidase fusion proteins (Figure 11). Clones pDV7 and pDV15 contain CIVPS3 insert. pDVC302, 306 and 307 carry the CIVPS3/Cys cassette while pDVT319, 321, 322 and 323 contain the CIVPS3/Thr cassette. pDVS712 and 742 containing the CIVPS3/Ser insert were previously described in Example 2.

### THERMAL CONTROL OF SPECIFIC PEPTIDE BOND CLEAVAGE IN CIVPS3-ß-GALACTOSIDASE FUSIONS USING MODIFIED CIVPS3 CASSETTES

The DVIVPS1 (CIVPS3)-ß-galactosidase fusions containing cassettes with a threonine or cysteine to substitute the serine at the carboxyl termini exhibit thermal-controllable cleavage at a specific peptide bond in the fusion proteins. The constructs described above (CIVPS3 cassettes inserted into the *lacZ Eco*RV site) yield fusion proteins after Induction by IPTG. Cell extracts prepared from cells grown at 25°C were treated at elevated temperatures (42°C or 65°C) and analyzed by Western blots using antibody against ß-galactosidase (Promega) or I*-Psp*I (the product of Deep Vent CIVPS3) (Figures 11 and 12). The IVPS1/Ser fusion protein can undergo protein splicing to generate a ligated protein and free IVPS endonuclease by incubation at elevated temperatures. While no ligation activity was observed, the fusion proteins with the CIVPS3/Thr or CIVPS3/Cys cassette cleave dominantly at the amino splice jnction at 42°C and both fuuion proteins exhibit increased cleavage activity at the carboxyl splice jnction at 65°C.

Preparation of cell extracts from the CIVPS3-*lac*Z fusion clones were performed as follows. All the fusion constructs originally constructed in different *E. coli* hosts were introduced into a *lacZ*-deletion *E. coli* strain ER1991 (New England Biolabs, Inc.), which did not synthesize ß-galactosidase, by the standard transformation procedure as described in Example 8. A single colony from pDV7, pDVC302, pDVT332 or pDVS712 clone was inoculated in 1.5 ml LB medium supplemented with 100 µg/ml ampicillin, incubated at 30°C until OD₆₀₀ₙₘ reached about 0.5 and induced with 0.3 mM IPTG by adding 1.5 ml of 0.6 mM IPTG, 100 µg ampicillin/ml LB at 25°C for 5 hours. 3 ml of cells were spun down and resuspended in 0.5 ml of LB, sonicated for 1 min. at 4°C and spun at 6,000 rpm for 5 min. at 4°C. The supernatants were recovered and stored at -20°C.

The cell extracts were heat-treated at 42°C or 65°C after being quickly thawed at room temperature. The untreated control sample was prepared by mixing 48 µl of extract with 12 µl of 5 x sample buffer (0.31 Tris-HCl, pH 6.8/10% SDS/25% 2-mercaptoethanol/50% glycerol/0.005% Bromophenol ble), followed by boiling for 10 min. Aliquots of 48 µl were transferred into 1.5 ml microfuge tubes and incubated for 30, 60, 120, or 240 min. in a 42°C water bath, or 15, 30, 60 or 120 min. in a 65°C water bath. Each was mixed with 12 µl of 5 x sample buffer and boiled for 10 min.

The treated samples were analyzed by Western blots using antibodies raised against I*-Psp*I (NEB) (Figure 11 and 12) or ß-galactosidase (Promega) (Figure 12), 5 µl of each sample was loaded on 4/20% SDS polyacrylamide gels (ISS, Daichi, Tokyo, Japan) and electrophoresed at 100 volts for 4 hours. Western blots were carried out according to the procedure of Promega.

The results show that fusion protein precursors were the dominant species and barely trace amounts of I-PspI endonuclease were present in cells after IPTG induction at 25°C from all for fusion constructs, indicating inefficient splicing and excision activity at low temperature. However, after shifting the pDVS712 (CIVPS3/Ser-ß-galactosidase fusion) extract to higher temperatures, 42°C or 65°C, abundant CIVPS3 product, I-*Psp*I, (of about 60 KDa) accumulated (Figures 11 and 12). Excision of the IVPS domains was coupled with ligation of the N-domain and the C-domain of the interrupted ß-galactosidase, producing a product of 116 KDa, identical in size to full-length ß-galactosidase (Figure 12). Another major product (IVPS1-C-EPS) of about 130 KDa (corresponding to cleavage at the amino splice junction) was observed.

The fusion proteins of the other three variants (with CIVPS3, CIVPS3/Cys and CIVPS3/Thr cassettes) were more stable at low temperature. Very little I*-Psp*I or other products corresponding to cleavage at splice junctions were detected from the untreated extracts (Figure 11). In contrast to the CIVPS3/Ser fusion, no ligated proteins were observed from the heat-treated samples of these three fusion constructs (Figure 12). The pDV7 (CIVPS3-ß-galactosidase fusion) sample produced only trace amounts of I*-Psp*I and products corresponding to cleavage at single splice junctions at 65°C, indicating poor excision at either splice junction (Figure 11, lanes 1-3). pDVC302, containing CIVPS3/Cys cassette, showed accumulation of moderate amounts of I*-Psp*I and CIVPS3-C-EPS species at 42°C (Figure 11, lane 5). The yield in *I-Psp*I*,* C-EPS and a product (N-EPS-CIVPS3) of about 110 KDa, corresponding to cleavage at the carboxyl splice junction, was increased at 65°C while CIVPS-C-EPS species is reduced (Figure 11, lanes 4-6; Figure 12). The results indicate that the peptide bond cleavage at the carboxyl splice junction from the fusion protein and/or CIVPS-C-EPS product was enhanced. pDVT321 (with CIVPS3/Thr cassette), when treated at 42°C, showed very little I-*Psp*l or C-EPS but a dominant product, CIVPS3-C-EPS (Figure 11, lane 8; Figure 12). The data indicates efficient cleavage of the peptide bond at the amino splice junction but not at the carboxyl splice junction at 42°C. The accumulation of small amount of I*-Psp*I at 65°C indicated that cleavage at the carboxyl splice junction is enhanced (Figure 11, lane 9).

In summary, the data has demonstrated that by substitution of a single native reside, serine, at the carboxyl splice junction of the Deep Vent IVPS1 (CIVPS3), processing of the fusion proteins is altered and can be better controlled by temperature. The CIVPS3/Thr-ß-galactosidase fusion protein (and CIVPS3/Cys fusion protein at a lesser extent) efficiently cleaved the specific peptide bond at the amino splice junction only at elevated temperatures.

### EXAMPLE 9

### CONSTRUCTION AND PURIFICATION OF MIP

### PURIFICATION OF CIVPS FUSIONS BY AFFINITY CHROMATOGRAPHY CLONING OF THE DEEP VENT IVPS1 INTO AN MBP FUSION PROTEIN

In one embodiment of the present invention a three-part fusion can be generated comprising a CIVPS; a segment which can be easily purified, e.g., a binding protein; and a protein or peptide of interest, i.e., a target protein. The order of these parts can be varied. The advantage of such a fusion is that it can be easily purified. Once the precursor protein is purified, the peptide of interest can be separated from the fusion by unidirectional protein cleavage induced by a modified CIVPS. In previous Examples, we have shown that if one of the CIVPS junctions is modified to reduce or prevent splicing or cleavage at that junction, then cleavage at the other junction will be favored over splicing (see, pages 13 and 14 and Example 8). This allows for separation of the peptide of interest away from the fusion.

This Example demonstrates that such a 3-part fusion composed of a binding protein, maltose binding protein (MBP), CIVPS3 and a paramyosin peptide can be easily purified on an amylose resin as an unspliced precursor. The precursor can then be induced to splice, in this case by thermal activation. In this Example, no attempt has been made to limit cleavage to one side of the CIVPS so as to interfere with splicing to generate only cleavage products without ligation.

### SYNTHESIS OF DEEP VENT IVPS1 INSERT (CIVPS3)

A CIVPS3 cassette was synthesized by PCR as described in previous Examples, with the following modifications. The PCR mixture contained Vent DNA polymerase buffer (NEB), 200M of each dNTP, 10pmoles of each primer, 40ng of plasmid DNA and 2 units of Vent DNA polymerase in 1001. Amplification was carried out using a Perken-Elmer thermal cycler at 94°C for 30 sec, 50°C for 30 sec and 72°C for 2 min for 20 cycles. Deep Vent IVPS1 was synthesized from pNEB #720.

The forward primer was, Primer 96-6, 5'-GGTACCCGTCGTGCTAG CATTTTACCGGAAGAATGGGTACCA-3'(SEQ ID NO:43), consisting of 26/27 bases at the 3' end which are identical to the 5' end of DV IVPS1, including 2 flanking *Kpn*I sites. The 3' *Kpn*I site includes a silent substitution which creates the restriction site without changing the amino acid residue. Deep Vent IVPS1 reverse primer, Primer 96-7, 5'-CCC GCTATTATGTGCATAGAGGGATCC-3' (SEQ ID NO:44) has a *Bam*HI site at the 3' end. 23/24 bases at the 3' end are homologous to the 3' end of DV IVPS1, with a single base substitution to create the *Bam*HI site. Primers 96-6 and 96-7 were used to synthesize the Deep Vent IVPS1 cassette (1.6kb).

The PCR sample was mixed 1:1 with chloroform and the top aqeous layer was loaded on a 1% low melt agarose gel for electrophoresis. The 1.6 kb band was excised from the gel and incubated at 65°C. After the gel melted, 0.25ml TE buffer (10mM Tris-HCl/0.1mM EDTA, pH7.5) at 65°C was added and the sample was phenol-chloroform extracted (1:1 mixtre). The DNA was precipitated in 0.5M NaCl and 2 volumes isopropanol at -20°C for 30 min. The DNA was spun down, dried and resuspended in 60µl TE bffer.

### PREPARATION OF pPR1002, A pMAL-c2-PARAMYOSIN ΔSal PLASMID

pPR1002, a pMAL-c2-paramyosin ΔSal fusion plasmid, is a 7.2 kb vector that contains a *tac* promoter driven malE gene linked to an EcoRI-*Sal*I fragment of the *D. immitis* Paramyosin gene, referred to as the paramyosin ΔSal deletion (Steel, et al., *J. Immnology,* 145:3917-3923 (1990)). Two samples of 4 µg each of pPR1002 were linearized with 6 units of *Xmn*l (NEB) in 20 µl of 1X NEB bffer #2 containing 100 µg/ml BSA at 37°C for 2 hours. The reactions were loaded onto a 1% low melting agarose gel. The 7.2 kb band was excised and purified from the gel as above, and resuspended in 40µl of TE buffer.

### CONSTRUCTION OF pMIP17

Ligation of pPR1002 and Deep Vent IVPS1 was carried out at 16°C for 16 hours in a 25µl volume with addition of 14.5µl distilled water, 2.5µl of 10X T4 DNA ligase buffer (NEB), 1µg/µg of cleaved pPR1002 DNA, 5µl of 0.2µg/µl Deep Vent IVPS1 prepared as described above and 800 units of T4 DNA ligase (NEB).

*E. coli* strain ER2252 was transformed on ice for 5 min. by mixing 100µl of competent ER2252 cells with 5µl of ligation sample in 100µl of a 1:2 mix of 0.1 MCaCl₂ and 1 XSSC (0.15M NaCl, 15mM NaCitrate), heating at 42°C for 3 min., chilling in ice for 5 min, adding 0.1 ml LB media (10 grams/liter tryptone, 5 grams/liter yeast extract, 10 grams/liter NaCl, 1 gram/liter Dextrose, 1 gram/liter MgCl₂·6H₂O, pH7.2 at 25°C) and incubating for 30 min. at 30°C. 300 µl of transformed cells were pelleted and resuspended in 100µl supernatant and plated onto an LB amp plate. After incubation overnight at 30°C, about 160 colonies were observed.

PCR amplification was utilized to screen for colonies that carried recombinant plasmids. Individual colonies were picked into 100µl of distilled water in a 96 well microtitre dish, and boiled for 5 min to lyse the cells. The PCR mixture contained Vent DNA polymerase buffer (NEB), 200µM of each dNTP, 10pmoles of each primer (same as above), 2.5µl of cell lysate and 2 units of Vent Exo⁻ DNA polymerase in a 50µl reaction. Amplification was carried out by using a Perkin-Elmer thermal cycler at 94°C for 30 sec, 50°C for 30 sec and 72°C for 2 min for 30 cycles. 10µl of each reaction was run on a 1% agarose gel. The positive clones had bands corresponding to IVPS1 (1.6kb) and one positive plasmid was designated pMIP17.

### EXPRESSION OF MIP: THE MBP-DEEP VENT IVPS1-PARAMYOSIN ΔSal FUSION

Positive clones containing pMIP17 were cultured in LB media supplemented with 100µg/ml ampicillin at 30°C until OD₆₀₀ₙₘ reached 0.5. To prepare a lysate from uninduced cells, 1.0ml of culture was pelleted and resuspended in 50µl Protein sample buffer (125mM Tris, 700mM ß-mercaptoethanol, 2% SDS, 15% glycerol and 1 mg/ml Bromophenol Blue). Samples from induced cultures were prepared as follows. After addition of IPTG to a final concentration of 1 mM, the cultures were grown at 30°C for 20 additional hours. Cells from 0.5 ml culture at 5 hours and 20 hours after induction were pelleted and then resuspended in 100µl 5X protein sample buffer. The pre-induction and 5-hour samples were frozen at -20°C for 16 hours and the 20-hour sample was frozen at -70°C for 15 minutes. To improve precursor yield, cultures were induced at 12°C-20°C and amounts of precursor determined by Coomassie Blue stained gel. All the samples were boiled for 5 minutes and the protein products were analyzed by electrophoresis in SDS-PAGE followed by Coomassie Blue staining or Western blots using antibody raised against I-*Psp*I (NEB). The samples were electrophoresed on 4-20% SDS gels (ISS, Daichi, Tokyo, Japan) with prestained markers (BRL), transferred to nitrocellulose, probed with antisera (mouse anti-I-*Psp*I), and detected using alkaline phosphate-linked anti-mouse secondary antibody as described by the manufacturer (Promega). A predicted major band at about 132kDa was observed in both the Coomassie Blue stained gels and Western blots (data not shown).

### LARGE SCALE PURIFICATION OF THE MBP-DEEP VENT IVPS1 PARAMYOSIN ΔSal FUSION ON AMYLOSE AND MONOQ COLUMNS

Single colonies were used to inoculate 4x10ml LB media supplemented with 100µg/ml ampicillin and incubated at 30°C until OD₆₀₀ₙₘ reached 0.5. These cultures were used to inoculate 4x1 litre LB media supplemented with 100µg/ml ampicillin and incubated at 30°C until OD₆₀₀ₙₘ reached 0.5. The cultures were then transferred to 12°C and induced with 1 mM IPTG overnight. The cells were pelleted and resuspended in column buffer (20mM NaPO₄ pH7.4, 200mM NaCl and 1mM EDTA), sonicated, spun down and the cleared culture lysate loaded over amylose resin (NEB Protein fusion and purification system). Fusion protein was eluted with maltose (as described by the manufacturer) and examined on an SDS-PAGE gel (Figure 13A and 13B). The amylose resin elute was further purified by chromatography on FPLC MonoQ anion exchange resin (Pharmacia). The column was washed with 0.2 M NaCl, 10 mM Tris-HCl, pH8.5 and eluted with a linear gradient of NaCl from 0.2 to 1.0 M in 10 mM Tris-HCl, pH8.5. Protein eluted between 0.4-0.6M NaCl.

Six protein bands were identified by Western blot with antibodies to MBP, I*-Psp*l and paramyosin. Two bands of apparent molecular mass 180kDa and 132kDa reacted with all three antibodies. The full length precursor should be 132kDa. The higher molecular weight band is thought to be a splicing intermediate and similar high molecular weight species have been seen with all CIVPS constructs. The excised I-*Psp*I ran at 60kDa and was only recognized by the I-*Psp*I antibody, and the spliced product (MBP-Paramyosin ΔSal, 72kDa) was only recognized by sera reactive with the MBP and Paramyosin antibodies. A band of approximately 103 kDa reacted with only the MBP and I-*Psp*I antibodies and represents the product of a single cleavage at the C terminus of the IVPS. A band of approximately 89kDa reacted with only the I*-Psp*I and Paramyosin antisera and represents the product of a single cleavage at the N terminus of the IVPS (Figure 13A and 13B).

### EXCISION AND LIGATION OF THE MBP-DEEP VENT IVPS1-PARAMYOSIN ΔSal FUSION

Amylose resin and MonoQ preparations containing several MIP-related polypeptides, including precursor (132kDa), slowly migrating species (180kDa apparent molecular mass), products of cleavage at a single splice junction (103kDa and 89kDa), and small amounts of spliced and excised products (72kDa and 60kDa) were heat-treated at 37°C for 2 hours in 20 mM sodium phosphate (pH6.0) and 0.5 M NaCl. The 132kDa precursor and 180kDa slowly migrating species decreased with time, while both the 72kDa spliced product and the 60kDa excised I-*Psp*l increased (Figure 13A and 13B).

These results indicate that not only is it possible to purify 3-part CIVPS fusions, but that it is also possible to obtain single cleavage products. Further manipulation of the CIVPS junctions can favor cleavage at either splice junctions without ligation.

### EXAMPLE 10

### MODIFICATION OF CIVPS IN MIP FUSIONS

### CONSTRUCTION OF MIP WITH REPLACEABLE SPLICE JUNCTION CASSETTES

In this Example, an MIP fusion (see Example 9) with replaceable cassettes at both splice junctions and modification of the CIVPS by cassette substitution was constructed. We also show in two cases that modified CIVPSs are capable of cleavage at predominantly a single splice junction in a thermal inducible manner.

In Example 9, we described a three part fusion, MIP, that can be generated with the following properties: a CIVPS, a binding domain which can be easily purified (MBP) and a gene of interest (Paramyosin ΔSal). Splicing of the purified fusion protein yielded two major products, the ligated protein domains, MBP-paramyosin ΔSal, and the excised CIVPS (or I*-Psp*I)*.* We reasoned that some modifications in the CIVPS may result in inhibition of the ligation reaction and enhancement of cleavage at one splice junction. This would result in separating the peptide of interest from the fusion protein by cleavage at a specific peptide bond catalyzed by a modified CIVPS. In Example 8, we have shown that cleavage at one splice junction can be enhanced by modification of CIVPS3 (substitution of the C-terminal Ser by Thr or Cys) and that these changes reduce or prevent splicing or cleavage at the other junction. In order to screen for modifications with favorable properties of controllable splicing or cleavage activity, it is necessary to introduce and analyze various mutations at the splice junctions. This could be accomplished by synthesis of the entire CIVPS cassette carrying each modification. However, this is likely to introduce extra mutations during PCR.

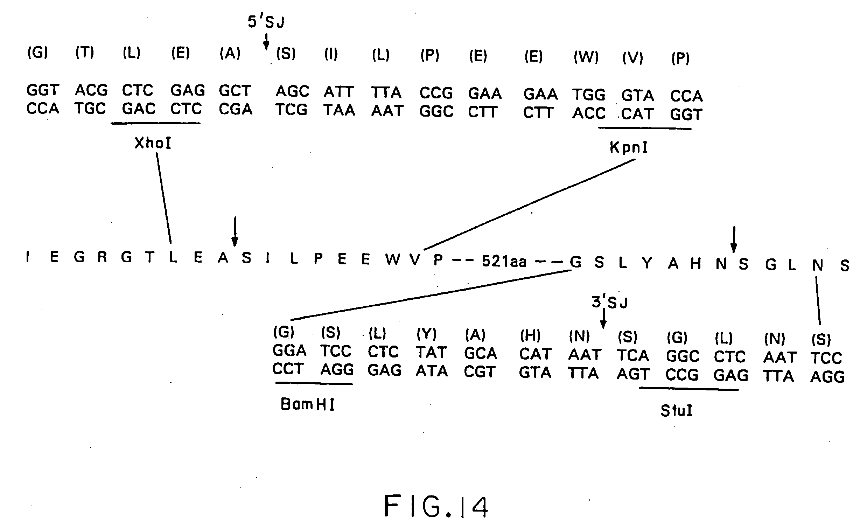

We have developed a strategy to facilitate the process by replacing only a short stretch of DNA around the splice junctions. In this Example, we describe how the original MIP fusion of Example 9 has been modified to contain two unique restriction sites flanking each splice junction. In a cassette replacement, following restriction digestion, the short stretch of DNA between the two unique restriction sites at one of the splice junctions can be replaced by another short DNA cassette. In this example, we modified the pMIP17 fusion described in Example 9 to contain two unique restriction sites at each junction: a *Xho*I site and a *Kpn*I site flanking the amino splice junction and a *Bam*HI site and a *Stu*I site flanking the carboxyl splice junction (see Figure 14).

The MIP fusion with splice junction cassettes is constructed in two steps. First, the *Bam*HI and *Stu*I sites were introduced as follows. 4 µg of pMIP17 (Example 9) was digested in 1x *Eco*RI buffer (NEB) with 0.5 units of *Eco*RI (NEB) in 50 µl at 37°C for 10 min. After electrophoretic separation in an 1% agarose gel, linearized pMIP17 plasmid DNA (8.8 Kb) was purified by using a GenecleanII kit (BIO101). The purified pMIP17 DNA was digested in 1 x *Bam*HI buffer (NEB) supplemented with 100 µg/ml BSA, 40 units of *Bam*HI (NEB) at 37°C for 3 hours and then extracted with phenol and chloroform. DNA was precipitated in 0.3 M NaAcetate (pH5.2) and 50% 2-propanol at -20°C for 2 hours. DNA was recovered by spinning for 10 min at 10,000 rpm in a microfuge, dried and resuspended in 20 µl sterile water.

Prior to ligation with the vector, two complementary oligomers, MIP301 F (5'-GATCCCTCTATGCACATAATTCAGGCCTC-3' (SEO ID NO:46)) and MIP302R (5'-AATTGAGGCCTGAATTATGTGCATAGAG G-3' (SEQ ID NO:47)) were allowed to anneal to form a double-stranded linker, MIP301 F/MIP302R. 50 pmols of oligomers MIP301F and MIP302R were incubated in 1 x T4 DNA ligase buffer (NEB) at 68°C for 15 min and slowly cooled to 20°C-30°C. 1 µg of *Eco*RI-*Bam*HI-digested pMIP17 DNA was ligated at 16°C for 14 hours in 35 µl 1x T4 ligase buffer (NEB) with 80 units of T4 DNA ligase (NEB) and 25 pmols of the linker MIP301 F/MIP302R.

The resulting construct was termed pMIP18. The upstream *Xho*l and *Kpn*l sites were introduced into pMIP18 as follows. 2 µg of pMIP18 was digested at 37°C for 4 hours in 100 µl of 1x Buffer 2 (NEB), 100 µg/ml BSA and 20 units of Kpnl (NEB). Following electrophoretic separation, linear pMIP18 DNA was purified by using the GenecleanII kit (BIO101). Prior to ligation with the vector, two complementary oligomers, MIP521F (5'-GCTCGAGGCTAGCATTTTACCGGAAGAATGGGTAC-3' (SEQ ID NO:48)) and MIP522R (5'-CCATTCTTCCGGTAAAATGCTAG CCTCGAGCGTAC-3' (SEQ ID NO:49)) were allowed to anneal to form a double-stranded linker, MIP521F/MIP522R. 50 pmols of oligomers MIP301 F and MIP302R were incubated in 1 x T4 DNA ligase buffer (NEB) at 75°C for 15 min and slowly cooled to 20°C-30°C. 0.2 µg of digested pMIP18 was ligated at room temperature for 3 hours in 35 µl of 1 x T4 DNA ligase buffer (NEB), 80 units of T4 DNA ligase (NEB) and 25 pmols of the linker MIP521 F/MIP522R. In each case, the ligated DNA samples were used to transform *E. coli* strain ER2252 (NEB). The final construct, pMIP21, contains two unique restriction sites at each splice junction. There is a *Xho*I site and a *Kpn*I site surrounding the N-terminal splice junction and a *Bam*HI site and a *Stu*I site surrounding the C-terminal splice junction (Figure 14).

Western blot analysis was performed to examine expression of modified MIP21 fusion protein and splicing activity. ER2252 containing pMIP21 was cultured at 30°C in LB medium supplemented by 100 µg/ml ampicillin until OD₆₀₀ₙₘ reached 0.5. The culture was then induced by 1 mM IPTG at 30°C for 3 hours. 4.5 ml of the culture was pelleted, resuspended in 0.5 ml LB medium and sonicated on ice. The cleared supernatant was electrophoresed on a 4/20% polyacrylamide gel at 100 volts for 4 hours. A Western blot was p. bed with anti-MBP sera. The results indicate that splicing activity from the modified MIP21 fusion was indistinguishable from that of MIP17.

### MODIFICATION OF MIP21 BY SPLICE JUNCTION CASSETTE REPLACEMENT

In the modified MIP fusion construct, pMIP21, the amino splice junction cassette includes 8 amino acid residues between the *Xho*l and *Kpn*I sites and the carboxyl splice junction cassette contains a sequence coding for 6 amino acid residues between the *Bam*HI and *Stu*l sites. Splice junctions can be changed by replacing either the N-terminal *Xho*I*-Kpn*I cassette or the C-terminal *Bam*HI*-Stu*I cassette. In the case of the C-terminal cassette replacement, pMIP21 is first digested with-*Bam*HI and *Stu*I. Complementary primers containing desired mutations are substituted for the original *Bam*HI*-Stu*I cassette. In this Example, two different junction cassettes were substituted for the MIP21 *Bam*HI*-Stu*I cassette.

In the following cassette replacement examples, we substituted Ala₅₃₅ by Lys or His₅₃₆ by Leu.

Complementary oligomers MIP303F (5'-GATCCCTCTATAAGCAT AATTCAGG-3' (SEQ ID NO:50) and MIP304R (5'-CCTGAATTATGCT TATAGAGG-3' (SEQ ID NO:51)) were used to substitute residue Ala₅₃₅ by Lys. Complementary oligomers MIP311 F (5'-GATCCCTCTATGCACT GAATTCAGG-3' (SEQ ID NO:52)) and MIP312R (5'-CCTGAATTCAGTG CATAGAGG-3' (SEQ ID NO:53)) were used to substitute His₅₃₆ by Leu. These two pairs of complementary oligomers were treated as described above to form a double-stranded linker. Both linkers contain compatible termini to replace the carboxyl splice junction cassette following *Bam*HI*-Stu*I cleavage of pMIP21. 2 µg of pMIP21 DNA was digested with 40 units of *Bam*HI (NEB) in 1 x *Bam*HI buffer (NEB) supplemented with 100 µg/ml BSA at 37°C for 4 hours, extracted with chloroform and precipitated in 0.3 M NaAcetate (pH5.2) and 50% 2-propanol at -20°C for 2 hours. DNA was recovered by spinning for 10 min at 10,000 rpm in a microfuge, dried and resuspended in 88 µl sterile water. The BamHl-digested pMIP21 DNA was then digested with 40 units of *Stul* (NEB) in 100 µl 1x Buffer 2 (NEB) at 37°C for 3 hours, extracted with chloroform, precipitated in 0.3 M NaAcetate (pH5.2) and 50% 2-propanol at -20°C for overnight. pMIP21 DNA was recovered by spinning for 10 min at 10,000 rpm in a microfuge, dried and resuspended in 30 µl sterile water. 0.1 µg *Bam*HI*-Stu*I digested DNA was ligated at 23°C for 6 hours with 6 pmols of linker MIP303F/MIP304R or MIP311F/MIP312R in 10 ul of 1x T4 DNA ligase buffer (NEB) in the presence of 40 units of T4 DNA ligase (NEB). The ligated DNA was used to transform *E. coli* RR1. pMIP23 contains the Ala₅₃₅ to Lys substitution and pMIP28 contains the His₅₃₆ to Leu substitution. Expression of the modified MIP fusions, MIP23 and MIP28, was tested by western blot analysis with anti-MBP antibody as described above. The results indicated that splicing activity was blocked in both fusion constructs. However, each modification resulted in increased cleavage activity at only one of the splice junctions. The Ala₅₃₅ to Lys substitution in MIP23 drastically enhanced cleavage activity at the carboxyl splice junction and the His₅₃₆ to Leu substitution in MIP28 showed strong amino splice junction cleavage.

### PURIFICATION OF MODIFIED MIP FUSION PROTEINS AND THERMAL INDUCIBLE CLEAVAGE ACTIVITY

Expression of the fusion proteins was induced at low temperature and MIP fusion proteins were purified by amylose resin columns. RR1 harboring pMIP23 or pMIP28 were cultured in 1 liter of LB medium supplemented with 100 µg/ml ampicillin at 30°C until OD₆₀₀ₙₘ reached 0.5. After the cultures were cooled on ice to about 15°C, IPTG was added to a final concentration of 0.3 mM, and the cultures were grown at 12°C-14°C for 12 additional hours. Cells were pelleted, immediately frozen at -70°C and stored at -20°C. The pellets were separately sonicated in column buffer (10mM Tris pH8.5, 500mM NaCl) and spun down. The cleared lysate from each MIP fusion was loaded over amylose resin (NEB Protein fusion and purification system), washed and eluted with maltose (as described in Example 9).

A purified sample of MIP23 was dialyzed in 20mM NaPO₄ (pH6.0)/500mM NaCl at 4°C. The sample was then incubated at 4°C, 37°C, 50°C, and 65°C for one hour and then electrophoresed on a 4/20% SDS-PAGE gel followed by Coomassie Blue staining (Figure 15A). The gel shows that with an increase in temperature MIP23 does not form the ligated product (MP) or the excised product (I), as the original construct does but instead accumulates the C-terminal cleavage products (MI, 103 kD and P, 29 kD).

A purified MIP28 sample was dialyzed in 20mM NaPO₄ (pH6.0)/500mM NaCl at 4°C for 1.5 hours. The sample was then incubated at 4°C, 42°C, 50°C, and 65°C for one hour and mixed with 1/5 volume of 5x Protein sample buffer (125mM Tris,700mM b-mercaptoethanol, 2% SDS,15% glycerol and 1mg/ml Bromophenol Blue). The protein products were analyzed by a 4/20% SDS-PAGE followed by Coomassie Blue staining (Figure 15A and 15B). The data indicated that splicing activity was completely blocked under these conditions. Cleavage activity at the amino splice junction was increased corresponding to the increase in temperature, yielding more MBP (M, 43 kD) and CIVPS3-paramyosin ΔSal (IP, 89 kD) at 65°C.

These results show that the splice junction cassette replacement method can be utilized to modify the splice junctions in a fusion construct and such modifications may result in drastic effects on splicing and cleavage activity. Furthermore, this data gives examples of constructs where cleavage at only one splice junction is observed in the absence of ligation and total excision of the CIVPS.

### EXAMPLE 11

### CONSTRUCTION AND PURIFICATION OF MIC

### REPLACEMENT OF FOREIGN GENE IN CIVPS FUSIONS

A three-part fusion protein (MIP), composed of a binding domain for easy purification, a splicing domain (CIVPS3), and a target protein (paramyosin), was constructed as described in Example 9. This construct was purified and shown to be able to splice by thermal activation. To test the ability of this system to accept different target proteins, paramyosin in the MIP construct was replaced by the chitin binding domain (CBD) from the *Saccharomyces cerevisiae* chitinase gene (Kuranda and Robbins, *J. Biological Chem.,* 266(29):19758-19767 (1991)). The ability of this second protein fusion to splice and form both ligated and excised products shows that this fusion method can be employed with other foreign proteins. In addition, the chitin binding domain can be used as an alternate binding protein for protein purification.

### SYNTHESIS OF THE CHITIN BINDING DOMAIN (CBD)

A chitin binding domain was synthesized by PCR as described in the previous examples, with the following modifications. The PCR mixture contained Vent DNA polymerase buffer (NEB), 200 µM of each dNTP, 10pmoles of each primer, 20ng of plasmid DNA and 1 unit of Vent DNA polymerase in 100 µl. Amplification was carried out using a Perkin-Elmer thermal cycler at 95°C for 30 sec, 55°C for 30 sec, and 72°C for 30 sec for 20 cycles. The chitin binding domain was synthesized from pCT30, a plasmid containing the *Saccharomyces cerevisiae* chitinase gene (Kuranda and Robbins, *J. Biological Chem.,* 266(29):19758-19767 (1991)).

The forward primer, primer 99-02, 5'-GTCAGGCCTCTCAGACAGT ACAGCTCGTACAT-3' (SEQ ID NO:54) has a *StuI* site (AGGCCT (SEQ ID NO:55)) at the 5' end. 22 bases at the 3' end of the primer are identical to the 5' end of the chitin binding domain of the chitinase gene. The reverse primer, primer 99-03, 5'-CCCCTGCAGTTAAAAGTAATTGC TTTCCAAATAAG-3' (SEQ ID NO:56) has a *Pst*I site (CTGCAG (SEQ ID NO:57)) at the 5' end. 26 bases at the 3' end of the primer are identical to the antisense strand at the 3' end of the chitin binding domain of the chitinase gene. Primers 99-02 and 99-03 were used to synthesize the chitin binding domain cassette (270bp).

The PCR sample was extracted with phenol-chloroform (1:1 mixture) and the DNA was precipitated in 0.5M NaCl and 2 volumes isopropanol at -20°C for 30 min. The DNA was spun down, dried and resuspended in 40µl TE buffer. (10mM Tris-HCl, 0.1 mM EDTA, ph 7.5) A digest containing 20µl of the resuspended DNA, 21µl distilled water, 5µl 10X NEB Buffer #2, 40 units *Pst*I (NEB) and 20 units *Stu*I (NEB) was then carried out at 37°C for two hours in a 50µl volume. The reaction was loaded on a 1.8% low melt agarose gel for electrophoresis. The 0.25kb *Pst*I/*Stu*I digested product was excised from the gel and incubated at 65°C until the gel melted. 0.25 ml TE buffer at 65°C was added and the sample was phenol-chloroform extracted (1:1 mixture). The DNA was precipitated in 0.5M NaCl and 2 volumes isopropanol at -20°C for 30 min, spun down, dried and resuspended in 40µl TE buffer.

### PREPARATION OF PMIP21

A *Pst*I/*Stu*I double digest separates the paramyosin coding region from the remainder of the pMIP21, described in Example 10. Two samples of 5µg each of pMIP21 were digested with 60 units *Pst*I (NEB) and 30 units *Stu*I (NEB), 5µl of NEB buffer #2, and 34µl distilled water in a 50µl volume at 37°C for two hours. The reactions were loaded onto a 1% low melting agarose gel. The 8.1 kb band was excised and purified from the gel as above, and resuspended in 40µl TE buffer.

### CONSTRUCTION OF MBP-DEEP VENT IVPS1-CBD FUSION (MIC)

The chitin binding domain was substituted for paramyosin in MIP21 as follows to create MBP-Deep Vent IVPS1-CBD constructs (MIC). 1µl of 8.1kb pMIP21 fragment, 10µl of chitin binding domain (both prepared as described above) were combined with 9.5µl distilled water, 2.5µl of 10X T4 DNA ligase buffer (NEB), and 800 units of T4 DNA ligase (NEB) and incubated at 16°C for 4 hours in a 25µl volume.

*E. coli* strain RR1 *tonA* (NEB) was transformed by (1) mixing 100µl of competent RR1 *tonA* cells with 5µl of ligation sample and 100µl of a 1:2 mix 0.1 MCaCl₂ and 1XSSC(0.15M NaCl, 15mM NaCitrate) on ice for 5 min., (2) heating at 42°C for 3 min., (3) chilling in ice for 5 min and (4) plating onto an LB amp plate. After incubation overnight at 30°C, about 200 colonies were observed.

Alkaline lysis mini-prep DNA (Sambrook, *supra)* was utilized to screen for clones that carry recombinant plasmids with the chitin binding domain. When digested with *Pst*I and *Stu*l, the positive clones had a band corresponding to chitin binding domain and a band corresponding to the vector. The restriction enzyme digests were carried out by mixing 10µl miniprep DNA, 2.5µl NEB buffer #2, 8.5 µl distilled water, 40 units *Pst*I (NEB) and 20 units *Stu*I (NEB) in a 25 µl volume at 37°C for 2 hours.

### EXPRESSION OF THE MIC FUSIONS

To verify MIC constructs, small scale protein preparations were analyzed on Coomassie Blue stained gels and western blots. The positive clones were cultured in LB Media supplemented with 100µg/ml ampicillin at 30°C until OD₆₀₀ reached approximately 0.5. To prepare lysate from uninduced cells, 1.5ml of culture was pelleted and resuspended in 25µl 5X Protein sample buffer (125mM Tris, 700mM b-Mercaptoethanol, 2% SDS, 15% glycerol and 1mg/ml Bromophenol Blue). Protein samples from induced cultures were prepared as follows. After cooling the cultures to 12°C, IPTG was added to a final concentration of 1mM and the cultures were grown at 12°C for 5 additional hours. After 2 hours of induction, a 1.5ml sample was taken and after 5 hours of induction a 3ml sample was taken. Samples were pelleted, resuspended in 50µl 5X protein sample buffer, frozen at -20°C for 16 hours, and then, thawed and boiled for 5 minutes. The protein products were analyzed by Coomassie Blue stained gels and Western blots using anti-MBP antibody (NEB). The samples were electrophoresed on 4-20%SDS gels (ISS, Daichi, Tokyo, Japan) with prestained markers (BRL), transferred to nitrocellulose, probed with anti-MBP antibody, and detected using alkaline phosphate-linked anti-rabbit secondary antibody as described by the manufacturer (Promega). A predicted major band at about 110kDa for the MIC fusion protein was observed in both the Coomassie Blue stained gels and Western blots.

### LARGE SCALE PURIFICATION OF MIC ON AMYLOSE AND MONOQ COLUMNS

Single colonies were used to inoculate 3×10ml LB media supplemented with 100µg/ml ampicillin and incubated at 30°C overnight. These cultures were used to inoculate 3x1 liter LB media supplemented with 100µg/ml ampicillin and incubated at 30°C until OD₆₀₀ reached 0.5. The cultures were then transferred to 12°C and induced with 1 mM IPTG overnight. The cells were pelleted and resuspended in column buffer (10mM Tris-HCl pH8.5, 500mM NaCl), sonicated, spun down and the cleared culture lysate loaded over amylose resin (NEB Protein fusion and purification system). Fusion protein was eluted with maltose (as described by the manufacturer) and examined on an SDS-PAGE gel. The amylose resin eluate was further purified by chromatography on FPLC MonoQ anion exchange resin (Pharmacia). The column was washed with 0.2M NaCl, 10mM Tris-HCl pH8.5 and eluted with a linear gradient of NaCl from 0.2 to 1.0M NaCl in 10mM Tris-HCl, pH8.5. Protein eluted between 0.4-0.6M NaCl. The MIC and MIP protein fusion products purified similarily on both the amylose resin and the MonoQ resin.

### EXCISION AND LIGATION OF THE MBP-DEEP VENT IVPS1-CBD FUSION

An amylose purified sample of MIC was dialyzed to 20mM NaPO₄ pH6.0, 500mM NaCl. The sample was then heat treated at 4°C, 37°C, 50°C, and 65°C for one hour and then examined on an SDS-PAGE gel. (Figure 16) The gel shows an abundance of MIC precursor, approximately 110kDa, in the 4°C sample which decreases after thermal induction. Along with the decrease in precursor, an accumulation of ligated product of approxiamtely 53kDa in size, MBP-CBD(MC), and excised product of approxiamtely 60kDa in size, Deep Vent IVPS1 (I=I-*Psp*I), is observed with the increase in temperature. Also, the gel shows that bands of the same size as cleavage products, MBP-Deep Vent IVPS1(MI), approximately 103kDa, and Deep Vent IVPS1-CBD(IC), approximately 70kDa, are present.

### EXAMPLE 12

### TRANS -SPLICING

This Example demonstrates that *in vitro* splicing can occur *in trans* between halves of a precursor protein. The position at which to split MIP (Example 9 and Xu et al., *Cell*, 75:1371-1377 (1993), the disclosure of which is hereby incorporated by reference herein) was chosen immediately upstream of a methionine residue in the native CIVPS3, although other sites might work equally well, including sites which result in gaps or overlapping CIVPS sequences. In this example, one of the MIP half proteins was insoluble and splicing *in trans* was therefore performed in urea. Partial or full denaturation should not be construed as a requirement in general, since other separation points may result in solubility of both halves and since the insoluble half can be rendered soluble for *trans* -splicing experiments under non-denaturing conditions.

### CONSTRUCTION OF MI'

A fusion of the *malE* gene (encoding MBP) with the first 249 amino acids of the *CIVPS3* gene was synthesized by polymerase chain reaction (PCR) from pMIP21 (Example 10 and Xu et al., *supra* (1993) carrying a fusion between *malE, CIVPS3* and *D. immitis paramyosin ΔSal* genes using the forward primer 5'-GGAATTCCATATGAAAATCG AAGAAGGT-3' (SEQ ID NO:58) (Nde I site underlined) and the reverse primer 5'-CGGGATCCCGTTATAGTGAGATAACGTCCC G-3' (SEQ ID NO:59) (BamHI site underlined). PCR reaction mixtures contained Vent DNA polymerase buffer (New England Biolabs, Inc.; Beverly, Massachusetts), 400 mM each dNTP, 0.84 mM primers, 5 mg/ml plasmid DNA, and 20 U/ml Vent Exo⁺ DNA polymerase (New England Biolabs, Inc.; Beverly, Massachusetts) in 50 µl. Amplification was carried out using a Perkin-Elmer Cetus thermal cycler at 94°C for 30 seconds (s), 52°C for 30 s, and 72°C for 135 s for 15 cycles. Restriction enzyme digests were performed as described by the manufacturer (New England Biolabs, Inc.; Beverly, Massachusetts). Gel purified NdeI/BamHI digested PCR products were ligated directly into gel purified BamHI/NdeI digested pAII-17 T7 vector (Perler et al., *Proc. Natl. Acad. Sci. USA,* 89:5577-5581 (1992), the disclosure of which is hereby incorporated by reference herein) to create pMI/L249 (Sambrook, Molecular Cloning: A Laboratory Manual, 2nd Edition, (1989). Cold Spring Harbor Laboratory Press, Cold Spring Harbor, NY) the disclosure of which is hereby incorporated by reference herein). *E. coli* ER2169 *pLysS* (BL21 (DE3) X P1 vir (ER1489)-->Tet^{R})McrB-)) was transformed with pMI/L249 to create NEB941. The protein produced by NEB941 was called MI' for MBP (maltose binding protein)-CIVPS3 N-terminal domain (IVPS) fusion.

### CONSTRUCTION OF I'P

Restriction enzyme digests were performed as described by the manufacturer (New England Biolabs, Inc.; Beverly, Massachusetts). Gel purified XbaI/Bpu 1102I digested pET-21 b fragments carrying the polylinker site and the 6 histidine tag sequence (Novagen; Madison, Wisconsin) were ligated directly into gel purified Bpu 1102I/XbaI digested pAII-17 T7 vector DNA (Sambrook, *supra* (1989)) to create the pPHT (Polylinker-HisTag)-T7 vector used for expression of I'P.

A fusion of the last 288 amino acids of the *CIVPS3* gene with the *D. immitis paramyosin ΔSal* gene was synthesized by PCR from pMIP21 (Xu et al., *supra* (1993)) using the forward primer 5'-GGAATTCCATATG CCAGAGGAAGAACTG-3' (SEQ ID NO:60) (Nde I site underlined) and the reverse primer 5'-ATAGTTTAGCGGCCGCTCACGACGTTGTAA AACG-3' (SEQ ID NO:61) (Not I site underlined). PCR mixtures were as described above, except in 100 µl. Amplification was carried out using a Perkin-Elmer Cetus thermal cycler at 94°C for 30 s, 52°C for 30 s, and 72°C for 105 s for 10 cycles. Gel purified NdeI/NotI digested PCR products were ligated directly into gel purified NotI/NdeI digested pPHT-T7 vector to create pI/M250-PH (Sambrook, *supra* (1989). *E.coli* ER2169 *pLysS*) was transformed with pI/M250-PH to create strain NEB942. The protein produced by NEB942 was called I'P for CIVPS3 C-terminal domain (IVPS) *-D. immitis* Paramyosin ΔSaI-HisTag fusion. The C-terminal domain has no additional amino acids since it begins with a methionine present in CIVPS.

### MI' EXPRESSION AND PURIFICATION

NEB941 was grown at 30°C in LB medium plus 100 µg/ml of ampicillin to an OD₆₀₀ of =0.5. The culture was induced at 30°C with 0.4 mM isopropyl ß-D-thiogalactoside (IPTG) and immediately transferred to a 22°C air shaker in a cold room overnight. The cells were harvested at 4°C and stored at -20°C. Frozen cells from a 1 liter culture were resuspended in 50 ml of amylose column buffer (0.01 M Tris-HCl pH 8.5, 0.2 M NaCl, 1.0 mM Na₂-EDTA) and broken by sonication. After centrifugation at 9,000 g for 30 min, the crude supernatant was passed through an amylose column (New England Biolabs, Inc.; Beverly, Massachusetts. 5 ml of resin), and the column was washed with 50 ml of the above buffer. Maltose, at a final concentration of 10 mM, was added to the column buffer and the elution continued until the MBP fusion was eluted.

### I'P EXPRESSION AND PURIFICATION

A HisTag was included in the construction of I'P to facilitate purification. When I'P protein was expressed in *E.coli,* approximately 90% was Insoluble, which is common with many HisTag (6-10 histidines) fusion proteins. Therefore, I'P samples were solubilized in 6M urea for purification and chromatographed over a Ni²⁺ affinity resin.

NEB942 was grown at 30°C in LB medium plus 100 µg/ml ampicillin to an OD₆₀₀ of =0.5. The culture was induced at 30°C with 0.4 mM IPTG overnight. The cells were harvested at 4°C and stored at -20°C. Frozen cells from a 1 liter culture were thawed in 130 ml of amylose column buffer (0.2 M NaCl, 0.01 M Tris-HCl pH 8.5, 1.0 mM Na₂-EDTA) and broken by sonication. After centrifugation at 20,000 g for 30 min, the pellet containing insoluble material, including the I'P protein, was resuspended in 130 ml of column buffer and centrifuged as before. The washed pellet was resuspended a second time in 130 ml of column buffer and spun as before. The twice washed pellet was finally resuspended in 130 ml of Ni²⁺ binding buffer (20 mM Tris-HCl pH 7.9, 500 mM NaCl, 16 mM imidazole) complemented with 6 M urea. The solubilized pellet was stirred overnight at 4°C, then centrifuged a last time at 31,000 g for 1 hour. The supernatant was filtered through a 0.45 mM membrane (Millex, Millipore; Bedford, Massachusetts), passed through a Ni²⁺ charged column (Novagen; Madison, Wisconsin, 2.5 ml of resin), and the column was washed with 10 volumes of binding buffer. Imidazole at a final concentration of 60 mM was added to the binding buffer and elution of contaminant proteins was continued until undetectable by Bradford assay (BioRad; Hercules, California). The I'P fusion protein was eluted with 180 mM of imidazole in the binding buffer and elution continued until the fusion had eluted completely as shown by the above assay.

### TRANS-SPLICING EXPERIMENTS

Two complementary halves of MIP were constructed as described above. The product of the N-terminal half of MIP, containing all of MBP and the N-terminal domain of CIVPS3 (amino acids 1-249) was termed MI' and the product of the C-terminal half of MIP, containing the C-terminal domain of CIVPS3 (amino acids 250-537) and all of Paramyosin ΔSal was termed I'P. Unfortunately, I'P was insoluble, and needed to be solubilized and purified in 6 M urea. The denaturation and renaturation of enzymes with recovery of enzymatic activity has been reported in the literature (Burbaum and Schimmel, *Biochemistry,* 30:319-324 (1991); Hattori, et al, *J. Biol. Chem.,* 268:22414-22419 (1993); Sancho and Fersht, *J. Mol. Biol.,* 224:741-747 (1992), among others, the disclosures of which are hereby incorporated by reference herein). However, each protocol differs. The initial protocol chosen for this study involved mixing both halves of MIP in urea, incubating at 4°C, rapidly diluting the proteins and then allowing the diluted proteins to refold. This was followed by a standard *in vitro* splicing protocol (Xu et al., *EMBO J.,* 13:5517-5522 (1994); Xu et al., *supra* (1993), the disclosures of which are hereby incorporated by reference herein) after concentration of the diluted proteins, although this concentration step is not necessary. Variation of the different parameters including initial concentration, urea concentration (or other denaturants), dilution factor, length of incubation and protein ratio, allows the optimization of refolding and *trans* -splicing efficiencies.

Purified MI' and I'P fusion proteins were exchanged with buffer A (50 mM Tris-HCl pH 7.5, 5% acetic acid, 0.1 mM EDTA, 1 mM DTT and 140 mM beta-mercapto-ethanol) supplemented with 7.2 M urea and equilibrated at pH 7.5 prior to use. Macrosep (15 ml) and Microsep (3.5 ml) concentrator devices (Filtron Technology Corp.; Northborough, Massachusetts) were used in every step that required a buffer exchange or a protein concentration as described by the manufacturer. The two fusions were then mixed together at a final concentration of 2.3 mg/ml and Incubated overnight at 4°C. The mixture was diluted 50-fold in buffer B (Tris-HCl pH 6, 500 mM NaCl) and renaturation was allowed to occur during the 2 hour concentration step to 0.5-2 mg/ml at 4°C. The mixture was heated in a Perkin-Elmer Cetus thermal cycler at 42°C for 1 hour to induce splicing. To follow the splicing reaction, samples were collected at time-points and Western blots (Sambrook, *supra*(1989)) were performed in duplicate with either mouse sera raised against CIVPS3 (anti-PI-PspI) or paramyosin ΔSal (Steel et al., *J. Immunology,* 145:3917-3923 (1990), the disclosure of which is hereby incorporated by reference herein). In later experiments, the concentration step after mixing was found to be unnecessary. Unfortunately, Western blots are necessary to follow splicing because both the substrate, MI', and the product, MP, have similar molecular masses (approximately 72 kDa). The anti-paramyosin antibody is diagnostic, since it shows the decay of the I'P substrate (approximately 60 kDa) and the formation of the MP product (-72 kDa). On the other hand, anti-MBP sera (New England Biolabs, Inc.; Beverly, Massachusetts) which reacts with the similarly sized MI' and MP, is not diagnostic since as MI' decreases, MP increases at the same position in the gel. As a result, the anti-MBP sera detects a relatively constant band at 72 kDa. The Western blot with the mouse anti-CIVPS3 sera demonstrates the decay of the substrates (MI' and I'P) and the formation of the I' products (which are often inseparable during electrophoresis because of their similar molecular masses). Western blots using anti-Paramyosin antibodies show that there is no cross-reactivity, since anti-Paramyosin sera fails to react with MI' (Figure 17B). Anti-CIVPS3 (anti-PI-PspI) antibody was shown to react with both MI' and I'P (Figure 17A).

Protein splicing of MIP, *in cis,* is more efficient at high temperatures (up to 65°C) and low pH (6.0) (Xu et al., *supra*(1994); Xu et al., *supra*(1993) and Example 11. After a few assays, the splicing reaction for *trans* -splicing was set at 42°C, pH 6.0, although other temperatures and pH's also work. A time course of *trans* -splicing is shown in Figure 17A and 17B. The *trans* -splicing reaction is best monitored by the accumulation of the 72 kDa MP as shown on Western blots using anti-Paramyosin sera and the decrease in MI' and I'P and the formation of I' using anti-CIVPS3 sera (anti-PI-PspI, Figure 17A). In this experiment, both MI' and I'P were exchanged into 7.2 M urea in buffer A using a Microsep concentrator (Filtron Technology Corp.; Northborough, Massachusetts) and mixed at a final concentration of 1 mg/ml each protein. The mixtures were incubated overnight at 4°C and then diluted 50-fold into buffer B. Diluted samples were immediately placed at 42°C or 4°C. Samples were taken after 5, 10, 20, and 30 minutes of incubation and placed on ice. A zero time point was taken prior to placing the tube at 42°C. 5µl of each time point was electrophoresed in duplicate 5-20% SDS-PAGE gels (Daiichi, Tokyo, Japan) and Western blots were performed (Perler et al., *supra*(1992); Sambrook, *surpa*(1989)) with either anti-CIVPS3 (anti-PI-PspI) or anti-Paramyosin sera. No *trans* -splicing was observed in the samples incubated at 4°C. Within 5 minutes at 42°C, the branched intermediate (MI'P*) was observed and by 10 minutes, spliced products (MP and both I') were observed (Figure 17A and 17B).

### RE-ESTABLISHMENT OF I-PSP I ACTIVITY

After *trans* -splicing, the protein mixture was tested for I-Psp I activity (I-PspI or PI-PspI is the same as CIVPS3 or I in this example). The substrate DNA used for I-PspI digestion is pAKR7, which was generated by subcloning a 714 bp EcoRI fragment from pAKK4 (Perler et al., *supra* (1992)) into the EcoRI site of Bluescript SK-. This 714 bp fragment contains the coding region surrounding the sites where IVPS1 and IVPS2 were found in the wild type Vent DNA polymerase clone. Cleavage with Xmni and I-PspI should give fragments of about 2327 and 1351 bp. Test substrate DNA, pAKR7, was reacted with either MI', I'P, the *trans* -splicing reaction products or cis-spliced MIP52 (Figure 18). pAKR7 was cut with Xmnl to linearize the plasmid at a point near the I-PspI restriction site. 5µg of pAKR7 DNA was digested with 6 Units Xmnl (New England Biolabs, Inc.; Beverly, Massachusetts) in NEB buffer 2 for 100 min at 37°C. One microgram of linearized pAKR7 DNA was mixed with 0.01, 0.1 or 1 µg of either MI', I'P or the *trans* -splicing reaction products in a final volume of 55µl I-Pspl buffer (New England Biolabs, Inc.; Beverly, Massachusetts) and incubated at 50°C for 1 hour. In an identical reaction, MIP52 protein was used as a control. MIP52 is a mutant form of MIP containing an insert of MILVA prior to Ser1 of CIVPS3 and this insertion has no effect on splicing or endonuclease activity. MIP52 was used rather than I-Psp I because the *cis*-spliced mixture more closely mimicked the *trans* -spliced mixture. The MIP52 control sample contained precursor MIP and cis-spliced MP and I products. Endonuclease activity was only present in the MIP52 enzyme and the *trans* -spliced mixture, indicating that the above *trans* -splicing protocol not only re-establishes the ability to splice, but also re-establishes endonuclease activity in CIVPS3. As another control, MI' and I'P were added separately to a digestion mixture as above; no digestion was observed (Figure 18), indicating that both protein fragments are required to restore endonuclease activity.

### EXAMPLE 13

### TRANS-CLEAVAGE

In this Example, we describe cleavage at the C-terminal of CIVPS3 *in trans* using the MIP fragments described in Example 12 as a starting point.

### CONSTRUCTION OF MI'22 CONTAINING A ILE2LYS MUTATION lN ClVPS3

In Example 12, we described the construction of MI' and I'P, which were used for *trans*-splicing. In this example we replaced the splice junction cassette in pMI/L249 (which encodes MI') with a duplex oligomer which replaces Ile2 of CIVPS3 with Lys. The techniques used are as described in Examples 10 and 12. Briefly, prior to ligation with the vector, pMI/L249, two complementary oligomers, DVMIP525FW (5'-TC GAGGCTAGCAAATTACCGGAAGAATGGGTAC-3' (SEQ ID NO:62)) and DVMIP526RV (5'-CCATTCTTCCGGTA' TTTGCTAGCC-3' (SEQ ID NO:63)) were allowed to anneal to form a double-stranded linker, DVMIP525FW/DVMIP526RV. 100 pmol of each oligomer was incubated in 50 µl of 1 x T4 DNA ligase buffer (New England Biolabs, Inc.; Beverly, Massachusetts) at 68°C for 15 min and slowly cooled to 20-30°C. pMI/L249 DNA was digested with Xhol-Kpnl (New England Biolabs, Inc.; Beverly, Massachusetts) as described by the manufacturer and the linear plasmid was purified after electrophoretic separation using the Genecleanll kit (BIO101; Vista, California). 0.1 µg of Xhol-Kpnl-digested pMI/L249 DNA was ligated overnight at 16°C in 10 µl 1x T4 ligase buffer (New England Biolabs, Inc.; Beverly, Massachusetts) with 80 units of T4 DNA ligase (New England Biolabs, Inc.; Beverly, Massachusetts) and 15.5 pmol of the linker DVMIP525FW/DVMIP526RV. The resulting construct was termed pMI'22 and the protein produced by this clone was called MI'22.

### PURIFICATION OF MI'22 AND I'P

I'P was purified as described in Example 12. MI'22 was purified as described for MI' in Example 12. *E.coli* strain ER2497 (NEB975) was transformed with pMI'22 and grown at 30°C in LB medium plus 100 µg/ml of ampicillin to an OD₆₀₀ of =0.5. The culture was induced overnight at 30°C with 0.4 mM isopropyl ß-D-thiogalactoside (IPTG). The cells were harvested at 4°C and stored at -20°C. Frozen cells from a 1 liter culture were resuspended in 60 ml of amylose column buffer (20 mM Sodium phosphate, pH 8.5, 0.5 M NaCl, 1.0 mM Na₂-EDTA) and broken by sonication. After centrifugation at 9,000 g for 20 min, the crude supernatant was diluted two-fold in column buffer and passed through an amylose column (New England Biolabs, Inc.; Beverly, Massachusetts, 12.5 ml of resin), and the column was washed with 60 ml of the above buffer followed by 60 ml of amylose column buffer adjusted to pH 6. Maltose, at a final concentration of 10 mM, was added to the pH 6 column buffer and the elution continued until the MBP fusion was eluted.

### TRANS-CLEAVAGE

Two complementary halves of MIP were constructed as described above. The product of the N-terminal half of MIP, containing all of MBP and the N-terminal domain of CIVPS3 including the Ile2Lys substitution (amino acids 1-249) was termed MI'22 and the product of the C-terminal half of MIP, containing the C-terminal domain of CIVPS3 (amino acids 250-537) and all of Paramyosin ΔSal was termed I'P. The products of the *trans*-splicing reaction are the unchanged MI'22 and the cleaved I'P which forms the I' fragment and the P fragment, both of which are approximately 30 kDa. Unfortunately, I'P was insoluble, and needed to be solubilized, and purified in 6 M urea. The initial protocol chosen for this study was as described in Example 12, involved mixing both halves of MIP in urea, incubating at 4°C, rapidly diluting the proteins and then allowing the diluted proteins to refold. This was followed by a standard *in vitro* splicing protocol (Xu, M., et al., *supra* (1994); Xu, M., et al., *supra* (1993)). Variation of the different parameters including initial concentration, urea concentration (or other denaturants), dilution factor, length of incubation and protein ratio, allows the optimization of refolding and *trans*-cleaving efficiencies.

Approximately 10 µg each of purified MI'22 and I'P fusion protein were mixed in 24 µl total volume of Novagen His Tag column binding buffer (20 mM Tris, HCl, pH 7.9, 0.5 M NaCl, 5 mM imidazole) adjusted to 6M urea and incubated on ice for 90 minutes. The sample was diluted 25-fold in 20 mM sodium phosphate buffer, pH6, 0.5 M NaCl and 1 mM EDTA and incubated at 42°C. Samples were taken and placed on ice at 0, 5, 10, 20,40 and 90 minutes. Samples were boiled in SDS-PAGE sample buffer (Sambrook et al., *supra* (1989)), electrophoresed on 4-20% gradient SDS-PAGE (Daiichi, Tokyo, Japan) and stained with Coomassie blue. As seen in Figure 19, I'P (~60 kDa) disappears with time and I' and P appear at approximately the same position in the gel (~30kDa). Control samples which are not shown include incubating the mixture of MI'22 plus I'P at 4°C or incubating each protein fragment separately at 42°C; none of these control experiments showed any cleavage activity.

### EXAMPLE 14

### CHEMICAL CONTROL OF IVPS ACTIVITY

In previous Examples, we have demonstrated that splicing and cleavage activities of IVPSs can be controlled by amino acid substitution, temperature and pH. In this Example, we demonstrate that chemical treatment may also be used to activate or inhibit IVPS activity. Thus, an IVPS can become a CIVPS when its activity can be controlled by chemical treatment. In Example 10, we described modification of CIVPS3 in the MIP fusion by cassette replacement which resulted in cleavage at one of the splice junctions instead of splicing. pMIP21 contains two unique restriction sites at each splice junction: an Xhol site and a KpnI site flanking the N-terminal splice junction and a BamHI site and a Stul site flanking the C-terminal splice junction (Figure 14, Example 10). The N-terminal splice junction residue(s) can be changed by replacing the XhoI-KpnI cassette, while the C-terminal splice junction residue(s) can be altered by substituting the BamHI-StuI cassette. In the case of the N-terminal cassette replacement, pMIP21 is first digested with Xhol and KpnI. A cassette carrying desired mutations, formed by annealing two complementary primers, is substituted for the original XhoI-KpnI cassette. Some modifications in the CIVPS may allow activation of cleavage or splicing activity by chemical treatment. In this specific example, we show that substitution of Ser1 by Cys in CIVPS3 results in a chemical-inducible CIVPS in the MI (a truncated form of MIP) context, which, upon chemical activation with hydroxylamine, results in cleavage of the bond between MBP and cysteine in the modified CIVPS3.

### MODIFICATION OF CIVPS3 BY REPLACING SER1 WITH CYS

In this Example, we first modified pMIP21 (Example 10) by substituting a serine with a cysteine at the N-terminal splice junction of CIVPS3 (Ser1Cys) by cassette replacement to yield pMIP47. 2 µg of pMIP21 was digested at 37°C for 4 hours in 100 µl of 1x Buffer 1 (New England Biolabs, Inc.; Beverly, Massachusetts), 100 µg/ml BSA and 20 units of Xhol and 20 units of Kpnl (New England Biolabs, Inc.; Beverly, Massachusetts). Following electrophoretic separation on an 1% agarose gel, pMIP21 DNA was purified by using the Geneclean II kit (BIO101; Vista, California). Two complementary oligomers, MIP535FW (5'-TCGAGGCTTGCATTTTACCGGAAGAATGGGTAC-3' (SEQ ID NO:64)) and MIP536RV (5'-CCATTCTTCCGGTAAAATGCAAGCC-3' (SEQ ID NO:65)) were allowed to anneal to form a double-stranded linker, MIP535FW/MIP536RV. 100 pmol of each of oligomers MIP535FW and MIP536RV were incubated in 50 µl of 1X T4 DNA ligase buffer (New England Biolabs, Inc.; Beverly, Massachusetts) at 65°C for 15 min and slowly cooled to 20-30°C. Approximately 0.1 µg of the XhoI-KpnI digested pMIP21 DNA was ligated at 16°C overnight in 10 µl of 1x T4 DNA ligase buffer (New England Biolabs, Inc.; Beverly, Massachusetts)), 80 units of T4 DNA ligase (New England Biolabs, Inc.; Beverly, Massachusetts) and 15.6 pmol of the linker MIP535FW/MIP536RV, to yield pMIP47. The ligated DNA sample was used to transform *E.coli* strain ER2426 (NEB974).

### CONSTRUCTION OF pMI84 ENCODING MI

pMI84 was constructed in two steps by the following cassette replacement experiments. pMIP21 was first modified by replacing the C-terminal splice junction cassette with linker MIP353FW/MIP354RV to yield pMIP66. The linker MIP353FW/MIP354RV, containing a SphI recognition sequence, was formed by annealing two complementary oligomers, MIP353FW (5'-GATCCCTCTATAAGCATAATATTGGCATG CAGTA-3' (SEQ ID NO:66)) and MIP354RV (5'-TACTGCATGCCAATATT ATGCTTATAGAGG-3' (SEQ ID NO:67)) as described above. pMIP21 DNA was digested with BamHI (New England Biolabs, Inc.; Beverly, Massachusetts) and Stul (New England Biolabs, Inc.; Beverly, Massachusetts) as described in Example 10. 0.1 µg BamHI/Stul digested pMIP21 DNA was ligated at 16°C overnight with 16.6 pmol of linker MIP353FW/MIP354RV in 10 µl of 1x T4 DNA ligase buffer (New England Biolabs, Inc.; Beverly, Massachusetts) in the presence of 40 units of T4 DNA ligase (New England Biolabs, Inc.; Beverly, Massachusetts). After addition of 1 µl of 10x buffer 2 (New England Biolabs, Inc.; Beverly, Massachusetts) and 0.5 µl (10 units) of Stul (New England Biolabs, Inc.; Beverly, Massachusetts), the ligated DNA sample was incubated at 37°C for 3 hours before *E.coli* ER2426 (NEB974) was transformed.

pMIP66 contains unique BamHI and SphI sites flanking the C-terminal splice junction, allowing linker replacement following BamHI and Sphl digestion. A stop codon was then inserted after the CIVPS C-terminus to create the MI truncated fusion. Ser538 was mutated to a translational stop codon (TAA) by replacing the BamHI-SphI cassette with the linker MIP385FW/MIP386RV. The linker was formed as described above by annealing two complementary oligomers, MIP385FW (5'-GATCCCTCTATGCACATAATTAAGGCATG-3' (SEQ ID NO:68)) and MIP386RV (5'-CCTTAATTATGTGCATAGAGG-3'(SEQ ID NO:69)). This mutagenesis cassette contains compatible termini to replace the C-terminal splice junction cassette following BamHI-SphI cleavage of pMIP66. Approximately 1 µg of pMIP66 was digested at 37°C for 4 hours in 30 µl of 1x BamHI Buffer (New England Biolabs, Inc.; Beverly, Massachusetts), 20 units of BamHI and 20 units of Sphl (New England Biolabs, Inc.; Beverly, Massachusetts). Following electrophoretic separation on 1 % agarose gel, pMIP66 DNA was purified by the Geneclean II kit (BIO101; Vista, California) in 20 µl of 10 mM Tris-HCl (pH 8.0)/0.1 mM EDTA. Approximately 0.05 µg of the BamHI-SphI digested pMIP66 DNA was ligated at 16°C overnight in 10 ml of 1 x T4 DNA ligase buffer (New England Biolabs, Inc.; Beverly, Massachusetts), 80 units of T4 DNA ligase (New England Biolabs, Inc.; Beverly, Massachusetts) and 16.6 pmol of the linker MIP535FW/MIP536RV, to yield pM184. The ligated DNA samples were used to transform *E.coli* strain ER2426 (NEB974).

### CONSTRUCTION OF pMI94 (MI WITH THE SERICYS MUTATION)

The translational stop codon (TAA) introduced at the C-terminal splice junction in pMI84 was transferred into pMIP47 to yield pMI94 by ligation of a 6.6 Kb KpnI-PstI fragment of pMIP47 and a 2.3 Kb KpnI-PstI fragment of pMI84. 1 µg of each pMIP47 and pMI84 DNA was incubated at 37°C for 4 hours in 30 µl of 1 x Buffer 1 (New England Biolabs, Inc.; Beverly, Massachusetts), 10 units of KpnI (New England Biolabs, Inc.; Beverly, Massachusetts) and 10 units of PstI (New England Biolabs, Inc.; Beverly, Massachusetts). Following electrophoretic separation on 1% agarose gel, the 6.6 Kb KpnI-PstI fragment from the pMIP47 sample and the 2.3 Kb KpnI-PstI fragment from the pMI84 sample were purified by the Geneclean II kit (BIO101), each in 20 µl of 10 mM Tris-HCl (pH 8.0)/0.1 mM EDTA. pMI94 was formed by incubation at 16°C overnight of 1 µl of the purified 6.6 Kb pMIP47 DNA and 7.8 µl of the purified 2.3 Kb pMI84 DNA, 1 µl of 10x T4 DNA ligase buffer (New England Biolabs, Inc.; Beverly, Massachusetts), 0.2 µl of 400,000 units/ml of T4 DNA ligase (New England Biolabs, Inc.; Beverly, Massachusetts). The ligated DNA sample were used to transform *E.coli* strain ER2426 (NEB974). pMI94 encodes the MI fusion protein with the Ser1Cys substitution which is present in pMI47 in the full MIP fusion context.

### PURIFICATION OF MI94 FOLLOWED BY CHEMICAL INDUCIBLE CLEAVAGE ACTIVITY

The pMI94 construct expresses the MBP-CIVPS3 fusion protein, termed MI94, containing a cysteine residue instead of the native serine residue at the N-terminal of CIVPS3. In order to conduct *in vitro* study of cleavage activity, expression of the MI94 fusion protein was induced at low temperature (12°C) and purified by amylose resin columns. ER2426 (NEB974) harboring pMI94 was cultured in 2 liters of LB medium supplemented with 100 µg/ml ampicillin and induced as described in Example 10. Cells were pelleted, sonicated in 100 ml of pH 8.5 column buffer (20 mM NaPO₄, pH 8.5, 500 mM NaCl) and spun down. The cleared lysate was loaded over a 15 ml amylose resin column (New England Biolabs, Inc.; Beverly, Massachusetts). The column was washed with 100 ml of pH 8.5 column buffer and subsequently with 100 ml pH 6 column buffer (20 mM NaPO₄, pH 6.0, 500 mM NaCl). MI94 was eluted with 10 mM maltose in pH 6 column buffer (as the procedure described in Example 9).

Hydroxylamine (NH₂OH) was used to activate cleavage activity at the N-terminal splice junction. The MI94 protein sample (0.6 mg/ml) was treated with 0.25 M NH₂OH at pH 6 and pH 7. 75 µl of the purified MI94 sample was mixed with 25 µl of 0.4 M Bis-Tris-Propane, 0.5 M NaCl and 1 M NH₂OH-HCl (Sigma) adjusted to pH 6 with 6 N HCl or with 25 µl of 0.4 M Bis-Tris-Propane, 0.5 M NaCl and 1 M NH₂OH-HCl (Sigma) adjusted to pH 7 with 6 N NaOH. In a control experiment, 100 µl of the MI94 sample was mixed with 33 µl of 0.4 M Bis-Tris-Propane, 0.5 M NaCl adjusted to pH 6 with 6 N HCl. 40 µl of the control sample was mixed with 20 µl of 3X Protein Sample Buffer (New England Biolabs, Inc.; Beverly, Massachusetts) and stored on ice. Two 40 µl aliquots of each mixture were incubated at 37°C for 0.5 and 2 hours, respectively. Each sample was mixed with 20 µl of 3X Protein Sample Buffer (New England Biolabs, Inc.; Beverly, Massachusetts) and boiled for 5 min. 5 µl of each sample was electrophoresed on a 4-12% SDS-Polyacrylamide gel (Novex) followed by Coomassie Blue staining (Figure 20). The data indicate that in comparison with the control experiment (minus NH₂OH), hydroxylamine treatment drastically increased cleavage activity at the N-terminal splice junction. At both pH 6 and pH 7, MI fusion protein was activated by hydroxylamine and efficiently cleaved, yielding more MBP (M, 43 kDa) and CIVPS3 (1, 60 kDa).

In this Example we demonstrate that modifications of an IVPS may result in drastic effects on splicing and cleavage activity after chemical treatment. Furthermore, this data gives another example of constructs where cleavage at N-terminal splice junction is observed in the absence of ligation and carboxyl junction cleavage activities of the CIVPS.

### EXAMPLE 15

### CHEMICAL CONTROL OF CLEAVAGE ACTIVITY OF IVPS FROM SACCHAROMYCES CEREVISIAE

Protein splicing activity of IVPS (yeast intein) from *Saccharomyces cerevisiae* has been described by Hirata *et al, supra* and Kane *et al., supra.* In this Example, we described the construction of a yeast intein fusion system similar to MIP fusion of Example 9. The yeast intein fusion system is a 3-part fusion composed of a maltose binding protein (MBP), a genetically engineered yeast intein (Y), a chitin binding domain (B). This yeast intein fusion system, named MYB fusion, can be induced to cleave at the N-terminal splicing juntion (Cys1) between the maltose binding protein and the yeast intein. MBP can be replaced by the target protein in the MYB protein purification system.

### CONSTRUCTION OF WILD-TYPE MYP

Splice junction amino acid residues of the yeast IVPS are shown in Figure 1. Yeast IVPS (Gimble, et al., *J. Biol. Chem.,* 268(29)21844-21853 (1993), the disclosure of which is hereby incorporated by reference herein) was amplified by PCR from the plasmid of pT7VDE and inserted into MIP21 (described in Example 10 ) between the Xhol site and the Stul site to replace the CIVPS3 (or the Pyroccocus IVPS). Primer pairs 5'-GCGCTCGAGGGGTGCTTTGCCAAGGGTACCAAT-3' (SEQ ID NO:70) and 5'-CCTCCGCAATTATGGACGACAACCTGGT-3' (SEQ ID NO:71) were used to to synthesize the IVPS fragment by PCR. pT7VDE plasmid DNA containing the yeast IVPS gene sequence in the orientation of T7 promoter, was used as template. The PCR mixture contains Vent DNA polymerase buffer (New England Biolabs, Inc; Beverly, Massachusetts), supplemented with 4 mM Magnesium sulfate, 400 uM of each dNTP, 1 uM of each primer, 50 ng pT7VDE DNA and 0.5 units of Vent DNA polymerase in 50 ul. Amplification was carried out by using a Perkin-Elmer/Cetus (Emeryville, California) thermal cycler at 94°C for 30 sec, 50°C for 30 sec and 72°C for 5 min for 20 cycles. The samples were electrophoresed on an 1 % agarose gel and approximately 2 ug of PCR-synthesized 1.3 Kb fragment were recovered in 20 ul of distilled water by Geneclean II kit (BIO101; Vista, California). The purified DNA was subjected to digestion in a 100 ul 1 X NEB buffer 2 with 40 units of Xhol (New England Biolabs, Inc.; Beverly, Massachusetts). The digested DNA was extracted with phenol and choroform and precipitated in 0.3 M NaAcetate pH5.2 and 70% ethanol at -20°C overnight. DNA was spun down, dried and resuspended in 40 ul distilled water. 0.5 ug of MIP21 DNA was digested by Xhol and Stul and the 7.2 Kb vector DNA was purified from 1 % agarose gel by Geneclean II (BIO101; Vista, California) at 0.5 ug/20ul.

MYP1 was created by ligation, of Xhol-digested IVPS fragment to the 7.2 Kb Xhol-Stul MIP21 fragment. The reaction was carried out at 22°C for 5 hours in 10 ul volume with addition of 2 ul of 10X T4 DNA ligase buffer (New England Biolabs, Inc.; Beverly, Massachusetts), 0.4 ug IVPS DNA, 0.025 ug MIP21 fragment. and 200 units of T4 DNA ligase (New England Biolabs, Inc.; Beverly, Massachusetts). Transformation of *E. coli* strain RR1 with the ligation samples was performed as described in Example 2. Transfomants were cultured in LB medium, supplemented with 100 ug/ml ampicillin, for extraction of plasmid DNA using Qiagen spin column (Qiagen, Inc.; Study City, California). The clones were further examined by their ability to splice to form MP species (71 KDa). Nine clones carrying MYP1-9 were cultured in LB medium supplemented with 100 ug/ml ampicillin, at 30°C until OD₆₀₀ₙₘ reached about 0.5. Expression of the MYP fusion gene was induced by addition of IPTG to a final concentration of 1 mM at 30°C for 3 additional hours. Cells were spun down and resuspended in 0.5 ml LB medium. Crude extracts were prepared as described in Example 3. Western blots using antibodies raised against MBP (New England Biolabs, Inc.; Beverly, Massachusetts) were performed to detect fusion protein and splicing products expressed from these clones. Samples were electophoresed on 4-12% Tris-Glycine gels (Novex; Encinitas, California) with prestained markers (Gibco BRL; Gaithersburg, Maryland), transferred to nitrocellulose, probed with anti-MBP antibody (New England Biolabs, Inc.; Beverly, Massachusetts, prepared from rabbit), and detected using alkaline phosphate-linked anti-rabbit secondary antibody as described by the manufacturer (Promega Corp.; Madison, Wisconsin). Western blot analysis showed that except in MYP2 clone, all the other 8 isolates yielded a major product of 71 kDa, indicating that wild-type MYP fusion protein are capable of efficient splicing *in vivo.*

### MODIFICATION OF WILD-TYPE YEAST INTEIN

The first modificaiton of yeast intein was to create two unique restriction sites (BamHI and EcoRI) on the either side of the C-terminal splicing junction. This would facilitate further cassette mutagenesis.

1 µg of pMYP1 and 1 µg LITMUS 29 (New England Biolabs, Inc.; Beverly, Massachusetts) were digested separately in a 15 µl reaction mixture containing 1 x buffer 2 (New England Biolabs, Inc; Beverly, Massachusetts), 0.5 unit Xhol (New England Biolabs, Inc.; Beverly, Massachusetts), and 0.5 unit PstI (New England Biolabs, Inc.; Beverly, Massachusetts) at 37°C for 2hr. After electrophoretic separation on a 1% low melting agarose gel (FMC Corp.; Rockland, Maine), the Xho-Pst fragment containing the yeast intein and the digested LITMUS 29 were excised from the gel. The gel slices were mixed and melt at 65°C for 10 min. The mixture was then incubated at 42°C for 10 min before 1 unit of ß-agarase (New England Biolabs, Inc.; Beverly, Massachusetts) was added. After further 1 hr incubation, the mixture was ready for DNA ligation reaction. The ligation was conducted in 1 x T4 DNA ligase buffer (New England Biolabs, Inc.; Beverly, Massachusetts) containing 0.5 unit of T4 DNA ligase (New England Biolabs, Inc.; Beverly, Massachusetts) at 15°C overnight. 15 µL of the ligation mixture was used to transform *E. coli* strain ER2267 (New England Biolabs, Inc.; Beverly, Massachusetts). The resulting construct was named pLit-YP, a LITMUS vector containing the yeast intein.

pLit-YP was used for the synthesis of the single-stranded DNA and the subsequent Kunkel mutagenesis (Kunkel, T.A., PNAS (1985), 82:488, the disclosure of which is hereby incorporated by reference herein). pLit-YP was first transformed into the competent *E. coli* strain CJ236 (New England Biolabs, Inc.; Beverly, Massachusetts). A single colony was picked to innoculate 50 ml rich LB medium. The cells were allowed to grow at 37°C for 2-3 hr under vigorous aeration. 50 µL of M13K07 helper phage (New England Biolabs, Inc.; Beverly, Massachusetts) was then added to the culture. After another one hour culture, kanamycin was added to the final concentration of 70 µg per mL culture. After overnight culture, the cells were spun down. 10 mL of 20% PEG containing 2.5 M NaCl was added into the supernatant. The phage which contained the single-stranded Lit-YP DNA (ss pLit-YP) was allowed to precipitated on ice for 1 hr. The supernatant was then centrifuged at 8000 rpm for 10 min. The phage pellet was resuspended in 1.6 mL TE buffer (20 mM Tris, pH 8.0, 1 mM EDTA). 400 µl of of 20% PEG containing 2.5 M NaCl was then added to re-precipitate the phage for 5 min at 25°C. The phage pellet was spun down again and resuspended in 600 µl TE buffer. After three times phenol extraction and one time chloroform extraction, the single-stranded DNA was precipitated in 60% ethanol containing 0.2M NaOAc. The DNA pellet was then dried and resuspended in 30 µl TE buffer.

Two mutagenic primers, MYP(EcoR) (5'- GAATGCGGAATTCAGG CCTCCGCA-3' (SEQ ID NO:72)), and MYP (Bam) (5'-ATGGACGACAAC CTGGGATCCAAGCAAAAACTGATGATC-3' (SEQ ID NO:73)) were first 5' phosphorylated. The mutagenic primers (20 pmol each) were added to a 20 µL reaction mixture containing 1 x T4 polynucleotide kinase buffer (New England Biolabs, Inc.; Beverly, Massachusetts), 1 mM ATP, and 1 unit of T4 polynucleotide kinase (New England Biolabs, Inc.; Beverly, Massachusetts). The reaction was conducted at 37°C for 30 min followed by a 10-min heat inactivation of the T4 polynucleotide kinase at 65°C. 10 pmol of the phosphorylated mutagenic primers were added to a 10 µL reaction mixture containing 0.1 µg of the single-stranded pLit-YP template, 1 x annealing buffer. The reaction mixture was heated to 94°C for 4 min and slowly cooled to 25°C to allow the primers to anneal to the template. The next elongation reaction was conducted at 37°C for 2 hrs in a 50 µL mixture containing 1 x T7 polymerase buffer (New England Biolabs, Inc.; Beverly, Massachusetts), 0.5 µg BSA, 300 mM dNTPs, 1 mM ATP, the annealed template, 1 unit of T7 DNA polymerase (New England Biolabs, Inc.; Beverly, Massachusetts) and 1 unit of T4 DNA ligase (New England Biolabs, Inc.; Beverly, Massachusetts). 15 µl of the elongation mixture was used to transform the *E. coli* strain ER 2267. The resulting plasmid, pLit-YP', contained two unique restriction sites, BamH1 and EcoR1, on the either side of the yeast intein C-terminal splicing junction. The Gly447 and S448 of the intein were mutated into Ala and Asn, respectively.

1 µg of pMYP and 1 µg pLit-YP' were digested separately in a 15 µl reaction mixture containing 1 x buffer 2 (New England Biolabs, Inc.; Beverly, Massachusetts), 0.5 unit Xhol (New England Biolabs, Inc.; Beverly, Massachusetts), and 0.5 unit Pstl (New England Biolabs, Inc.; Beverly, Massachusetts) at 37°C for 2hr. After electrophoretic separation on a 1% low melting agarose gel (FMC Corp.; Rockland, Maine), the Xho-Pst fragment from pLit-YP' and the digested pMYP were excised from the gel. The gel slices were mixed and melt at 65°C for 10 min. The mixture was then incubated at 42°C for 10 min before 1 unit of ß-agarase (New England Biolabs, Inc.; Beverly, Massachusetts) was added. After further 1 hr incubation, the mixture was ready for DNA ligation reaction. The ligation was conducted in 1 x T4 DNA ligase buffer (New England Biolabs, Inc.; Beverly, Massachusetts) containing 0.5 unit of T4 DNA ligase (New England Biolabs, Inc.; Beverly, Massachusetts) at 15°C overnight. 15 µL of the ligation mixture was used to transform *E.* *coli* strain ER2267 (NEB#746; New England Biolabs, Inc.; Beverly, Massachusetts). The resulting construct was named pMYP'.

The second modification was to replace Asn454 with Ala. This was achieved by cassette mutagenesis.

1 µg of pMYP' was digested at 37°C for 2 hours in 15 µL of 1 x Buffer 1 (New England Biolabs, Inc.; Beverly, Massachusetts), 100 µg/ml BSA and 1 unit of Xhol and 1 unit of Kpnl (New England Biolabs, Inc.; Beverly, Massachusetts). After electrophoretic separation on a 1% low melting agarose gel (FMC Corp.; Rockland, Maine), the digested pMYP' plasmid DNA was excised from the gel. The gel slices were melt at 65°C for 10 min and then incubated at 42°C for 10 min before 1 unit of ß-agarase (New England Biolabs, Inc.; Beverly, Massachusetts) was added. After further 1 hr incubation, the purified pMYP' digest was ready for DNA ligation reaction. Two complementary oligomers, MYP' (N454A)FW (5'GATCCCAG GTTGTCGTCCATGCATGCGGAGGCCTG-3' (SEQ ID NO:74)) and MYP'(N454A)RV (5'AATTCAGGCCTCCGCATGCA TGGACGACAACCTGG-3' (SEQ ID NO:75)) were allowed to anneal to form a double-stranded linker, MYP'(N454A)FW/RV. 100 pmol of each of the oligomers MYP' (N454A)FW and MYP' (N454A)RV were incubated in 20 µL of 1X annealing buffer at 90°C for 4 min and slowly cooled to 37°C. Approximately 0.1 µg of the Xhol-Kpnl digested pMYP' DNA was ligated with 20 pmol of the annealed linker MYP'(N454A)FW/RV at 16°C overnight In a 20 µl reaction mixture containing 1 x T4 DNA ligase buffer (New England Biolabs, Inc.; Beverly, Massachusetts), 80 units of T4 DNA ligase (New England Biolabs, Inc.; Beverly, Massachusetts). The ligated DNA sample was used to transform *E.coli* strain ER2267. The resulting plasmid was named pMYP'(N454A).

### CONSTRUCTION OF THE YEAST INTEIN PURIFICATION VECTOR pMYB 129

The yeast intein purification vector employed the chitin-binding domain as the affinity tag for affinity purification. Since pMIC (Example 11) contains the chitin-binding domain and compatible restriction sites for direct cloning, the XhoI-BamHI fragment from pMYP'(N454A) was first transfered into pMIC, replacing the original XhoI-BamHI sequence. On the next step, a BamHI-Agel linker insertion was conducted to restore the yeast intein C-terminal splicing junction sequence.

1 µg of pMYP' (N454A) and 1 µg pMIC were digested separately in a 15 µl reaction mixture containing 1 x BamHI buffer (New England Biolabs, Inc.; Beverly, Massachusetts), 0.5 unit Xhol (New England Biolabs, Inc.; Beverly, Massachusetts), and 0.5 unit BamHI (New England Biolabs, Inc.; Beverly, Massachusetts) at 37°C for 2hr. After electrophoretic separation on a 1% low melting agarose gel (FMC Corp.; Rockland, Maine), the XhoI-BamHI fragment from pMYP(N454A) and the digested pMIC were excised from the gel. The gel slices were mixed and melt at 65°C for 10 min. The mixture was then incubated at 42°C for 10 min before 1 unit of ß-agarase (New England Biolabs, Inc.; Beverly, Massachusetts) was added. After further 1 hr incubation, the mixture was ready for DNA ligation reaction. The ligation was conducted in 1 x ligase buffer (New England Biolabs, Inc.; Beverly, Massachusetts) containing 0.5 unit of T4 DNA ligase (New England Biolabs, Inc.; Beverly, Massachusetts) at 15°C overnight. 15 µl of the ligation mixture was used to transform *E. coli* strain ER2267 (New England Biolabs, Inc.; Beverly, Massachusetts). The resulting construct was pMY-IC.

1 µg of pMY-IC was digested at 37°C for 2 hours in 15 µL of 1x BamHI buffer (New England Biolabs, Inc.; Beverly, Massachusetts), 1 unit of BamHI (New England Biolabs, Inc.; Beverly, Massachusetts) and 1 unit of Agel (New England Biolabs, Inc.; Beverly, Massachusetts). After electrophoretic separation on a 1% low melting agarose gel (FMC Corp.; Rockland, Maine), the digested pMY-IC plasmid DNA was excised from the gel. The gel slices were melt at 65°C for 10 min and then incubated at 42°C for 10 min before 1 unit of ß-agarase (New England Biolabs, Inc.; Beverly, Massachusetts) was added. After further 1 hr incubation, the purified pMY-IC digest was ready for DNA ligation reaction. Two complementary oligomers, MYB(Bam-Age)FW (5' GATCCCAGGTTGT CGTCCATGCATGCGGTGGCCTGA-3' (SEQ ID NO:76)) and MYB(Bam-Age)RV (5'-CCGGTCAGGCCTCCGCATGCATGGACGACAACCTGG-3' (SEQ ID NO:77)) were allowed to anneal to form a double-stranded linker, MYB(Bam-Age)FW/RV. 100 pmol of each of the oligomers MYB(Bam-Age)FW and MYB(Bam-Age)RV were incubated in 20 µL of 1X annealing buffer at 90°C for 4 min and slowly cooled to 37°C. Approximately 0.1 µg of the BamHI-Agel digested pMY-IC DNA was ligated with 20 pmol annealed linkers at 16°C overnight in a 20 µl reaction mixture containing 1 x T4 DNA ligase buffer (New England Biolabs, Inc.; Beverly, Massachusetts), 80 units of T4 DNA ligase (New England Biolabs, Inc.; Beverly, Massachusetts). The ligated DNA sample was used to transform *E.coli* strain ER2267. The resulting plasmid was named pMYB129 (Figure 21), a sample of which has been deposited under the terms and conditions of the Budapest Treaty with the American Type Culture Collection on December 28, 1995 and received ATCC Accession Number 97398.

### ONE STEP PURIFICATION OF THE TARGET PROTEIN BY THE CHEMICAL INDUCIBLE CLEAVAGE ACTIVITY OF THE MODIFIED IVPS FROM SACCHAROMYCES CEREVISIAE

The pMYB129 construct was used to illustrate the one step purification of a target protein. Here the maltose binding protein is the target protein. The *E. coli* strain ER2267 harboring pMYB129 was cultured at 37°C in 1 liter of LB medium supplemented with 100 µg/mL ampicillin. The culture was allowed to grow until the OD at 600 nm reached 0.7. The induction was conducted by adding IPTG to the final concentration of 0.4 mM. The induced culture was grown at 30°C for 3 hr before the cells was harvested by centrifugation at 4000 rpm for 25 min. The cell pellet was resuspended in 50 mL of the column buffer (20 mM HEPES, pH 7.6, 0.5 M NaCl). The cell suspension was sonicated for 6 min and then centrifuged at 13,000 rpm for 30 min to give the clear lysate (around 50 mL).

The lysate was directly loaded onto a chitin (Sigma; St. Louis, Missouri) and binding was allowed at 4°C for 30 min. (Other preferred chitin resins which can be employed are described hereinbelow.) The chitin was then washed with 10 volumes of column buffer (20 mM HEPES, pH 7.6, 0.5 M NaCl). The column buffer containing 30 mM dithiothreitol (DTT) was used to elute the MBP protein (Figure 22A and 22B). The elution was conducted at 4°C for 16 hr. Only the maltose binding protein was eluted from the chitin under these conditions (Figure 22A and 22B).

MYT fusion protein was purified on a amylose resin (NEB Protein fusion and purification system) as described in Example 9. *In vitro* cleavage experiments have shown that 30 mM ß-mercaptoethanol (ß-ME) and 30 mM DTT result in approximately 70% and 90% cleavage of MYB, respectively (Figure 23A and 23B).

### PREPARATION OF CHITIN BOUND TO SEPHAROSE 4B

One liter settled bed volume Sepharose 4B (Pharmacia; Piscataway, New Jersey) (prewashed with 5 volume of water) is suspended in 1 liter of 0.3 M NaOH, 1 liter of 1,4-Butanediol diglycidoxy ether and 2 grams of sodium borohydride. The suspension is gently rocked in a closed container at room temperature for 4 hours. The epoxy activated Sepharose 4B beads are washed in a buchner funnel (placed on a side arm flask equipped with vacuum or aspirator) with 3 liters of 0.3 M NaOH aqueous solution followed by 6 volumes of deionized water until the effluent pH is neutral. After washing, the epoxy activated Sepharose 4B beads are suspended in 1 liter of aqueous solution containing 40 grams of Sodium meta-periodate. The suspension is shaken in a closed container at room temperature for 90 minutes. The resulting spacer linked aldehyde Sepharose 4B beads are washed with 3 liter of water in a buchner funnel (vacuum assisted or aspirator). The bead paste is added to 1.2 liter of 4% (v/v) aqueous acetic acid solution containing 45 grams of chitosan (Pfanstiehl Laboratories; Wauken, Illinois) and 4 grams of sodium cyanoborohydride. (the chitosan solution is prepared by autoclaving the carbohydrate polymer in the 4% (v/v) aqueous acetic acid in an autoclave for one hour). The suspension of aldehyde sepharose 4B beads in the chitosan solution is gently rocked in a closed container for 18 hours at room temperature. The resulting chitosan coupled sepharose 4B is washed in a buchner funnel (vacuum assisted or aspirator) with 10 liters of water. The beads are then washed with 1 liter of methanol. The methanol bead paste is suspended into 750 ml of acetic anhdride and gently rocked in a sealed polyethylene container for 18 hours a room temperature. The resulting chitin bound bead suspension is transferred to buchner funnel. After removal of acetic anhydride by filtration (vacuum assisted or aspirator). The beads are washed with 3 liters of methanol followed by 6 liters of deionized water. Test for completion of acetylation is accomplished by using a glucosamine standard and the TNBS: perchloric acid assay (Wilkie, S. Landry , D. *BioChromatography*, 3(5):205-214 (1988), the disclosure of which is hereby incorporated by reference herein). If amine is detected the beads are reacetylated as already described. Finally the beads are washed in a buchner funnel with 1 liter of 0.3M NaOH. The chitin beads are suspended in 1 liter of 0.3 M NaOH containing 0.5 grams of sodium borohydride and gently rocked in a sealed container for 18 hours at room temperature. The beads are washed in a buchner funnel with 6 liters of deionized water until the pH of the effluent is neutral. The chitin bound sepharose beads are stored suspended in 30% methanol/H₂O (v/v).

### PREPARATION OF CHITIN BEADS

The beaded form of chitin is prepared by the solidification (precipitation) of chitosan in aqueous solution while the aqueous solution is shaped into beaded droplets. Beaded droplets of the aqueous solution are created by a stirring the aqueous solution with an organic water insoluble layer (pentanol) forming an emulsion which is stabilized by adding a surfactant or stabiliser (Tween 80). The beads of chitosan are formed as the pH is increased and are crosslinked in the reaction with the addition of 1,4 butanediol diglycidyl ether. The bead quality such as size and shape is directly affected by concentration and length of chitosan, volumes and densities of water and oil layer, shapes and relative dimensions of stirrer and reaction vessel, the amount and chemical type of stabiliser and temperature.

The apparatus with dimensions shown in Figure 24 is set up. To the reaction vessel is added 1 liter pentanol, 50 ml polyoxy-ethylenesorbitan monooleate (Tween 80; Sigma Chemical Co., St. Louis, Missouri), and 50 ml 1,4 butanediol diglycidyl ether. The stirring solution is equilibrated to 70°C. The stirring shaft is maintained at 300 rpm. A filtered solution of 7.5 chitosan ( MW = 70,000; Fluka Chemical Co., Ronkonkoma, New York) in 1 liter of 5% acetic acid in water (v:v; preheated to 70°C) is added to the stirring solution of pentanol, detergent and crosslinker. The emulsion is maintained at 70°C and 100 ml of 10 M NaOH is added dropwise over a period of 12 minutes. The emulsion is allowed to stir at 300 rpm at 70°C for one hour. The stirring and heating is stopped after one hour and the pentanol layer (top) is allowed to separate from the aqueous bead suspension. The top alcohol layer is siphoned off from the bottom aqueous layer by an aspirator.

The aqueous chitosan bead suspension is transferred to a buchner funnel equipped with an aspirator pump and washed with 5 liters of water followed by 1.5 liters of methanol. The methanol bead paste is transferred to a polyethylene container and suspended in 200 ml of acetic anhydride. The beads are acetylated in the sealed container at room temperature with gentle rocking for 18 hours. The resulting chitin beads are transferred to a buchner funnel and washed with 2 liters of methanol followed by 4 liters of water. Finally the beads are washed with 1 liter of 0.3 M NaOH.

The alkaline beads paste is transferred to a polyethylene container and suspended in 1 liter of 0.3 M NaOH containing 0.5 grams of sodium borohydride. The chitin bead suspension is gently rocked in the sealed polyethylene container for 18 hours at room temperature. The chitin bead suspension is transferred to a buchner funnel and washed with 4 liters deionized water or until the pH of the effluent as neutral. The beads are stored in 500 ml of 30% methanol water ( v/v).

This invention has been described in detail including the preferred embodiments thereof. However, it will be appreciated that those skilled in the art, upon consideration of this disclosure, may make modifications and improvements thereon without departing from the spirit and scope of the invention as set forth in the claims.

As will be appreciated from the foregoing, certain embodiments of the present invention may be summarized as follows:
1. A modified protein comprising a target protein and a controllable intervening protein sequence, wherein said controllable intervening protein sequence is capable of excision or cleavage under predetermined conditions in *cis* or in *trans.*
2. The modified protein of 1, wherein the controllable intervening protein is inserted into the target protein.
3. The modified protein of 1, wherein the controllable intervening protein sequence is inserted into a region of the target protein such that the target protein is rendered substantially inactive.
4. The modified protein of 3, wherein upon excision of the controllable intervening protein sequence the activity of the target protein is substantially restored.
5. The modified protein of 1, wherein the controllable intervening protein sequence is fused to the target protein.
6. The modified protein of 5, wherein the controllable intervening protein sequence is fused at C-terminal or N-terminai of the target protein.
7. The modified protein of 1, wherein the controllable intervening protein sequence encodes an endonuclease having homology to a homing endonuclease.
8. The modified protein of 7, wherein the endonuclease function of the controllable intervening protein sequence has been substantially inactivated.
9. The modified protein of 1, wherein the controllable intervening protein sequence and the target protein form a splice junction.
10. The modified protein of 9, wherein the splice junction comprises at the C-terminal side amino acid residues having -OH or -SH side chains for splicing.
11. The modified protein of 10, wherein the splice junction comprises at the 3' end of the controllable intervening protein sequence a His-Asn dipeptide.
12. The modified protein of 11, wherein the controllable intervening protein sequence is selected from the group consisting of controllable intervening protein sequence 1, 2, or 3.
13. The modified protein of 12, wherein the controllable intervening protein sequence is inserted immediately before a serine, threonine or cysteine residue of the target protein.
14. The modified protein of 12, wherein the controllable intervening protein sequence contains a serine, threonine or cysteine residue at its 5' end.
15. The modified protein of 10, wherein at least one residue having an -OH or -SH side chain is modified such that cleavage is reduced.
16. The modified protein of 15, wherein the modification is an amino acid substitution.
17. The modified protein of 15, wherein the modification is a post-translational or co-translational chemical derivatization of the side chain.
18. The modified protein of 16, wherein the amino acid substitution, substitutes at least one of the amino acids involved in the splicing reaction with a derivative in which the functionality of the side chain is masked by a removable group.
19. The modified protein of 17, wherein at least one of the amino acids involved in the splicing reaction is chemically derivatized such that the functionality of the side chain is masked by a removable group.
20. The modified protein of 18, wherein the removable group is chemically or photolytically removable.
21. The modified protein of 1, wherein the predetermined condition is selected from the group consisting of increase in temperature, addition of a chemical reagent which facilitates splicing or cleavage, change in pH and exposure to light.
22. The modified protein of 1, wherein the controllable intervening sequence is derived from *Saccharomyces.*
23. A method of producing a modified protein which comprises:
   (a) joining a DNA encoding a controllable intervening protein sequence with a DNA encoding a target protein to form a fusion DNA;
   (b) expressing said fusion DNA to produce the modified target protein.
24. The method of 24, wherein the DNA encoding the controllable intervening protein sequence is joined with the DNA encoding the target protein by inserting it into the DNA encoding the target protein.
25. The method of 24, wherein the controllable intervening protein sequence DNA is inserted at a site appropriate for substantially decreasing the activity of the target protein.
26. The method of 25 wherein upon excision of the controllable intervening protein sequence the activity of the target protein is substantially restored.
27. The method of 23, wherein the DNA encoding the controllable intervening protein sequence is joined with the DNA encoding the target protein by fusing it to the DNA encoding the target protein.
28. The method of 23, wherein the controllable intervening protein sequence encodes for an endonuclease having homology to a homing endonuclease.
29. The method of 28, wherein the endonuclease function of the controllable intervening protein sequence has been substantially inactivated.
30. The method of 28, wherein the controllable intervening protein sequence is selected from the group consisting of controllable intervening protein sequence 1, 2, or 3.
31. The method of 30, wherein the controllable intervening protein sequence is inserted immediately before a serine, threonine or cysteine residue of the target protein.
32. The method 30, wherein the controllable intervening protein sequence contains a serine, threonine or cysteine residue at its 5' end.
33. A method of producing a protein comprising:
   (a) inserting a DNA encoding a controllable intervening protein sequence into a DNA encoding a target protein, wherein said controllable intervening protein sequence is capable of excision under predetermined conditions;
   (b) expressing the DNA of step (a) to produce a modified target protein; and
   (c) subjecting the modified target protein to conditions under which said controllable intervening protein sequence will undergo excision.
34. A method of producing a protein comprising:
   (a) producing a first modified protein comprising an amino portion of a target protein into which is inserted at its carboxy terminus a controllable intervening protein sequence;
   (b) producing a second modified protein comprising the remaining portion of the target protein of step (a) into which is inserted at its amino terminus a controllable Intervening protein sequence; and
   (c) placing the first and second modified proteins under predetermined conditions appropriate for splicing of the controllable intervening protein sequence.
35. The method of 34, wherein the controllable intervening protein sequence inserted at the carboxy terminus of the target protein comprises an amino terminal fragment of the controllable intervening protein sequence and the controllable intervening protein sequence inserted at the amino terminus of the remaining portion of the target protein comprises the remaining fragment of the controllable intervening protein sequence.
36. A method of producing a protein comprising:
   (a) producing a first modified protein comprising a portion of a target protein into which a controllable intervening protein sequence is inserted at a splice junction; and
   (b) placing the first modified protein with the remaining portion of the target protein under predetermined conditions appropriate for splicing of the controllable intervening protein sequence.
37. A method for purification of a target protein comprising:
   (a) forming a fusion protein comprising a controllable intervening protein sequence between a target protein and a binding protein having affinity for a substrate;
   (b) contacting the fusion protein with substrate to which the binding protein binds;
   (c) subjecting the substrate bound fusion protein to conditions under which cleavage of the controllable intervening protein sequence occurs, thus separating the target protein from the binding protein; and
   (d) recovering the target protein.
38. The method of 37, wherein the substrate is contained within an affinity column.
39. The method of 37, wherein the binding protein is selected from the group consisting of sugar binding protein, chitin binding protein, receptor protein, amino acid binding protein, sulfate binding protein, vitamin binding protein, metal binding protein, phosphate binding protein, lectin binding protein or nucleic acid binding protein.
40. A method for purification of a target protein comprising:
   (a) forming a fusion protein comprising a controllable intervening protein sequence and a target protein;
   (b) contacting a fusion protein with a substrate to which the controllable intervening protein sequence binds;
   (c) subjecting the substrate bound fusion protein to conditions under which cleavage of the controllable intervening protein sequence occurs; thus separating the target protein from the controllable intervening sequence; and
   (d) recovering the target protein.
41. The method of 39, wherein the substrate is an antibody against the controllable intervening protein sequence.
42. The modified protein of 9, wherein for cleavage, the splice junction comprises at the C-terminal side of the splice junction amino acid residues not having -OH or -SH side chains for C-terminal cleavage and having amino acid residues having -OH or -SH side chains only at the upstream splice junction for N-terminal cleavage.
43. A method of producing a protein comprising:
   (a) producing a first modified protein comprising a target protein into which is inserted at its carboxy terminus an amino-terminal portion of a controllable intervening protein sequence;
   (b) producing a second modified protein comprising the remaining portion of the controllable intervening protein sequence; and
   (c) placing the first and second modified proteins under predetermined conditions appropriate for cleavage of the controllable intervening protein sequence *in trans.*
44. A method of producing a protein comprising:
   (a) producing a first modified protein comprising a target protein into which is inserted at its amino terminus a carboxy-terminal portion of a controllable intervening protein sequence;
   (b) producing a second modified protein comprising the remaining portion of the controllable intervening protein sequence;and
   (c) placing the first and second modified proteins under predetermined conditions appropriate for cleavage of the controllable intervening protein sequence *in trans.*
45. A method for purification of a target protein comprising:
   (a) forming a fusion protein comprising a portion of a controllable intervening protein sequence between a target protein and a binding protein having affinity for a substrate;
   (b) contacting the fusion protein with substrate to which the binding protein binds;
   (c) combining the remaining portion of the controllable intervening protein sequence with the fusion protein;
   (d) subjecting the combined substrate bound fusion protein of step (c) to conditions under which cleavage of the controllable intervening protein sequence occurs, thus separating the target protein from the binding protein; and
   (e) recovering the target protein.
46. The method of 45, wherein the remaining portion of the controllable intervening protein sequence has an affinity tag.
47 A method for purification of a target protein comprising:
   (a) forming a fusion protein comprising a portion of a controllable intervening protein sequence between a target protein and a binding protein having affinity for a substrate;
   (b) contacting the fusion protein with substrate to which the binding protein binds;
   (c) recovering the fusion protein of step (b);
   (d) combining the remaining portion of the controllable intervening protein sequence with the fusion protein;
   (e) subjecting the substrate bound fusion protein to conditions unde : which cleavage of the controllable intervening protein sequence occurs, thus separating the target protein from the binding protein; and
   (f) recovering the target protein.
48. The method of 47, wherein the remaining portion of the controllable intervening protein sequence has an affinity tag.
49. The method of 23, 33, 34, 36, 37, 40, 43, 44, 45, and 47, wherein the controllable intervening protein sequence is derived from *Saccharomyces.*

## Claims

1. A modified protein comprising a target protein, or, portion thereof, fused, either internally or terminally, to a controllable intervening protein sequence, or to an amino-terminal element or a carboxyl-terminal element of a controllable intervening protein sequence, wherein the controllable intervening protein sequence or element thereof permits a process, conducted *in cis* or *in trans,* selected from the group of processes consisting of excision, cleavage, ligation, combined excision and ligation, combined cleavage and ligation, and cyclization under conditions suitable for said excision, cleavage, ligation, combined excision and ligation, combined cleavage and ligation and cyclization.

2. The modified protein of claim 1, wherein suitable condition of excision, cleavage, ligation, combined excision and ligation, combined cleavage and ligation, and cyclization is selected from the group of conditions consisting of a change in temperature, an addition or a removal of a chemical reagent which facilitates or inhibits splicing or cleavage, a change in pH, an exposure to or an absence of light, dephosphorylation or deglycosylation of an amino acid residue, and contact with or removal of a peptide or a peptidomimetic capable of activating or blocking splicing or cleavage.

3. The modified protein of claim 1, wherein the controllable intervening protein sequence is derived from *Saccharomyces.*

4. A method of producing a protein comprising:
(a) inserting a DNA encoding a controllable intervening protein sequence into a DNA encoding a target protein, wherein said controllable intervening protein sequence is capable of excision under predetermined conditions;
(b) expressing the DNA of step (a) to produce a modified target protein; and
(c) subjecting the modified target protein to conditions under which said controllable intervening protein sequence will undergo excision.

5. The modified protein of claim 1, wherein the controllable intervening protein sequence and target protein forms a splice junction, wherein for cleavage, the splice junction comprises at the C-terminal side of the splice junction amino acid residues not having -OH or -SH side chains for C-terminal cleavage and having amino acid residues having -OH or -SH side chains only at the upstream splice junction for N-terminal cleavage.

6. A method of producing a protein comprising:
(a) producing a first modified protein comprising a target protein into which is inserted at its carboxy terminus an amino-terminal portion of a controllable intervening protein sequence;
(b) producing a second modified protein comprising the remaining portion of the controllable intervening protein sequence; and
(c) placing the first and second modified proteins under predetermined conditions appropriate for cleavage of the controllable intervening protein sequence *in trans.*

7. A method of producing a protein comprising:
(a) producing a first modified protein comprising a target protein into which is inserted at its amino terminus a carboxy-terminal portion of a controllable intervening protein sequence;
(b) producing a second modified protein comprising the remaining portion of the controllable intervening protein sequence; and
(c) placing the first and second modified proteins under predetermined conditions appropriate for cleavage of the controllable intervening protein sequence *in trans.*

8. A method for purification of a target protein comprising:
(a) forming a fusion protein comprising a portion of a controllable intervening protein sequence between a target protein and a binding protein having affinity for a substrate;
(b) contacting the fusion protein with substrate to which the binding protein binds;
(c) combining the remaining portion of the controllable intervening protein sequence with the fusion protein;
(d) subjecting the combined substrate bound fusion protein of step (c) to conditions under which cleavage of the controllable intervening protein sequence occurs, thus separating the target protein from the binding protein; and
(e) recovering the target protein.

9. The method of claim 8, wherein the remaining portion of the controllable intervening protein sequence has an affinity tag.

10. A method for purification of a target protein comprising:
(a) forming a fusion protein comprising a portion of a controllable intervening protein sequence between a target protein and a binding protein having affinity for a substrate;
(b) contacting the fusion protein with substrate to which the binding protein binds;
(c) recovering the fusion protein of step (b);
(d) combining the remaining portion of the controllable intervening protein sequence with the fusion protein;
(e) subjecting the substrate bound fusion protein to conditions under which cleavage of the controllable intervening protein sequence occurs, thus separating the target protein from the binding protein; and
(f) recovering the target protein.

11. The method of claim 10, wherein the remaining portion of the controllable intervening protein sequence has an affinity tag.

12. The method of claim 1, 6, 7, 8, and 10, wherein the controllable intervening protein sequence is derived from *Saccharomyces.*


SEQUENCE LISTING

(1) GENERAL INFORMATION:

(i) APPLICANT: COMB, DONALD G.
PERLER, FRANCINE B.
JACK, WILLIAM E.
XU, MING-QUN
HODGES, ROBERT A.
NOREN, CHRISTOPHER J.

(ii) TITLE OF INVENTION: MODIFIED PROTEINS AND METHODS OF
THEIR PRODUCTION

(iii) NUMBER OF SEQUENCES: 77

(iv) CORRESPONDENCE ADDRESS:

(A) ADDRESSEE: GREGORY D. WILLIAMS; NEW ENGLAND BIOLABS,
INC.
(B) STREET: 32 TOZER RAOD
(C) CITY: BEVERLY
(D) STATE: MASSACHUSETTS
(E) COUNTRY: USA
(F) ZIP: 01915

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:

(A) APPLICATION NUMBER: PCT/US96/
(B) FILING DATE:
(C) CLASSIFICATION:

(vii) PRIOR APPLICATION DATA:

(A) APPLICATION NUMBER: US 08/580,555
(B) FILING DATE: 29-DEC-1995
(C) CLASSIFICATION:

(vii) PRIOR APPLICATION DATA:

(A) APPLICATION NUMBER: US 08/496,247
(B) FILING DATE: 28-JUN-1995
(C) CLASSIFICATION:

(vii) PRIOR APPLICATION DATA:

(A) APPLICATION NUMBER: US 08/146,885
(B) FILING DATE: 03-NOV-1993
(C) CLASSIFICATION:

(vii) PRIOR APPLICATION DATA:

(A) APPLICATION NUMBER: US 08/004,139
(B) FILING DATE: 09-DEC-1992
(C) CLASSIFICATION:

(viii) ATTORNEY/AGENT INFORMATION:

(A) NAME: WILLIAMS, GREGORY D.
(B) REGISTRATION NUMBER: 30901
(C) REFERENCE/DOCKET NUMBER: NEB-036C2

(ix) TELECOMMUNICATION INFORMATION:
(A) TELEPHONE: (508) 927-5054
(B) TELEFAX: (508) 927-1705
(C) TELEX:

(2) INFORMATION FOR SEQ ID NO:1:

(i) SEQUENCE CHARACTERISTICS:
(A) LENGTH: 5837 base pairs
(B) TYPE: nucleic acid
(C) STRANDEDNESS: double
(D) TOPOLOGY: not relevant

(xi) SEQUENCE DESCRIPTION: SEQ ID NO:1:

GAATTCGCGA TAAAATCTAT TTTCTTCCTC CATTTTTCAA TTTCAAAAAC GTAAGCATGA 60

GCCAAACCTC TCGCCCTTTC TCTGTCCTTC CCGCTAACCC TCTTGAAAAC TCTCTCCAAA 120

GCATTTTTTG ATGAAAGCTC ACGCTCCTCT ATGAGGGTCA GTATATCTGC AATGAGTTCG 180

TGAAGGGTTA TTCTGTAGAA CAACTCCATG ATTTTCGATT TGGATGGGGG TTTAAAAATT 240

TGGCGGAACT TTTATTTAAT TTGAACTCCA GTTTATATCT GGTGGTATTT ATGATACTGG 300

ACACTGATTA CATAACAAAA GATGGCAAGC CTATAATCCG AATTTTTAAG AAAGAGAACG 360

GGGAGTTTAA AATAGAACTT GACCCTCATT TTCAGCCCTA TATATATGCT CTTCTCAAAG 420

ATGACTCCGC TATTGAGGAG ATAAAGGCAA TAAAGGGCGA GAGACATGGA AAAACTGTGA 480

GAGTGCTCGA TGCAGTGAAA GTCAGGAAAA AATTTTTGGG AAGGGAAGTT GAAGTCTGGA 540

AGCTCATTTT CGAGCATCCC CAAGACGTTC CAGCTATGCG GGGCAAAATA AGGGAACATC 600

CAGCTGTGGT TGACATTTAC GAATATGACA TACCCTTTGC CAAGCGTTAT CTCATAGACA 660

AGGGCTTGAT TCCCATGGAG GGAGACGAGG AGCTTAAGCT CCTTGCCTTT GATATTGAAA 720

CGTTTTATCA TGAGGGAGAT GAATTTGGAA AGGGCGAGAT AATAATGATT AGTTATGCCG 780

ATGAAGAAGA GGCCAGAGTA ATCACATGGA AAAATATCGA TTTGCCGTAT GTCGATGTTG 840

TGTCCAATGA AAGAGAAATG ATAAAGCGTT TTGTTCAAGT TGTTAAAGAA AAAGACCCCG 900

ATGTGATAAT AACTTACAAT GGGGACAATT TTGATTTGCC GTATCTCATA AAACGGGCAG 960
AAAAGCTGGG AGTTCGGCTT GTCTTAGGAA GGGACAAAGA ACATCCCGAA CCCAAGATTC 1020
AGAGGATGGG TGATAGTTTT GCTGTGGAAA TCAAGGGTAG AATCCACTTT GATCTTTTCC 1080
CAGTTGTGCG AAGGACGATA AACCTCCCAA CGTATACGCT TGAGGCAGTT TATGAAGCAG 1140
TTTTAGGAAA AACCAAAAGC AAATTAGGAG CAGAGGAAAT TGCCGCTATA TGGGAAACAG 1200
AAGAAAGCAT GAAAAAACTA GCCCAGTACT CAATGGAAGA TGCTAGGGCA ACGTATGAGC 1260
TCGGGAAGGA ATTCTTCCCC ATGGAAGCTG AGCTGGCAAA GCTGATAGGT CAAAGTGTAT 1320
GGGACGTCTC GAGATCAAGC ACCGGCAACC TCGTGGAGTG GTATCTTTTA AGGGTGGCAT 1380
ACGCGAGGAA TGAACTTGCA CCGAACAAAC CTGATGAGGA AGAGTATAAA CGGCGCTTAA 1440
GAACAACTTA CCTGGGAGGA TATGTAAAAG AGCCAGAAAA AGGTTTGTGG GAAAATATCA 1500
TTTATTTGGA TTTCCGCAGT CTGTACCCTT CAATAATAGT TACTCACAAC GTATCCCCAG 1560
ATACCCTTGA AAAAGAGGGC TGTAAGAATT ACGATGTTGC TCCGATAGTA GGATATAGGT 1620
TCTGCAAGGA CTTTCCGGGC TTTATTCCCT CCATACTCGG GGACTTAATT GCAATGAGGC 1680
AAGATATAAA GAAGAAAATG AAATCCACAA TTGACCCGAT CGAAAAGAAA ATGCTCGATT 1740
ATAGGCAAAG GGCTATTAAA TTGCTTGCAA ACAGCATCTT ACCCAACGAG TGGTTACCAA 1800
TAATTGAAAA TGGAGAAATA AAATTCGTGA AAATTGGCGA GTTTATAAAC TCTTACATGG 1860
AAAAACAGAA GGAAAACGTT AAAACAGTAG AGAATACTGA AGTTCTCGAA GTAAACAACC 1920
TTTTTGCATT CTCATTCAAC AAAAAAATCA AAGAAAGTGA AGTCAAAAAA GTCAAAGCCC 1980
TCATAAGACA TAAGTATAAA GGGAAAGCTT ATGAGATTCA GCTTAGCTCT GGTAGAAAAA 2040
TTAACATAAC TGCTGGCCAT AGTCTGTTTA CAGTTAGAAA TGGAGAAATA AAGGAAGTTT 2100
CTGGAGATGG GATAAAAGAA GGTGACCTTA TTGTAGCACC AAAGAAAATT AAACTCAATG 2160
AAAAAGGGGT AAGCATAAAC ATTCCCGAGT TAATCTCAGA TCTTTCCGAG GAAGAAACAG 2220
CCGACATTGT GATGACGATT TCAGCCAAGG GCAGAAAGAA CTTCTTTAAA GGAATGCTGA 2280
GAACTTTAAG GTGGATGTTT GGAGAAGAAA ATAGAAGGAT AAGAACATTT AATCGCTATT 2340
TGTTCCATCT CGAAAAACTA GGCCTTATCA AACTACTGCC CCGCGGATAT GAAGTTACTG 2400
ACTGGGAGAG ATTAAAGAAA TATAAACAAC TTTACGAGAA GCTTGCTGGA AGCGTTAAGT 2460
ACAACGGAAA CAAGAGAGAG TATTTAGTAA TGTTCAACGA GATCAAGGAT TTTATATCTT 2520
ACTTCCCACA AAAAGAGCTC GAAGAATGGA AAATTGGAAC TCTCAATGGC TTTAGAACGA 2580
ATTGTATTCT CAAAGTCGAT GAGGATTTTG GGAAGCTCCT AGGTTACTAT GTTAGTGAGG 2640
GCTATGCAGG TGCACAAAAA AATAAAACTG GTGGTATCAG TTATTCGGTG AAGCTTTACA 2700
ATGAGGACCC TAATGTTCTT GAGAGCATGA AAAATGTTGC AGAAAAATTC TTTGGCAAGG 2760
TTAGAGTTGA CAGAAATTGC GTAAGTATAT CAAAGAAGAT GGCATACTTA GTTATGAAAT 2820

GCCTCTGTGG AGCATTAGCC GAAAACAAGA GAATTCCTTC TGTTATACTC ACCTCTCCCG 2880
AACCGGTACG GTGGTCATTT TTAGAGGCGT ATTTTACAGG CGATGGAGAT ATACATCCAT 2940
CAAAAAGGTT TAGGCTCTCA ACAAAAAGCG AGCTCCTTGC AAATCAGCTT GTGTTCTTGC 3000
TGAACTCTTT GGGAATATCC TCTGTAAAGA TAGGCTTTGA CAGTGGGGTC TATAGAGTGT 3060
ATATAAATGA AGACCTGCAA TTTCCACAAA CGTCTAGGGA GAAAAACACA TACTACTCTA 3120
ACTTAATTCC CAAAGAGATC CTTAGGGACG TGTTTGGAAA AGAGTTCCAA AAGAACATGA 3180
CGTTCAAGAA ATTTAAAGAG CTTGTTGACT CTGGAAAACT TAACAGGGAG AAAGCCAAGC 3240
TCTTGGAGTT CTTCATTAAT GGAGATATTG TCCTTGACAG AGTCAAAAGT GTTAAAGAAA 3300
AGGACTATGA AGGGTATGTC TATGACCTAA GCGTTGAGGA TAACGAGAAC TTTCTTGTTG 3360
GTTTTGGTTT GCTCTATGCT CACAACAGCT ATTACGGCTA TATGGGGTAT CCTAAGGCAA 3420
GATGGTACTC GAAGGAATGT GCTGAAAGCG TTACCGCATG GGGGAGACAC TACATAGAGA 3480
TGACGATAAG AGAAATAGAG GAAAAGTTCG GCTTTAAGGT TCTTTATGCG GACAGTGTCT 3540
CAGGAGAAAG TGAGATCATA ATAAGGCAAA ACGGAAAGAT TAGATTTGTG AAAATAAAGG 3600
ATCTTTTCTC TAAGGTGGAC TACAGCATTG GCGAAAAAGA ATACTGCATT CTCGAAGGTG 3660
TTGAAGCACT AACTCTGGAC GATGACGGAA AGCTTGTCTG GAAGCCCGTC CCCTACGTGA 3720
TGAGGCACAG AGCGAATAAA AGAATGTTCC GCATCTGGCT GACCAACAGC TGGTATATAG 3780
ATGTTACTGA GGATCATTCT CTCATAGGCT ATCTAAACAC GTCAAAAACG AAAACTGCCA 3840
AAAAAATCGG GGAAAGACTA AAGGAAGTAA AGCCTTTTGA ATTAGGCAAA GCAGTAAAAT 3900
CGCTCATATG CCCAAATGCA CCGTTAAAGG ATGAGAATAC CAAAACTAGC GAAATAGCAG 3960
TAAAATTCTG GGAGCTCGTA GGATTGATTG TAGGAGATGG AAACTGGGGT GGAGATTCTC 4020
GTTGGGCAGA GTATTATCTT GGACTTTCAA CAGGCAAAGA TGCAGAAGAG ATAAAGCAAA 4080
AACTTCTGGA ACCCCTAAAA ACTTATGGAG TAATCTCAAA CTATTACCCA AAAAACGAGA 4140
AAGGGGACTT CAACATCTTG GCAAAGAGCC TTGTAAAGTT TATGAAAAGG CACTTTAAGG 4200
ACGAAAAAGG AAGACGAAAA ATTCCAGAGT TCATGTATGA GCTTCCGGTT ACTTACATAG 4260
AGGCATTTCT ACGAGGACTG TTTTCAGCTG ATGGTACTGT AACTATCAGG AAGGGAGTTC 4320
CAGAGATCAG GCTAACAAAC ATTGATGCTG ACTTTCTAAG GGAAGTAAGG AAGCTTCTGT 4380
GGATTGTTGG AATTTCAAAT TCAATATTTG CTGAGACTAC TCCAAATCGC TACAATGGTG 4440
TTTCTACTGG AACCTACTCA AAGCATCTAA GGATCAAAAA TAAGTGGCGT TTTGCTGAAA 4500
GGATAGGCTT TTTAATCGAG AGAAAGCAGA AGAGACTTTT AGAACATTTA AAATCAGCGA 4560
GGGTAAAAAG GAATACCATA GATTTTGGCT TTGATCTTGT GCATGTGAAA AAAGTCGAAG 4620
AGATACCATA CGAGGGTTAC GTTTATGACA TTGAAGTCG[?] AGAGACGCAT AGGTTCTTTG 4680
CAAACAACAT CCTGGTACAC AATACTGACG GCTTTTATG[?] CACAATACCC GGGGAAAAGC 4740

CTGAACTCAT TAAAAAGAAA GCCAAGGAAT TCCTAAACTA CATAAACTCC AAACTTCCAG 4800

GTCTGCTTGA GCTTGAGTAT GAGGGCTTTT ACTTGAGAGG ATTCTTTGTT ACAAAAAAGC 4860

GCTATGCAGT CATAGATGAA GAGGGCAGGA TAACAACAAG GGGCTTGGAA GTAGTAAGGA 4920

GAGATTGGAG TGAGATAGCT AAGGAGACTC AGGCAAAGGT TTTAGAGGCT ATACTTAAAG 4980

AGGGAAGTGT TGAAAAAGCT GTAGAAGTTG TTAGAGATGT TGTAGAGAAA ATAGCAAAAT 5040

ACAGGGTTCC ACTTGAAAAG CTTGTTATCC ATGAGCAGAT TACCAGGGAT TTAAAGGACT 5100

ACAAAGCCAT TGGCCCTCAT GTCGCGATAG CAAAAAGACT TGCCGCAAGA GGGATAAAAG 5160

TGAAACCGGG CACAATAATA AGCTATATCG TTCTCAAAGG GAGCGGAAAG ATAAGCGATA 5220

GGGTAATTTT ACTTACAGAA TACGATCCTA GAAAACACAA GTACGATCCG GACTACTACA 5280

TAGAAAACCA AGTTTTGCCG GCAGTACTTA GGATACTCGA AGCGTTTGGA TACAGAAAGG 5340

AGGATTTAAG GTATCAAAGC TCAAAACAAA CCGGCTTAGA TGCATGGCTC AAGAGGTAGC 5400

TCTGTTGCTT TTTAGTCCAA GTTTCTCCGC GAGTCTCTCT ATCTCTCTTT TGTATTCTGC 5460

TATGTGGTTT TCATTCACTA TTAAGTAGTC CGCCAAAGCC ATAACGCTTC CAATTCCAAA 5520

CTTGAGCTCT TTCCAGTCTC TGGCCTCAAA TTCACTCCAT GTTTTTGGAT CGTCGCTTCT 5580

CCCTCTTCTG CTAAGCCTCT CGAATCTTTT TCTTGGCGAA GAGTGTACAG CTATGATGAT 5640

TATCTCTTCC TCTGGAAACG CATCTTTAAA CGTCTGAATT TCATCTAGAG ACCTCACTCC 5700

GTCGATTATA ACTGCCTTGT ACTTCTTTAG TAGTTCTTTT ACCTTTGGGA TCGTTAATTT 5760

TGCCACGGCA TTGTCCCCAA GCTCCTGCCT AAGCTGAATG CTCACACTGT TCATACCTTC 5820

GGGAGTTCTT GGGATCC 5837

(2) INFORMATION FOR SEQ ID NO:2:

(i) SEQUENCE CHARACTERISTICS:
(A) LENGTH: 4707 base pairs
(B) TYPE: nucleic acid
(C) STRANDEDNESS: double
(D) TOPOLOGY: not relevant
(iii) HYPOTHETICAL: NO
(iv) ANTI-SENSE: NO
(v) FRAGMENT TYPE: N-terminal
(ix) FEATURE:
(A) NAME/KEY: CDS
(B) LOCATION: 363..4298

(xi) SEQUENCE DESCRIPTION: SEQ ID NO:2:

GGATCCCTCT CTTTTTGGTA ACCCCATACG TCATTCCCTC AACCAAAACT TCAGCATCGT 60

TGCAGTGGTC AGTGTGTCTG TGGGAGATGA AGAGGACGTC GATTTTTCTG GGGTCTATCT 120

TGTATCTCCA CATTCTAACT AACGCTCCAG GCCCAGGATC AACGTAGATG TTTTTGCTCG 180

CCTTAATGAA GAAGCCACCA GTGGCTCTTG CCTGCGTTAT CGTGACGAAC CTTCCACCAC 240

CGCCACCGAG AAAAGTTATC TCTATCATCT CACACCTCCC CCATAACATC ACCTGCTCAA 300

TTTTTAAGCG TTCTTAAAGG CTTAAATACG TGAATTTAGC GTAAATTATT GAGGGATTAA 360

GT ATG ATA CTT GAC GCT GAC TAC ATC ACC GAG GAT GGG AAG CCG ATT 407
Met Ile Leu Asp Ala Asp Tyr Ile Thr Glu Asp Gly Lys Pro Ile
1               5                   10                  15

ATA AGG ATT TTC AAG AAA GAA AAC GGC GAG TTT AAG GTT GAG TAC GAC 455
Ile Arg Ile Phe Lys Lys Glu Asn Gly Glu Phe Lys Val Glu Tyr Asp
                20                  25                  30

AGA AAC TTT AGA CCT TAC ATT TAC GCT CTC CTC AAA GAT GAC TCG CAG 503
Arg Asn Phe Arg Pro Tyr Ile Tyr Ala Leu Leu Lys Asp Asp Ser Gln
            35                  40                  45

ATT GAT GAG GTT AGG AAG ATA ACC GCC GAG AGG CAT GGG AAG ATA GTG 551
Ile Asp Glu Val Arg Lys Ile Thr Ala Glu Arg His Gly Lys Ile Val
        50                  55                  60

AGA ATT ATA GAT GCC GAA AAG GTA AGG AAG AAG TTC CTG GGG AGG CCG 599
Arg Ile Ile Asp Ala Glu Lys Val Arg Lys Lys Phe Leu Gly Arg Pro
    65                  70                  75

ATT GAG GTA TGG AGG CTG TAC TTT GAA CAC CCT CAG GAC GTT CCC GCA 647
Ile Glu Val Trp Arg Leu Tyr Phe Glu His Pro Gln Asp Val Pro Ala
80                  85                  90                  95

ATA AGG GAT AAG ATA AGA GAG CAT TCC GCA GTT ATT GAC ATC TTT GAG 695
Ile Arg Asp Lys Ile Arg Glu His Ser Ala Val Ile Asp Ile Phe Glu
                100                 105                 110

TAC GAC ATT CCG TTC GCG AAG AGG TAC CTA ATA GAC AAA GGC CTA ATT 743
Tyr Asp Ile Pro Phe Ala Lys Arg Tyr Leu Ile Asp Lys Gly Leu Ile
            115                 120                 125

CCA ATG GAA GGC GAT GAA GAG CTC AAG TTG CTC GCA TTT GAC ATA GAA 791
Pro Met Glu Gly Asp Glu Glu Leu Lys Leu Leu Ala Phe Asp Ile Glu
        130                 135                 140

ACC CTC TAT CAC GAA GGG GAG GAG TTC GCG AAG GGG CCC ATT ATA ATG 839
Thr Leu Tyr His Glu Gly Glu Glu Phe Ala Lys Gly Pro Ile Ile Met
    145                 150                 155

ATA AGC TAT GCT GAT GAG GAA GAA GCC AAA GTC ATA ACG TGG AAA AAG 887
Ile Ser Tyr Ala Asp Glu Glu Glu Ala Lys Val Ile Thr Trp Lys Lys
160                 165                 170                 175

ATC GAT CTC CCG TAC GTC GAG GTA GTT TCC AGC GAG AGG GAG ATG ATA 935
Ile Asp Leu Pro Tyr Val Glu Val Val Ser Ser Glu Arg Glu Met Ile
                180                 185                 190

AAG CGG TTC CTC AAG GTG ATA AGG GAG AAA GAT CCC GAT GTT ATA ATT 983
Lys Arg Phe Leu Lys Val Ile Arg Glu Lys Asp Pro Asp Val Ile Ile
            195                 200                 205

ACC TAC AAC GGC GAT TCT TTC GAC CTT CCC TAT CTA GTT AAG AGG GCC 1031
Thr Tyr Asn Gly Asp Ser Phe Asp Leu Pro Tyr Leu Val Lys Arg Ala
        210                 215                 220

GAA AAG CTC GGG ATA AAG CTA CCC CTG GGA AGG GAC GGT AGT GAG CCA 1079
Glu Lys Leu Gly Ile Lys Leu Pro Leu Gly Arg Asp Gly Ser Glu Pro
    225                 230                 235

AAG ATG CAG AGG CTT GGG GAT ATG ACA GCG GTG GAG ATA AAG GGA AGG 1127
Lys Met Gln Arg Leu Gly Asp Met Thr Ala Val Glu Ile Lys Gly Arg
240                 245                 250                 255

ATA CAC TTT GAC CTC TAC CAC GTG ATT AGG AGA ACG ATA AAC CTC CCA 1175
Ile His Phe Asp Leu Tyr His Val Ile Arg Arg Thr Ile Asn Leu Pro
                260                 265                 270

ACA TAC ACC CTC GAG GCA GTT TAT GAG GCA ATC TTC GGA AAG CCA AAG 1223
Thr Tyr Thr Leu Glu Ala Val Tyr Glu Ala Ile Phe Gly Lys Pro Lys
            275                 280                 285

GAG AAA GTT TAC GCT CAC GAG ATA GCT GAG GCC TGG GAG ACT GGA AAG 1271
Glu Lys Val Tyr Ala His Glu Ile Ala Glu Ala Trp Glu Thr Gly Lys
        290                 295                 300

GGA CTG GAG AGA GTT GCA AAG TAT TCA ATG GAG GAT GCA AAG GTA ACG 1319
Gly Leu Glu Arg Val Ala Lys Tyr Ser Met Glu Asp Ala Lys Val Thr
    305                 310                 315

TAC GAG CTC GGT AGG GAG TTC TTC CCA ATG GAG GCC CAG CTT TCA AGG 1367
Tyr Glu Leu Gly Arg Glu Phe Phe Pro Met Glu Ala Gln Leu Ser Arg
320                 325                 330                 335

TTA GTC GGC CAG CCC CTG TGG GAT GTT TCT AGG TCT TCA ACT GGC AAC 1415
Leu Val Gly Gln Pro Leu Trp Asp Val Ser Arg Ser Ser Thr Gly Asn
                340                 345                 350

TTG GTG GAG TGG TAC CTC CTC AGG AAG GCC TAC GAG AGG AAT GAA TTG 1463
Leu Val Glu Trp Tyr Leu Leu Arg Lys Ala Tyr Glu Arg Asn Glu Leu
            355                 360                 365

GCT CCA AAC AAG CCG GAT GAG AGG GAG TAC GAG AGA AGG CTA AGG GAG 1511
Ala Pro Asn Lys Pro Asp Glu Arg Glu Tyr Glu Arg Arg Leu Arg Glu
        370                 375                 380

AGC TAC GCT GGG GGA TAC GTT AAG GAG CCG GAG AAA GGG CTC TGG GAG 1559
Ser Tyr Ala Gly Gly Tyr Val Lys Glu Pro Glu Lys Gly Leu Trp Glu
    385                 390                 395

GGG TTA GTT TCC CTA GAT TTC AGG AGC CTG TAC CCC TCG ATA ATA ATC 1607
Gly Leu Val Ser Leu Asp Phe Arg Ser Leu Tyr Pro Ser Ile Ile Ile
400                 405                 410                 415

ACC CAT AAC GTC TCA CCG GAT ACG CTG AAC AGG GAA GGG TGT AGG GAA 1655
Thr His Asn Val Ser Pro Asp Thr Leu Asn Arg Glu Gly Cys Arg Glu
                420                 425                 430

TAC GAT GTC GCC CCA GAG GTT GGG CAC AAG TTC TGC AAG GAC TTC CCG 1703
Tyr Asp Val Ala Pro Glu Val Gly His Lys Phe Cys Lys Asp Phe Pro
            435                 440                 445

GGG TTT ATC CCC AGC CTG CTC AAG AGG TTA TTG GAT GAA AGG CAA GAA 1751
Gly Phe Ile Pro Ser Leu Leu Lys Arg Leu Leu Asp Glu Arg Gln Glu
        450                 455                 460

ATA AAA AGG AAG ATG AAA GCT TCT AAA GAC CCA ATC GAG AAG AAG ATG 1799
Ile Lys Arg Lys Met Lys Ala Ser Lys Asp Pro Ile Glu Lys Lys Met
    465                 470                 475

CTT GAT TAC AGG CAA CGG GCA ATC AAA ATC CTG GCA AAC AGC ATT TTA 1847
Leu Asp Tyr Arg Gln Arg Ala Ile Lys Ile Leu Ala Asn Ser Ile Leu
480                 485                 490                 495

CCG GAA GAA TGG GTT CCA CTA ATT AAA AAC GGT AAA GTT AAG ATA TTC 1895
Pro Glu Glu Trp Val Pro Leu Ile Lys Asn Gly Lys Val Lys Ile Phe
                500                 505                 510

CGC ATT GGG GAC TTC GTT GAT GGA CTT ATG AAG GCG AAC CAA GGA AAA 1943
Arg Ile Gly Asp Phe Val Asp Gly Leu Met Lys Ala Asn Gln Gly Lys
            515                 520                 525

GTG AAG AAA ACG GGG GAT ACA GAA GTT TTA GAA GTT GCA GGA ATT CAT 1991
Val Lys Lys Thr Gly Asp Thr Glu Val Leu Glu Val Ala Gly Ile His
        530                 535                 540

GCG TTT TCC TTT GAC AGG AAG TCC AAG AAG GCC CGT GTA ATG GCA GTG 2039
Ala Phe Ser Phe Asp Arg Lys Ser Lys Lys Ala Arg Val Met Ala Val
    545                 550                 555

AAA GCC GTG ATA AGA CAC CGT TAT TCC GGA AAT GTT TAT AGA ATA GTC 2087
Lys Ala Val Ile Arg His Arg Tyr Ser Gly Asn Val Tyr Arg Ile Val
560                 565                 570                 575

TTA AAC TCT GGT AGA AAA ATA ACA ATA ACA GAA GGG CAT AGC CTA TTT 2135
Leu Asn Ser Gly Arg Lys Ile Thr Ile Thr Glu Gly His Ser Leu Phe
                580                 585                 590

GTC TAT AGG AAC GGG GAT CTC GTT GAG GCA ACT GGG GAG GAT GTC AAA 2183
Val Tyr Arg Asn Gly Asp Leu Val Glu Ala Thr Gly Glu Asp Val Lys
            595                 600                 605

ATT GGG GAT CTT CTT GCA GTT CCA AGA TCA GTA AAC CTA CCA GAG AAA 2231
Ile Gly Asp Leu Leu Ala Val Pro Arg Ser Val Asn Leu Pro Glu Lys
        610                 615                 620

AGG GAA CGC TTG AAT ATT GTT GAA CTT CTT CTG AAT CTC TCA CCG GAA 2279
Arg Glu Arg Leu Asn Ile Val Glu Leu Leu Leu Asn Leu Ser Pro Glu
    625                 630                 635

GAG ACA GAA GAT ATA ATA CTT ACG ATT CCA GTT AAA GGC AGA AAG AAC 2327
Glu Thr Glu Asp Ile Ile Leu Thr Ile Pro Val Lys Gly Arg Lys Asn
640                 645                 650                 655

TTC TTC AAG GGA ATG TTG AGA ACA TTA CGT TGG ATT TTT GGT GAG GAA 2375
Phe Phe Lys Gly Met Leu Arg Thr Leu Arg Trp Ile Phe Gly Glu Glu
                660                 665                 670

AAG AGA GTA AGG ACA GCG AGC CGC TAT CTA AGA CAC CTT GAA AAT CTC 2423
Lys Arg Val Arg Thr Ala Ser Arg Tyr Leu Arg His Leu Glu Asn Leu
            675                 680                 685

GGA TAC ATA AGG TTG AGG AAA ATT GGA TAC GAC ATC ATT GAT AAG GAG 2471
Gly Tyr Ile Arg Leu Arg Lys Ile Gly Tyr Asp Ile Ile Asp Lys Glu
        690                 695                 700

GGG CTT GAG AAA TAT AGA ACG TTG TAC GAG AAA CTT GTT GAT GTT GTC 2519
Gly Leu Glu Lys Tyr Arg Thr Leu Tyr Glu Lys Leu Val Asp Val Val
    705                 710                 715

CGC TAT AAT GGC AAC AAG AGA GAG TAT TTA GTT GAA TTT AAT GCT GTC 2567
Arg Tyr Asn Gly Asn Lys Arg Glu Tyr Leu Val Glu Phe Asn Ala Val
720                 725                 730                 735

CGG GAC GTT ATC TCA CTA ATG CCA GAG GAA GAA CTG AAG GAA TGG CGT 2615
Arg Asp Val Ile Ser Leu Met Pro Glu Glu Glu Leu Lys Glu Trp Arg
                740                 745                 750

ATT GGA ACT AGA AAT GGA TTC AGA ATG GGT ACG TTC GTA GAT ATT GAT 2663
Ile Gly Thr Arg Asn Gly Phe Arg Met Gly Thr Phe Val Asp Ile Asp
            755                 760                 765

GAA GAT TTT GCC AAG CTT CTT GGC TAC TAT GTG AGC GAG GGA AGT GCG 2711
Glu Asp Phe Ala Lys Leu Leu Gly Tyr Tyr Val Ser Glu Gly Ser Ala
        770                 775                 780

AGG AAG TGG AAG AAT CAA ACT GGA GGT TGG AGT TAC ACT GTG AGA TTG 2759
Arg Lys Trp Lys Asn Gln Thr Gly Gly Trp Ser Tyr Thr Val Arg Leu
    785                 790                 795

TAC AAC GAG AAC GAT GAA GTT CTT GAC GAC ATG GAA CAC TTA GCC AAG 2807
Tyr Asn Glu Asn Asp Glu Val Leu Asp Asp Met Glu His Leu Ala Lys
800                 805                 810                 815

AAG TTT TTT GGG AAA GTC AAA CGT GGA AAG AAC TAT GTT GAG ATA CCA 2855
Lys Phe Phe Gly Lys Val Lys Arg Gly Lys Asn Tyr Val Glu Ile Pro
                820                 825                 830

AAG AAA ATG GCT TAT ATC ATC TTT GAG AGC CTT TGT GGG ACT TTG GCA 2903
Lys Lys Met Ala Tyr Ile Ile Phe Glu Ser Leu Cys Gly Thr Leu Ala
            835                 840                 845

GAA AAC AAA AGG GTT CCT GAG GTA ATC TTT ACC TCA TCA AAG GGC GTT 2951
Glu Asn Lys Arg Val Pro Glu Val Ile Phe Thr Ser Ser Lys Gly Val
        850                 855                 860

AGA TGG GCC TTC CTT GAG GGT TAT TTC ATC GGC GAT GGC GAT GTT CAC 2999
Arg Trp Ala Phe Leu Glu Gly Tyr Phe Ile Gly Asp Gly Asp Val His
    865                 870                 875

CCA AGC AAG AGG GTT CGC CTA TCA ACG AAG AGC GAG CTT TTA GTA AAT 3047
Pro Ser Lys Arg Val Arg Leu Ser Thr Lys Ser Glu Leu Leu Val Asn
880                 885                 890                 895

GGC CTT GTT CTC CTA CTT AAC TCC CTT GGA GTA TCT GCC ATT AAG CTT 3095
Gly Leu Val Leu Leu Leu Asn Ser Leu Gly Val Ser Ala Ile Lys Leu
                900                 905                 910

GGA TAC GAT AGC GGA GTC TAC AGG GTT TAT GTA AAC GAG GAA CTT AAG 3143
Gly Tyr Asp Ser Gly Val Tyr Arg Val Tyr Val Asn Glu Glu Leu Lys
            915                 920                 925

TTT ACG GAA TAC AGA AAG AAA AAG AAT GTA TAT CAC TCT CAC ATT GTT 3191
Phe Thr Glu Tyr Arg Lys Lys Lys Asn Val Tyr His Ser His Ile Val
        930                 935                 940

CCA AAG GAT ATT CTC AAA GAA ACT TTT GGT AAG GTC TTC CAG AAA AAT 3239
Pro Lys Asp Ile Leu Lys Glu Thr Phe Gly Lys Val Phe Gln Lys Asn
    945                 950                 955

ATA AGT TAC AAG AAA TTT AGA GAG CTT GTA GAA AAT GGA AAA CTT GAC 3287
Ile Ser Tyr Lys Lys Phe Arg Glu Leu Val Glu Asn Gly Lys Leu Asp
960                 965                 970                 975

AGG GAG AAA GCC AAA CGC ATT GAG TGG TTA CTT AAC GGA GAT ATA GTC 3335
Arg Glu Lys Ala Lys Arg Ile Glu Trp Leu Leu Asn Gly Asp Ile Val
                980                 985                 990

CTA GAT AGA GTC GTA GAG ATT AAG AGA GAG TA[?] TAT GAT GGT TAC GTT 3383
Leu Asp Arg Val Val Glu Ile Lys Arg Glu T[?] Tyr Asp Gly Tyr Val
995 1000 1005

TAC GAT CTA AGT GTC GAT GAA GAT GAG AAT TTC CTT GCT GGC TTT GGA 3431
Tyr Asp Leu Ser Val Asp Glu Asp Glu Asn Phe Leu Ala Gly Phe Gly
1010 1015 1020

TTC CTC TAT GCA CAT AAT AGC TAT TAT GGG TAT TAT GGG TAC GCA AAA 3479
Phe Leu Tyr Ala His Asn Ser Tyr Tyr Gly Tyr Tyr Gly Tyr Ala Lys
1025 1030 1035

GCC CGT TGG TAC TGT AAG GAG TGC GCA GAG AGC GTT ACG GCC TGG GGG 3527
Ala Arg Trp Tyr Cys Lys Glu Cys Ala Glu Ser Val Thr Ala Trp Gly
1040 1045 1050 1055

AGG GAA TAT ATA GAG TTC GTA AGG AAG GAA CTG GAG GAA AAG TTC GGG 3575
Arg Glu Tyr Ile Glu Phe Val Arg Lys Glu Leu Glu Glu Lys Phe Gly
1060 1065 1070

TTC AAA GTC TTA TAC ATA GAC ACA GAT GGA CTC TAC GCC ACA ATT CCT 3623
Phe Lys Val Leu Tyr Ile Asp Thr Asp Gly Leu Tyr Ala Thr Ile Pro
1075 1080 1085

GGG GCA AAA CCC GAG GAG ATA AAG AAG AAA GCC CTA GAG TTC GTA GAT 3671
Gly Ala Lys Pro Glu Glu Ile Lys Lys Lys Ala Leu Glu Phe Val Asp
1090 1095 1100

TAT ATA AAC GCC AAG CTC CCA GGG CTG TTG GAG CTT GAG TAC GAG GGC 3719
Tyr Ile Asn Ala Lys Leu Pro Gly Leu Leu Glu Leu Glu Tyr Glu Gly
1105 1110 1115

TTC TAC GTG AGA GGG TTC TTC GTG ACG AAG AAG AAG TAT GCG TTG ATA 3767
Phe Tyr Val Arg Gly Phe Phe Val Thr Lys Lys Lys Tyr Ala Leu Ile
1120 1125 1130 1135

GAT GAG GAA GGG AAG ATA ATC ACT AGG GGG CTT GAA ATA GTC AGG AGG 3815
Asp Glu Glu Gly Lys Ile Ile Thr Arg Gly Leu Glu Ile Val Arg Arg
1140 1145 1150

GAC TGG AGC GAA ATA GCC AAA GAA ACC CAA GCA AAA GTC CTA GAG GCT 3863
Asp Trp Ser Glu Ile Ala Lys Glu Thr Gln Ala Lys Val Leu Glu Ala
1155 1160 1165

ATC CTA AAG CAT GGC AAC GTT GAG GAG GCA GTA AAG ATA GTT AAG GAG 3911
Ile Leu Lys His Gly Asn Val Glu Glu Ala Val Lys Ile Val Lys Glu
1170 1175 1180

GTA ACT GAA AAG CTG AGC AAG TAC GAA ATA CCT CCA GAA AAG CTA GTT 3959
Val Thr Glu Lys Leu Ser Lys Tyr Glu Ile Pro Pro Glu Lys Leu Val
1185 1190 1195

ATT TAC GAG CAG ATC ACG AGG CCC CTT CAC GAG TAC AAG GCT ATA GGT 4007
Ile Tyr Glu Gln Ile Thr Arg Pro Leu His Glu Tyr Lys Ala Ile Gly
1200 1205 1210 1215

CCG CAC GTT GCC GTG GCA AAA AGG TTA GCC GCT AGA GGA GTA AAG GTG 4055
Pro His Val Ala Val Ala Lys Arg Leu Ala Ala Arg Gly Val Lys Val
1220 1225 1230

AGG CCT GGC ATG GTG ATA GGG TAC ATA GTG CTG AGG GGA GAC GGG CCA 4103
Arg Pro Gly Met Val Ile Gly Tyr Ile Val Leu Arg Gly Asp Gly Pro
1235 1240 1245

ATA AGC AAG AGG GCT ATC CTT GCA GAG GAG TTC GAT CTC AGG AAG CAT 4151
Ile Ser Lys Arg Ala Ile Leu Ala Glu Glu Phe Asp Leu Arg Lys His
1250 1255 1260

AAG TAT GAC GCT GAG TAT TAC ATA GAA AAT CAG GTT TTA CCT GCC GTT 4199
Lys Tyr Asp Ala Glu Tyr Tyr Ile Glu Asn Gln Val Leu Pro Ala Val
1265 1270 1275

CTT AGA ATA TTA GAG GCC TTT GGG TAC AGG AAA GAA GAC CTC AGG TGG 4247
Leu Arg Ile Leu Glu Ala Phe Gly Tyr Arg Lys Glu Asp Leu Arg Trp
1280 1285 1290 1295

CAG AAG ACT AAA CAG ACA GGT CTT ACG GCA TGG CTT AAC ATC AAG AAG 4295
Gln Lys Thr Lys Gln Thr Gly Leu Thr Ala Trp Leu Asn Ile Lys Lys
1300 1305 1310

AAG TAATGTTTAT GTACTCGTAA TGCGAGTATT AAGTGGGTGA TGAGATGGCA 4348
Lys

GTATTGAGCA TAAGGATTCC GGATGATCTA AAAGAGAAGA TGAAGGAGTT TGACATAAAC 4408

TGGAGTGAGG AGATCAGGAA GTTCATAAAA GAGAGGATAG AGTATGAGGA AAGGAAGAGA 4468

ACCCTTGAGA AAGCTCTAGA ACTTCTAAAG AATACTCCAG GATCAGTCGA GAGAGGATTT 4528

TCAGCAAGGG CAGTGAGGGA GGATCGTGAT AGTCATTGAT GCATCAATCC TAGCTAAAAT 4588

AATTCTAAAA GAAGAGGGCT GGGAACAGAT AACTCTTACA CCGAGCACGA TAACTTTGGA 4648

CTATGCTTTT GTTGAATGTA CAAACGCAAT ATGGAAGGCT GTCAGGCGGA ACAGGATCC 4707

(2) INFORMATION FOR SEQ ID NO:3:

(i) SEQUENCE CHARACTERISTICS:
(A) LENGTH: 23 base pairs
(B) TYPE: nucleic acid
(C) STRANDEDNESS: single
(D) TOPOLOGY: linear

(xi) SEQUENCE DESCRIPTION: SEQ ID NO:3:

AGTGTCTCCG GAGAAAGTGA GAT 23

(2) INFORMATION FOR SEQ ID NO:4:

(i) SEQUENCE CHARACTERISTICS:
(A) LENGTH: 22 base pairs
(B) TYPE: nucleic acid
(C) STRANDEDNESS: single
(D) TOPOLOGY: linear

(xi) SEQUENCE DESCRIPTION: SEQ ID NO:4:

AGTATTGTGT ACCAGGATGT TG 22

(2) INFORMATION FOR SEQ ID NO:5:

(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:5:

AGCATTTTAC CGGAAGAATG GGTT 24

(2) INFORMATION FOR SEQ ID NO:6:

(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:6:

GCTATTATGT GCATAGAGGA ATCCA 25

(2) INFORMATION FOR SEQ ID NO:7:

(i) SEQUENCE CHARACTERISTICS:
(A) LENGTH: 24 base pairs
(B) TYPE: nucleic acid
(C) STRANDEDNESS: single
(D) TOPOLOGY: linear

(xi) SEQUENCE DESCRIPTION: SEQ ID NO:7:

AGGGTCGACA GATTTGATCC AGCG 24

(2) INFORMATION FOR SEQ ID NO:8:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:8:

GAGAACTTTG TTCGTACCTG 20
(2) INFORMATION FOR SEQ ID NO:9:

(i) SEQUENCE CHARACTERISTICS:
(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO:9:

GGTATTATTT CTTCTAAAGC A 21

(2) INFORMATION FOR SEQ ID NO:10:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:10:

GTTGTTTGTT GGTTTTACCA 20

(2) INFORMATION FOR SEQ ID NO:11:

(i) SEQUENCE CHARACTERISTICS:
(A) LENGTH: 20 base pairs
(B) TYPE: nucleic acid

(C) STRANDEDNESS: single
(D) TOPOLOGY: linear

(xi) SEQUENCE DESCRIPTION: SEQ ID NO:11:

ATGGCAAATG CTGTATGGAT 20

(2) INFORMATION FOR SEQ ID NO:12:

(i) SEQUENCE CHARACTERISTICS:
(A) LENGTH: 23 base pairs
(B) TYPE: nucleic acid
(C) STRANDEDNESS: single
(D) TOPOLOGY: linear

(xi) SEQUENCE DESCRIPTION: SEQ ID NO:12:

AGTGTCTCCG GAGAAAGTGA GAT 23

(2) INFORMATION FOR SEQ ID NO:13:

(i) SEQUENCE CHARACTERISTICS:
(A) LENGTH: 26 base pairs
(B) TYPE: nucleic acid
(C) STRANDEDNESS: single
(D) TOPOLOGY: linear

(xi) SEQUENCE DESCRIPTION: SEQ ID NO:13:

ATTGTGTACT AGTATGTTGT TTGCAA 26

(2) INFORMATION FOR SEQ ID NO:14:

(i) SEQUENCE CHARACTERISTICS:
(A) LENGTH: 37 base pairs
(B) TYPE: nucleic acid
(C) STRANDEDNESS: single
(D) TOPOLOGY: linear

(xi) SEQUENCE DESCRIPTION: SEQ ID NO:14:

GCCTCCGGAG ACACTATCGC CAAAATCACC GCCGTAA 37

(2) INFORMATION FOR SEQ ID NO:15:

(i) SEQUENCE CHARACTERISTICS:
(A) LENGTH: 38 base pairs
(B) TYPE: nucleic acid
(C) STRANDEDNESS: single
(D) TOPOLOGY: linear

(xi) SEQUENCE DESCRIPTION: SEQ ID NO:15:

GCCACTAGTA CACAATACGC CGAACGATCG CCAGTTCT 38

(2) INFORMATION FOR SEQ ID NO:16:

(i) SEQUENCE CHARACTERISTICS:
(A) LENGTH: 26 base pairs
(B) TYPE: nucleic acid
(C) STRANDEDNESS: single
(D) TOPOLOGY: linear

(xi) SEQUENCE DESCRIPTION: SEQ ID NO:16:

CCTTCTAGAC CGGTGCAGTA TGAAGG 26

(2) INFORMATION FOR SEQ ID NO:17:

(i) SEQUENCE CHARACTERISTICS:
(A) LENGTH: 36 base pairs
(B) TYPE: nucleic acid
(C) STRANDEDNESS: single
(D) TOPOLOGY: linear

(xi) SEQUENCE DESCRIPTION: SEQ ID NO:17:

GCCGTCGACC CTAGTGTCTC AGGAGAAAGT GAGATC 36

(2) INFORMATION FOR SEQ ID NO:18:

(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:18:

GCCTCTAGAA TTGTGTACCA GGATGTTGTT TGC 33

(2) INFORMATION FOR SEQ ID NO:19:

(i) SEQUENCE CHARACTERISTICS:
(A) LENGTH: 23 base pairs
(B) TYPE: nucleic acid
(C) STRANDEDNESS: single
(D) TOPOLOGY: linear

(xi) SEQUENCE DESCRIPTION: SEQ ID NO:19:

GCAAAGAACC GGTGCGTCTC TTC 23

(2) INFORMATION FOR SEQ ID NO:20:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:20:

AGCAACAGAG TTACCTCTTG 20

(2) INFORMATION FOR SEQ ID NO:21:

(i) SEQUENCE CHARACTERISTICS:
(A) LENGTH: 33 base pairs
(B) TYPE: nucleic acid
(C) STRANDEDNESS: single
(D) TOPOLOGY: linear

(xi) SEQUENCE DESCRIPTION: SEQ ID NO:21:

CAGTTTCCAG CTCCTACAAT GAGACCTACG AGC 33

(2) INFORMATION FOR SEQ ID NO:22:

(i) SEQUENCE CHARACTERISTICS:
(A) LENGTH: 20 base pairs
(B) TYPE: nucleic acid
(C) STRANDEDNESS: single
(D) TOPOLOGY: linear

(xi) SEQUENCE DESCRIPTION: SEQ ID NO:22:

GTAGTGTCGA CCCCATGCGG 20

(2) INFORMATION FOR SEQ ID NO:23:

(i) SEQUENCE CHARACTERISTICS:
(A) LENGTH: 28 base pairs
(B) TYPE: nucleic acid
(C) STRANDEDNESS: single
(D) TOPOLOGY: linear

(xi) SEQUENCE DESCRIPTION: SEQ ID NO:23:

CGTTTTGCCT GATTATTATC TCACTTTC 28

(2) INFORMATION FOR SEQ ID NO:24:

(i) SEQUENCE CHARACTERISTICS:
(A) LENGTH: 22 base pairs
(B) TYPE: nucleic acid
(C) STRANDEDNESS: single
(D) TOPOLOGY: linear

(xi) SEQUENCE DESCRIPTION: SEQ ID NO:24:

GTCCACCTTC GAAAAAAGAT CC 22

(2) INFORMATION FOR SEQ ID NO:25:

(i) SEQUENCE CHARACTERISTICS:
(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO:25:

CCGCATAAAG GACCTTAAAG C 21

(2) INFORMATION FOR SEQ ID NO:26:

(i) SEQUENCE CHARACTERISTICS:
(A) LENGTH: 23 base pairs
(B) TYPE: nucleic acid
(C) STRANDEDNESS: single
(D) TOPOLOGY: linear
(xi) SEQUENCE DESCRIPTION: SEQ ID NO:26:

GAGGAAGAGA TCATCATCAT AGC 23

(2) INFORMATION FOR SEQ ID NO:27:

(i) SEQUENCE CHARACTERISTICS:
(A) LENGTH: 40 base pairs
(B) TYPE: nucleic acid
(C) STRANDEDNESS: double

(D) TOPOLOGY: linear

(xi) SEQUENCE DESCRIPTION: SEQ ID NO:27:

GTCCTTCGTG CGGACAGTGT CTCAGGAGAA AGTGAGATAA 40

(2) INFORMATION FOR SEQ ID NO:28:

(i) SEQUENCE CHARACTERISTICS:
(A) LENGTH: 40 base pairs
(B) TYPE: nucleic acid
(C) STRANDEDNESS: double
(D) TOPOLOGY: linear

(xi) SEQUENCE DESCRIPTION: SEQ ID NO:28:

GTCCTTTATG CGGACTAGGT CTCAGGAGAA AGTGAGATAA 40

(2) INFORMATION FOR SEQ ID NO:29:

(i) SEQUENCE CHARACTERISTICS:
(A) LENGTH: 61 base pairs
(B) TYPE: nucleic acid
(C) STRANDEDNESS: double
(D) TOPOLOGY: linear

(xi) SEQUENCE DESCRIPTION: SEQ ID NO:29:

CCGGTTCTTT GCAAACAACA TCCTGGTACA CAATTAAGAC GGCTTTTATG CCACAATACC 60

C 61

(2) INFORMATION FOR SEQ ID NO:30:

(i) SEQUENCE CHARACTERISTICS:
(A) LENGTH: 22 amino acids
(B) TYPE: amino acid
(C) STRANDEDNESS: unknown
(D) TOPOLOGY: unknown

(xi) SEQUENCE DESCRIPTION: SEQ ID NO:30:

Ile Lys Ile Leu Ala Asn Ser Ile Leu Pro Glu Glu Trp Val Pro Leu
1               5                   10                  15

Ile Lys Asn Gly Lys Val
            20

(2) INFORMATION FOR SEQ ID NO:31:

(i) SEQUENCE CHARACTERISTICS:
(A) LENGTH: 22 amino acids
(B) TYPE: amino acid
(C) STRANDEDNESS: unknown
(D) TOPOLOGY: unknown

(xi) SEQUENCE DESCRIPTION: SEQ ID NO:31:

Ile Lys Leu Leu Ala Asn Ser Ile Leu Pro Asn Glu Trp Leu Pro
1               5                   10                  15

Ile Ile Glu Asn Gly Glu Ile
                20

(2) INFORMATION FOR SEQ ID NO:32:

(i) SEQUENCE CHARACTERISTICS:
(A) LENGTH: 22 amino acids
(B) TYPE: amino acid
(C) STRANDEDNESS: unknown
(D) TOPOLOGY: unknown

(xi) SEQUENCE DESCRIPTION: SEQ ID NO:32:

Lys Val Leu Tyr Ala Asp Ser Val Ser Gly Glu Ser Glu Ile Ile Ile
1               5                   10                  15

Arg Gln Asn Gly Lys Ile
            20

(2) INFORMATION FOR SEQ ID NO:33:

(i) SEQUENCE CHARACTERISTICS:
(A) LENGTH: 22 amino acids
(B) TYPE: amino acid
(C) STRANDEDNESS: unknown
(D) TOPOLOGY: unknown

(xi) SEQUENCE DESCRIPTION: SEQ ID NO:33:

Ala Ile Leu Tyr Val Gly Cys Gly Ala Lys Gly Thr Asn Val Leu Met
1               5                   10                  15

Ala Asp Gly Ser Ile Glu
            20

(2) INFORMATION FOR SEQ ID NO:34:
(i) SEQUENCE CHARACTERISTICS:
(A) LENGTH: 22 amino acids
(B) TYPE: amino acid
(C) STRANDEDNESS: unknown
(D) TOPOLOGY: unknown

(xi) SEQUENCE DESCRIPTION: SEQ ID NO:34:

Lys Val Val Lys Asn Lys Cys Leu Ala Glu Gly Thr Arg Ile Arg Asp
1               5                   10                  15

Pro Val Thr Gly Thr Thr
            20

(2) INFORMATION FOR SEQ ID NO:35:

(i) SEQUENCE CHARACTERISTICS:
(A) LENGTH: 23 amino acids
(B) TYPE: amino acid
(C) STRANDEDNESS: unknown
(D) TOPOLOGY: unknown

(xi) SEQUENCE DESCRIPTION: SEQ ID NO:35:

Glu Asx Gly Lys Ala Gly Phe Gly Phe Leu Tyr Ala His Asn Ser Tyr
1               5                   10                  15

Tyr Gly Tyr Tyr Gly Tyr Ala
            20

(2) INFORMATION FOR SEQ ID NO:36:

(i) SEQUENCE CHARACTERISTICS:
(A) LENGTH: 23 amino acids
(B) TYPE: amino acid
(C) STRANDEDNESS: unknown
(D) TOPOLOGY: unknown

(xi) SEQUENCE DESCRIPTION: SEQ ID NO:36:

Glu Asn Phe Leu Val Gly Phe Gly Leu Leu Tyr Ala His Asn Ser Tyr
1               5                   10                  15

Tyr Gly Tyr Met Gly Tyr Pro
            20

(2) INFORMATION FOR SEQ ID NO:37:

(i) SEQUENCE CHARACTERISTICS:
(A) LENGTH: 23 amino acids
(B) TYPE: amino acid
(C) STRANDEDNESS: unknown
(D) TOPOLOGY: unknown

(xi) SEQUENCE DESCRIPTION: SEQ ID NO:37:

Glu Thr His Arg Phe Phe Ala Asn Asn Ile Leu Val His Asn Thr Asp
1               5                   10                  15

Gly Phe Tyr Ala Thr Ile Pro
            20

(2) INFORMATION FOR SEQ ID NO:38:

(i) SEQUENCE CHARACTERISTICS:
(A) LENGTH: 23 amino acids
(B) TYPE: amino acid
(C) STRANDEDNESS: unknown
(D) TOPOLOGY: unknown

(xi) SEQUENCE DESCRIPTION: SEQ ID NO:38:

Asp His Gln Phe Leu Leu Ala Asn Gln Val Val Val His Asn Cys Gly
1               5                   10                  15

Glu Arg Gly Asn Glu Met Ala
            20

(2) INFORMATION FOR SEQ ID NO:39:

(i) SEQUENCE CHARACTERISTICS:
(A) LENGTH: 23 amino acids
(B) TYPE: amino acid
(C) STRANDEDNESS: unknown
(D) TOPOLOGY: unknown

(xi) SEQUENCE DESCRIPTION: SEQ ID NO:39:

Glu Leu His Thr Leu Val Ala Glu Gly Val Val Val His Asn Cys Ser
1               5                   10                  15

Pro Pro Phe Lys Gln Ala Glu
            20

(2) INFORMATION FOR SEQ ID NO:40:

(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:40:

GCAATTATGT GCATAGAGGA ATCCA 25

(2) INFORMATION FOR SEQ ID NO:41:

(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:41:

GGTATTATGT GCATAGAGGA ATCCA 25

(2) INFORMATION FOR SEQ ID NO:42:

(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:42:

ATTATGTGCA TAGAGGAATC CAAAG 25

(2) INFORMATION FOR SEQ ID NO:43:

(i) SEQUENCE CHARACTERISTICS:
(A) LENGTH: 42 base pairs
(B) TYPE: nucleic acid
(C) STRANDEDNESS: single
(D) TOPOLOGY: linear

(xi) SEQUENCE DESCRIPTION: SEQ ID NO:43:

GGTACCCGTC GTGCTAGCAT TTTACCGGAA GAATGGGTAC CA 42

(2) INFORMATION FOR SEQ ID NO:44:

(i) SEQUENCE CHARACTERISTICS:
(A) LENGTH: 27 base pairs
(B) TYPE: nucleic acid
(C) STRANDEDNESS: single
(D) TOPOLOGY: linear

(xi) SEQUENCE DESCRIPTION: SEQ ID NO:44

CCCGCTATTA TGTGCATAGA GGGATCC 27

(2) INFORMATION FOR SEQ ID NO:45

(i) SEQUENCE CHARACTERISTICS:
(A) LENGTH: 4 amino acids
(B) TYPE: amino acid

(C) STRANDEDNESS: unknown
(D) TOPOLOGY: unknown
(ix) FEATURE
(A) NAME/KEY: peptide
(B) LOCATION: 1
(D) OTHER INFORMATION: /note= "Xaa at position 1 = (Ala/Val)"
(ix) FEATURE
(A) NAME/KEY: peptide
(B) LOCATION: 4
(D) OTHER INFORMATION: /note= "Xaa at position 4 = (Ser/Cys/Thr)"

(xi) SEQUENCE DESCRIPTION: SEQ ID NO:45

Xaa His Asn Xaa 4
1

(2) INFORMATION FOR SEQ ID NO:46

(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:46

GATCCCTCTA TGCACATAAT TCAGGCCTC 29

(2) INFORMATION FOR SEQ ID NO:47

(i) SEQUENCE CHARACTERISTICS:
(A) LENGTH: 29 base pairs
(B) TYPE: nucleic acid
(C) STRANDEDNESS: single
(D) TOPOLOGY: linear

(xi) SEQUENCE DESCRIPTION: SEQ ID NO:47:

AATTGAGGCC TGAATTATGT GCATAGAGG 29

(2) INFORMATION FOR SEQ ID NO:48:

(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:48:

GCTCGAGGCT AGCATTTTAC CGGAAGAATG GGTAC 35

(2) INFORMATION FOR SEQ ID NO:49:

(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:49:

CCATTCTTCC GGTAAAATGC TAGCCTCGAG CGTAC 35

(2) INFORMATION FOR SEQ ID NO:50:

(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:50:

GATCCCTCTA TAAGCATAAT TCAGG 25

(2) INFORMATION FOR SEQ ID NO:51:

(i) SEQUENCE CHARACTERISTICS:
(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO:51:

CCTGAATTAT GCTTATAGAG G 21

(2) INFORMATION FOR SEQ ID NO:52:

(i) SEQUENCE CHARACTERISTICS:
(A) LENGTH: 25 base pairs
(B) TYPE: nucleic acid
(C) STRANDEDNESS: single
(D) TOPOLOGY: linear

(xi) SEQUENCE DESCRIPTION: SEQ ID NO:52:

GATCCCTCTA TGCACTGAAT TCAGG 25

(2) INFORMATION FOR SEQ ID NO:53:

(i) SEQUENCE CHARACTERISTICS:
(A) LENGTH: 21 base pairs
(B) TYPE: nucleic acid
(C) STRANDEDNESS: single
(D) TOPOLOGY: linear

(xi) SEQUENCE DESCRIPTION: SEQ ID NO:53:

CCTGAATTCA GTGCATAGAG G 21

(2) INFORMATION FOR SEQ ID NO:54:

(i) SEQUENCE CHARACTERISTICS:
(A) LENGTH: 32 base pairs
(B) TYPE: nucleic acid
(C) STRANDEDNESS: single
(D) TOPOLOGY: linear

(xi) SEQUENCE DESCRIPTION: SEQ ID NO:54:

GTCAGGCCTC TCAGACAGTA CAGCTCGTAC AT 32

(2) INFORMATION FOR SEQ ID NO:55:

(i) SEQUENCE CHARACTERISTICS:
(A) LENGTH: 6 base pairs

(B) TYPE: nucleic acid
(C) STRANDEDNESS: single
(D) TOPOLOGY: linear

(xi) SEQUENCE DESCRIPTION: SEQ ID NO:55:

AGGCCT 6

(2) INFORMATION FOR SEQ ID NO:56:

(i) SEQUENCE CHARACTERISTICS:
(A) LENGTH: 35 base pairs
(B) TYPE: nucleic acid
(C) STRANDEDNESS: single
(D) TOPOLOGY: linear

(xi) SEQUENCE DESCRIPTION: SEQ ID NO:56:

CCCCTGCAGT TAAAAGTAAT TGCTTTCCAA ATAAG 35

(2) INFORMATION FOR SEQ ID NO:57:

(i) SEQUENCE CHARACTERISTICS:
(A) LENGTH: 6 base pairs
(B) TYPE: nucleic acid
(C) STRANDEDNESS: single
(D) TOPOLOGY: linear

(xi) SEQUENCE DESCRIPTION: SEQ ID NO:57:

CTGCAG 6

(2) INFORMATION FOR SEQ ID NO:58:

(i) SEQUENCE CHARACTERISTICS:
(A) LENGTH: 28 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:58:

GGAATTCCAT ATGAAAATCG AAGAAGGT 28

(2) INFORMATION FOR SEQ ID NO:59:

(i) SEQUENCE CHARACTERISTICS:
(A) LENGTH: 31 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:59:

CGGGATCCCG TTATAGTGAG ATAACGTCCC G 31

(2) INFORMATION FOR SEQ ID NO:60:

(i) SEQUENCE CHARACTERISTICS:
(A) LENGTH: 28 base pairs
(B) TYPE: nucleic acid
(C) STRANDEDNESS: single

(D) TOPOLOGY: unknown
(ii) MOLECULE TYPE: Other Nucleic Acid

(xi) SEQUENCE DESCRIPTION: SEQ ID NO:60:

GGAATTCCAT ATGCCAGAGG AAGAACTG 28

(2) INFORMATION FOR SEQ ID NO:61:

(i) SEQUENCE CHARACTERISTICS:
(A) LENGTH: 34 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:61:

ATAGTTTAGC GGCCGCTCAC GACGTTGTAA AACG 34

(2) INFORMATION FOR SEQ ID NO:62:

(i) SEQUENCE CHARACTERISTICS:
(A) LENGTH: 33 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:62:

TCGAGGCTAG CAAATTACCG GAAGAATGGG TAC 33

(2) INFORMATION FOR SEQ ID NO:63:

(i) SEQUENCE CHARACTERISTICS:
(A) LENGTH: 25 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:63:

CCATTCTTCC GGTAATTTGC TAGCC 25

(2) INFORMATION FOR SEQ ID NO:64:

(i) SEQUENCE CHARACTERISTICS:
(A) LENGTH: 33 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:64:

TCGAGGCTTG CATTTTACCG GAAGAATGGG TAC 33

(2) INFORMATION FOR SEQ ID NO:65:

(i) SEQUENCE CHARACTERISTICS:
(A) LENGTH: 25 base pairs
(B) TYPE: nucleic acid

(C) STRANDEDNESS: single
(D) TOPOLOGY: unknown
(ii) MOLECULE TYPE: Other Nucleic Acid

(xi) SEQUENCE DESCRIPTION: SEQ ID NO:65:

CCATTCTTCC GGTAAAATGC AAGCC 25

(2) INFORMATION FOR SEQ ID NO:66:

(i) SEQUENCE CHARACTERISTICS:
(A) LENGTH: 34 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:66:

GATCCCTCTA TAAGCATAAT ATTGGCATGC AGTA 34

(2) INFORMATION FOR SEQ ID NO:67:

(i) SEQUENCE CHARACTERISTICS:
(A) LENGTH: 30 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:67:

TACTGCATGC CAATATTATG CTTATAGAGG 30

(2) INFORMATION FOR SEQ ID NO:68:

(i) SEQUENCE CHARACTERISTICS:
(A) LENGTH: 29 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:68:

GATCCCTCTA TGCACATAAT TAAGGCATG 29

(2) INFORMATION FOR SEQ ID NO:69:

(i) SEQUENCE CHARACTERISTICS:
(A) LENGTH: 21 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:69:

CCTTAATTAT GTGCATAGAG G 21

(2) INFORMATION FOR SEQ ID NO:70:

(i) SEQUENCE CHARACTERISTICS:
(A) LENGTH: 33 base pairs

(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:70:

GCGCTCGAGG GGTGCTTTGC CAAGGGTACC AAT 33

(2) INFORMATION FOR SEQ ID NO:71:

(i) SEQUENCE CHARACTERISTICS:
(A) LENGTH: 28 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:71:

CCTCCGCAAT TATGGACGAC AACCTGGT 28

(2) INFORMATION FOR SEQ ID NO:72:

(i) SEQUENCE CHARACTERISTICS:
(A) LENGTH: 24 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:72:

GAATGCGGAA TTCAGGCCTC CGCA 24

(2) INFORMATION FOR SEQ ID NO:73:

(i) SEQUENCE CHARACTERISTICS:
(A) LENGTH: 39 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:73:

ATGGACGACA ACCTGGGATC CAAGCAAAAA CTGATGATC 39

(2) INFORMATION FOR SEQ ID NO:74:

(i) SEQUENCE CHARACTERISTICS:
(A) LENGTH: 35 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:74:

GATCCCAGGT TGTCGTCCAT GCATGCGGAG GCCTG 35

(2) INFORMATION FOR SEQ ID NO:75:

(i) SEQUENCE CHARACTERISTICS:

(A) LENGTH: 35 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:75:

AATTCAGGCC TCCGCATGCA TGGACGACAA CCTGG 35

(2) INFORMATION FOR SEQ ID NO:76:

(i) SEQUENCE CHARACTERISTICS:
(A) LENGTH: 36 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:76:

GATCCCAGGT TGTCGTCCAT GCATGCGGTG GCCTGA 36

(2) INFORMATION FOR SEQ ID NO:77:

(i) SEQUENCE CHARACTERISTICS:
(A) LENGTH: 36 base pairs
(B) TYPE: nucleic acid
(C) STRANDEDNESS: single
(D) TOPOLOGY: unknown
(ii) MOLECULE TYPE: Other Nucleic Acid

(xi) SEQUENCE DESCRIPTION: SEQ ID NO:77:

CCGGTCAGGC CTCCGCATGC ATGGACGACA ACCTGG 36

SEQUENCE LISTING

<110> New England Biolabs, Inc.

<120> Modified Proteins and Methods of Production

<130> NEB-036C3P DIV-EP

<150> EP 96 922 498.9
<151> 1996-06-19

<150> US 08/496,297
<151> 1995-06-28

<150> US 08/580,555
<151> 1995-12-29

<150> PCT/US96/10545
<151> 1996-06-19

<160> 78

<170> PatentIn version 3.2

<210> 1
<211> 5837
<212> DNA
<213> unknown

<220>
<223> Vent DNA polymerase gene from hyperthermophilic archaea
      Thermococcus litoralis

<400> 1
   gaattcgcga taaaatctat tttcttcctc catttttcaa tttcaaaaac gtaagcatga      60
   gccaaacctc tcgccctttc tctgtccttc ccgctaaccc tcttgaaaac tctctccaaa     120
   gcattttttg atgaaagctc acgctcctct atgagggtca gtatatctgc aatgagttcg     180
   tgaagggtta ttctgtagaa caactccatg attttcgatt tggatggggg tttaaaaatt     240
   tggcggaact tttatttaat ttgaactcca gtttatatct ggtggtattt atgatactgg     300
   acactgatta cataacaaaa gatggcaagc ctataatccg aatttttaag aaagagaacg     360
   gggagtttaa aatagaactt gaccctcatt ttcagcccta tatatatgct cttctcaaag     420
   atgactccgc tattgaggag ataaaggcaa taaagggcga gagacatgga aaaactgtga     480
   gagtgctcga tgcagtgaaa gtcaggaaaa aatttttggg aagggaagtt gaagtctgga     540
   agctcatttt cgagcatccc caagacgttc cagctatgcg gggcaaaata agggaacatc     600
   cagctgtggt tgacatttac gaatatgaca taccctttgc caagcgttat ctcatagaca     660
   agggcttgat tcccatggag ggagacgagg agcttaagct ccttgccttt gatattgaaa     720
   cgttttatca tgagggagat gaatttggaa agggcgagat aataatgatt agttatgccg     780
   atgaagaaga ggccagagta atcacatgga aaaatatcga tttgccgtat gtcgatgttg     840
   tgtccaatga aagagaaatg ataaagcgtt ttgttcaagt tgttaaagaa aaagaccccg     900
   atgtgataat aacttacaat ggggacaatt ttgatttgcc gtatctcata aaacgggcag 960
   aaaagctggg agttcggctt gtcttaggaa gggacaaaga acatcccgaa cccaagattc 1020
   agaggatggg tgatagtttt gctgtggaaa tcaagggtag aatccacttt gatcttttcc 1080
   cagttgtgcg aaggacgata aacctcccaa cgtatacgct tgaggcagtt tatgaagcag 1140
   ttttaggaaa aaccaaaagc aaattaggag cagaggaaat tgccgctata tgggaaacag 1200
   aagaaagcat gaaaaaacta gcccagtact caatggaaga tgctagggca acgtatgagc 1260
   tcgggaagga attcttcccc atggaagctg agctggcaaa gctgataggt caaagtgtat 1320
   gggacgtctc gagatcaagc accggcaacc tcgtggagtg gtatctttta agggtggcat 1380
   acgcgaggaa tgaacttgca ccgaacaaac ctgatgagga agagtataaa cggcgcttaa 1440
   gaacaactta cctgggagga tatgtaaaag agccagaaaa aggtttgtgg gaaaatatca 1500
   tttatttgga tttccgcagt ctgtaccctt caataatagt tactcacaac gtatccccag 1560
   atacccttga aaaagagggc tgtaagaatt acgatgttgc tccgatagta ggatataggt 1620
   tctgcaagga ctttccgggc tttattccct ccatactcgg ggacttaatt gcaatgaggc 1680
   aagatataaa gaagaaaatg aaatccacaa ttgacccgat cgaaaagaaa atgctcgatt 1740
   ataggcaaag ggctattaaa ttgcttgcaa acagcatctt acccaacgag tggttaccaa 1800
   taattgaaaa tggagaaata aaattcgtga aaattggcga gtttataaac tcttacatgg 1860
   aaaaacagaa ggaaaacgtt aaaacagtag agaatactga agttctcgaa gtaaacaacc 1920
   tttttgcatt ctcattcaac aaaaaaatca aagaaagtga agtcaaaaaa gtcaaagccc 1980
   tcataagaca taagtataaa gggaaagctt atgagattca gcttagctct ggtagaaaaa 2040
   ttaacataac tgctggccat agtctgttta cagttagaaa tggagaaata aaggaagttt 2100
   ctggagatgg gataaaagaa ggtgacctta ttgtagcacc aaagaaaatt aaactcaatg 2160
   aaaaaggggt aagcataaac attcccgagt taatctcaga tctttccgag gaagaaacag 2220
   ccgacattgt gatgacgatt tcagccaagg gcagaaagaa cttctttaaa ggaatgctga 2280
   gaactttaag gtggatgttt ggagaagaaa atagaaggat aagaacattt aatcgctatt 2340
   tgttccatct cgaaaaacta ggccttatca aactactgcc ccgcggatat gaagttactg 2400
   actgggagag attaaagaaa tataaacaac tttacgagaa gcttgctgga agcgttaagt 2460
   acaacggaaa caagagagag tatttagtaa tgttcaacga gatcaaggat tttatatctt 2520
   acttcccaca aaaagagctc gaagaatgga aaattggaac tctcaatggc tttagaacga 2580
   attgtattct caaagtcgat gaggattttg ggaagctcct aggttactat gttagtgagg 2640
   gctatgcagg tgcacaaaaa aataaaactg gtggtatcag ttattcggtg aagctttaca 2700
   atgaggaccc taatgttctt gagagcatga aaaatgttgc agaaaaattc tttggcaagg 2760
   ttagagttga cagaaattgc gtaagtatat caaagaagat ggcatactta gttatgaaat 2820
   gcctctgtgg agcattagcc gaaaacaaga gaattccttc tgttatactc acctctcccg 2880
   aaccggtacg gtggtcattt ttagaggcgt attttacagg cgatggagat atacatccat 2940
   caaaaaggtt taggctctca acaaaaagcg agctccttgc aaatcagctt gtgttcttgc 3000
   tgaactcttt gggaatatcc tctgtaaaga taggctttga cagtggggtc tatagagtgt 3060
   atataaatga agacctgcaa tttccacaaa cgtctaggga gaaaaacaca tactactcta 3120
   acttaattcc caaagagatc cttagggacg tgtttggaaa agagttccaa aagaacatga 3180
   cgttcaagaa atttaaagag cttgttgact ctggaaaact taacagggag aaagccaagc 3240
   tcttggagtt cttcattaat ggagatattg tccttgacag agtcaaaagt gttaaagaaa 3300
   aggactatga agggtatgtc tatgacctaa gcgttgagga taacgagaac tttcttgttg 3360
   gttttggttt gctctatgct cacaacagct attacggcta tatggggtat cctaaggcaa 3420
   gatggtactc gaaggaatgt gctgaaagcg ttaccgcatg ggggagacac tacatagaga 3480
   tgacgataag agaaatagag gaaaagttcg gctttaaggt tctttatgcg gacagtgtct 3540
   caggagaaag tgagatcata ataaggcaaa acggaaagat tagatttgtg aaaataaagg 3600
   atcttttctc taaggtggac tacagcattg gcgaaaaaga atactgcatt ctcgaaggtg 3660
   ttgaagcact aactctggac gatgacggaa agcttgtctg gaagcccgtc ccctacgtga 3720
   tgaggcacag agcgaataaa agaatgttcc gcatctggct gaccaacagc tggtatatag 3780
   atgttactga ggatcattct ctcataggct atctaaacac gtcaaaaacg aaaactgcca 3840
   aaaaaatcgg ggaaagacta aaggaagtaa agccttttga attaggcaaa gcagtaaaat 3900
   cgctcatatg cccaaatgca ccgttaaagg atgagaatac caaaactagc gaaatagcag 3960
   taaaattctg ggagctcgta ggattgattg taggagatgg aaactggggt ggagattctc 4020
   gttgggcaga gtattatctt ggactttcaa caggcaaaga tgcagaagag ataaagcaaa 4080
   aacttctgga acccctaaaa acttatggag taatctcaaa ctattaccca aaaaacgaga 4140
   aaggggactt caacatcttg gcaaagagcc ttgtaaagtt tatgaaaagg cactttaagg 4200
   acgaaaaagg aagacgaaaa attccagagt tcatgtatga gcttccggtt acttacatag 4260
   aggcatttct acgaggactg ttttcagctg atggtactgt aactatcagg aagggagttc 4320
   cagagatcag gctaacaaac attgatgctg actttctaag ggaagtaagg aagcttctgt 4380
   ggattgttgg aatttcaaat tcaatatttg ctgagactac tccaaatcgc tacaatggtg 4440
   tttctactgg aacctactca aagcatctaa ggatcaaaaa taagtggcgt tttgctgaaa 4500
   ggataggctt tttaatcgag agaaagcaga agagactttt agaacattta aaatcagcga 4560
   gggtaaaaag gaataccata gattttggct ttgatcttgt gcatgtgaaa aaagtcgaag 4620
   agataccata cgagggttac gtttatgaca ttgaagtcga agagacgcat aggttctttg 4680
   caaacaacat cctggtacac aatactgacg gcttttatgc cacaataccc ggggaaaagc 4740
   ctgaactcat taaaaagaaa gccaaggaat tcctaaacta cataaactcc aaacttccag 4800
   gtctgcttga gcttgagtat gagggctttt acttgagagg attctttgtt acaaaaaagc 4860
   gctatgcagt catagatgaa gagggcagga taacaacaag gggcttggaa gtagtaagga 4920
   gagattggag tgagatagct aaggagactc aggcaaaggt tttagaggct atacttaaag 4980
   agggaagtgt tgaaaaagct gtagaagttg ttagagatgt tgtagagaaa atagcaaaat 5040
   acagggttcc acttgaaaag cttgttatcc atgagcagat taccagggat ttaaaggact 5100
   acaaagccat tggccctcat gtcgcgatag caaaaagact tgccgcaaga gggataaaag 5160
   tgaaaccggg cacaataata agctatatcg ttctcaaagg gagcggaaag ataagcgata 5220
   gggtaatttt acttacagaa tacgatccta gaaaacacaa gtacgatccg gactactaca 5280
   tagaaaacca agttttgccg gcagtactta ggatactcga agcgtttgga tacagaaagg 5340
   aggatttaag gtatcaaagc tcaaaacaaa ccggcttaga tgcatggctc aagaggtagc 5400
   tctgttgctt tttagtccaa gtttctccgc gagtctctct atctctcttt tgtattctgc 5460
   tatgtggttt tcattcacta ttaagtagtc cgccaaagcc ataacgcttc caattccaaa 5520
   cttgagctct ttccagtctc tggcctcaaa ttcactccat gtttttggat cgtcgcttct 5580
   ccctcttctg ctaagcctct cgaatctttt tcttggcgaa gagtgtacag ctatgatgat 5640
   tatctcttcc tctggaaacg catctttaaa cgtctgaatt tcatctagag acctcactcc 5700
   gtcgattata actgccttgt acttctttag tagttctttt acctttggga tcgttaattt 5760
   tgccacggca ttgtccccaa gctcctgcct aagctgaatg ctcacactgt tcataccttc 5820
   gggagttctt gggatcc 5837

<210> 2
<211> 4707
<212> DNA
<213> unknown

<220>
<223> Deep Vent DNA polymerase gene of thermophilic archaebacteria,
Pyrococcus species

<220>
<221> CDS
<222> (363)..(4298)

<400> 2
ggatccctct ctttttggta accccatacg tcattccctc aaccaaaact tcagcatcgt 60

tgcagtggtc agtgtgtctg tgggagatga agaggacgtc gatttttctg gggtctatct 120

tgtatctcca cattctaact aacgctccag gcccaggatc aacgtagatg tttttgctcg 180

ccttaatgaa gaagccacca gtggctcttg cctgcgttat cgtgacgaac cttccaccac 240

cgccaccgag aaaagttatc tctatcatct cacacctccc ccataacatc acctgctcaa 300

tttttaagcg ttcttaaagg cttaaatacg tgaatttagc gtaaattatt gagggattaa 360

gt atg ata ctt gac gct gac tac atc acc gag gat ggg aag ccg att 407
Met Ile Leu Asp Ala Asp Tyr Ile Thr Glu Asp Gly Lys Pro Ile
1               5                   10                  15

ata agg att ttc aag aaa gaa aac ggc gag ttt aag gtt gag tac gac 455
Ile Arg Ile Phe Lys Lys Glu Asn Gly Glu Phe Lys Val Glu Tyr Asp
                20                  25                  30

aga aac ttt aga cct tac att tac gct ctc ctc aaa gat gac tcg cag 503
Arg Asn Phe Arg Pro Tyr Ile Tyr Ala Leu Leu Lys Asp Asp Ser Gln
            35                  40                  45

att gat gag gtt agg aag ata acc gcc gag agg cat ggg aag ata gtg 551
Ile Asp Glu Val Arg Lys Ile Thr Ala Glu Arg His Gly Lys Ile Val
        50                  55                  60

aga att ata gat gcc gaa aag gta agg aag aag ttc ctg ggg agg ccg 599
Arg Ile Ile Asp Ala Glu Lys Val Arg Lys Lys Phe Leu Gly Arg Pro
    65                  70                  75

att gag gta tgg agg ctg tac ttt gaa cac cct cag gac gtt ccc gca 647
Ile Glu Val Trp Arg Leu Tyr Phe Glu His Pro Gln Asp Val Pro Ala
80                  85                  90                  95

ata agg gat aag ata aga gag cat tcc gca gtt att gac atc ttt gag 695
Ile Arg Asp Lys Ile Arg Glu His Ser Ala Val Ile Asp Ile Phe Glu
                100                 105                 110

tac gac att ccg ttc gcg aag agg tac cta ata gac aaa ggc cta att 743
Tyr Asp Ile Pro Phe Ala Lys Arg Tyr Leu Ile Asp Lys Gly Leu Ile
            115                 120                 125

cca atg gaa ggc gat gaa gag ctc aag ttg ctc gca ttt gac ata gaa 791
Pro Met Glu Gly Asp Glu Glu Leu Lys Leu Leu Ala Phe Asp Ile Glu
        130                 135                 140

acc ctc tat cac gaa ggg gag gag ttc gcg aag ggg ccc att ata atg 839
Thr Leu Tyr His Glu Gly Glu Glu Phe Ala Lys Gly Pro Ile Ile Met
    145                 150                 155

ata agc tat gct gat gag gaa gaa gcc aaa gtc ata acg tgg aaa aag 887
Ile Ser Tyr Ala Asp Glu Glu Glu Ala Lys Val Ile Thr Trp Lys Lys
160                 165                 170                 175

atc gat ctc ccg tac gtc gag gta gtt tcc agc gag agg gag atg ata 935
Ile Asp Leu Pro Tyr Val Glu Val Val Ser Ser Glu Arg Glu Met Ile
                180                 185                 190

aag cgg ttc ctc aag gtg ata agg gag aaa gat ccc gat gtt ata att 983
Lys Arg Phe Leu Lys Val Ile Arg Glu Lys Asp Pro Asp Val Ile Ile
            195                 200                 205

acc tac aac ggc gat tct ttc gac ctt ccc tat cta gtt aag agg gcc 1031
Thr Tyr Asn Gly Asp Ser Phe Asp Leu Pro Tyr Leu Val Lys Arg Ala
        210                 215                 220

gaa aag ctc ggg ata aag cta ccc ctg gga agg gac ggt agt gag cca 1079

Glu Lys Leu Gly Ile Lys Leu Pro Leu Gly Arg Asp Gly Ser Glu Pro
    225                 230                 235

aag atg cag agg ctt ggg gat atg aca gcg gtg gag ata aag gga agg      1127
Lys Met Gln Arg Leu Gly Asp Met Thr Ala Val Glu Ile Lys Gly Arg
240                 245                 250                 255

ata cac ttt gac ctc tac cac gtg att agg aga acg ata aac ctc cca      1175
Ile His Phe Asp Leu Tyr His Val Ile Arg Arg Thr Ile Asn Leu Pro
                260                 265                 270

aca tac acc ctc gag gca gtt tat gag gca atc ttc gga aag cca aag      1223
Thr Tyr Thr Leu Glu Ala Val Tyr Glu Ala Ile Phe Gly Lys Pro Lys
            275                 280                 285

gag aaa gtt tac gct cac gag ata gct gag gcc tgg gag act gga aag      1271
Glu Lys Val Tyr Ala His Glu Ile Ala Glu Ala Trp Glu Thr Gly Lys
        290                 295                 300

gga ctg gag aga gtt gca aag tat tca atg gag gat gca aag gta acg      1319
Gly Leu Glu Arg Val Ala Lys Tyr Ser Met Glu Asp Ala Lys Val Thr
    305                 310                 315

tac gag ctc ggt agg gag ttc ttc cca atg gag gcc cag ctt tca agg      1367
Tyr Glu Leu Gly Arg Glu Phe Phe Pro Met Glu Ala Gln Leu Ser Arg
320                 325                 330                 335

tta gtc ggc cag ccc ctg tgg gat gtt tct agg tct tca act ggc aac      1415
Leu Val Gly Gln Pro Leu Trp Asp Val Ser Arg Ser Ser Thr Gly Asn
                340                 345                 350

ttg gtg gag tgg tac ctc ctc agg aag gcc tac gag agg aat gaa ttg      1463
Leu Val Glu Trp Tyr Leu Leu Arg Lys Ala Tyr Glu Arg Asn Glu Leu
            355                 360                 365

gct cca aac aag ccg gat gag agg gag tac gag aga agg cta agg gag      1511
Ala Pro Asn Lys Pro Asp Glu Arg Glu Tyr Glu Arg Arg Leu Arg Glu
        370                 375                 380

agc tac gct ggg gga tac gtt aag gag ccg gag aaa ggg ctc tgg gag      1559
Ser Tyr Ala Gly Gly Tyr Val Lys Glu Pro Glu Lys Gly Leu Trp Glu
    385                 390                 395

ggg tta gtt tcc cta gat ttc agg agc ctg tac ccc tcg ata ata atc      1607
Gly Leu Val Ser Leu Asp Phe Arg Ser Leu Tyr Pro Ser Ile Ile Ile
400                 405                 410                 415

acc cat aac gtc tca ccg gat acg ctg aac agg gaa ggg tgt agg gaa      1655
Thr His Asn Val Ser Pro Asp Thr Leu Asn Arg Glu Gly Cys Arg Glu
                420                 425                 430

tac gat gtc gcc cca gag gtt ggg cac aag ttc tgc aag gac ttc ccg      1703
Tyr Asp Val Ala Pro Glu Val Gly His Lys Phe Cys Lys Asp Phe Pro
            435                 440                 445

ggg ttt atc ccc agc ctg ctc aag agg tta ttg gat gaa agg caa gaa      1751
Gly Phe Ile Pro Ser Leu Leu Lys Arg Leu Leu Asp Glu Arg Gln Glu
        450                 455                 460

ata aaa agg aag atg aaa gct tct aaa gac cca atc gag aag aag atg      1799
Ile Lys Arg Lys Met Lys Ala Ser Lys Asp Pro Ile Glu Lys Lys Met
    465                 470                 475

ctt gat tac agg caa cgg gca atc aaa atc ctg gca aac agc att tta 1847
Leu Asp Tyr Arg Gln Arg Ala Ile Lys Ile Leu Ala Asn Ser Ile Leu
480                 485                 490                 495

ccg gaa gaa tgg gtt cca cta att aaa aac ggt aaa gtt aag ata ttc 1895
Pro Glu Glu Trp Val Pro Leu Ile Lys Asn Gly Lys Val Lys Ile Phe
                500                 505                 510

cgc att ggg gac ttc gtt gat gga ctt atg aag gcg aac caa gga aaa 1943
Arg Ile Gly Asp Phe Val Asp Gly Leu Met Lys Ala Asn Gln Gly Lys
            515                 520                 525

gtg aag aaa acg ggg gat aca gaa gtt tta gaa gtt gca gga att cat 1991
Val Lys Lys Thr Gly Asp Thr Glu Val Leu Glu Val Ala Gly Ile His
        530                 535                 540

gcg ttt tcc ttt gac agg aag tcc aag aag gcc cgt gta atg gca gtg 2039
Ala Phe Ser Phe Asp Arg Lys Ser Lys Lys Ala Arg Val Met Ala Val
    545                 550                 555

aaa gcc gtg ata aga cac cgt tat tcc gga aat gtt tat aga ata gtc 2087
Lys Ala Val Ile Arg His Arg Tyr Ser Gly Asn Val Tyr Arg Ile Val
560                 565                 570                 575

tta aac tct ggt aga aaa ata aca ata aca gaa ggg cat agc cta ttt 2135
Leu Asn Ser Gly Arg Lys Ile Thr Ile Thr Glu Gly His Ser Leu Phe
                580                 585                 590

gtc tat agg aac ggg gat ctc gtt gag gca act ggg gag gat gtc aaa 2183
Val Tyr Arg Asn Gly Asp Leu Val Glu Ala Thr Gly Glu Asp Val Lys
            595                 600                 605

att ggg gat ctt ctt gca gtt cca aga tca gta aac cta cca gag aaa 2231
Ile Gly Asp Leu Leu Ala Val Pro Arg Ser Val Asn Leu Pro Glu Lys
        610                 615                 620

agg gaa cgc ttg aat att gtt gaa ctt ctt ctg aat ctc tca ccg gaa 2279
Arg Glu Arg Leu Asn Ile Val Glu Leu Leu Leu Asn Leu Ser Pro Glu
    625                 630                 635

gag aca gaa gat ata ata ctt acg att cca gtt aaa ggc aga aag aac 2327
Glu Thr Glu Asp Ile Ile Leu Thr Ile Pro Val Lys Gly Arg Lys Asn
640                 645                 650                 655

ttc ttc aag gga atg ttg aga aca tta cgt tgg att ttt ggt gag gaa 2375
Phe Phe Lys Gly Met Leu Arg Thr Leu Arg Trp Ile Phe Gly Glu Glu
                660                 665                 670

aag aga gta agg aca gcg agc cgc tat cta aga cac ctt gaa aat ctc 2423
Lys Arg Val Arg Thr Ala Ser Arg Tyr Leu Arg His Leu Glu Asn Leu
            675                 680                 685

gga tac ata agg ttg agg aaa att gga tac gac atc att gat aag gag 2471
Gly Tyr Ile Arg Leu Arg Lys Ile Gly Tyr Asp Ile Ile Asp Lys Glu
        690                 695                 700

ggg ctt gag aaa tat aga acg ttg tac gag aaa ctt gtt gat gtt gtc 2519
Gly Leu Glu Lys Tyr Arg Thr Leu Tyr Glu Lys Leu Val Asp Val Val
    705                 710                 715

cgc tat aat ggc aac aag aga gag tat tta gtt gaa ttt aat gct gtc 2567
Arg Tyr Asn Gly Asn Lys Arg Glu Tyr Leu Val Glu Phe Asn Ala Val
720                 725                 730                 735

cgg gac gtt atc tca cta atg cca gag gaa gaa ctg aag gaa tgg cgt     2615
Arg Asp Val Ile Ser Leu Met Pro Glu Glu Glu Leu Lys Glu Trp Arg
            740                 745                 750

att gga act aga aat gga ttc aga atg ggt acg ttc gta gat att gat     2663
Ile Gly Thr Arg Asn Gly Phe Arg Met Gly Thr Phe Val Asp Ile Asp
        755                 760                 765

gaa gat ttt gcc aag ctt ctt ggc tac tat gtg agc gag gga agt gcg     2711
Glu Asp Phe Ala Lys Leu Leu Gly Tyr Tyr Val Ser Glu Gly Ser Ala
    770                 775                 780

agg aag tgg aag aat caa act gga ggt tgg agt tac act gtg aga ttg     2759
Arg Lys Trp Lys Asn Gln Thr Gly Gly Trp Ser Tyr Thr Val Arg Leu
785                 790                 795

tac aac gag aac gat gaa gtt ctt gac gac atg gaa cac tta gcc aag     2807
Tyr Asn Glu Asn Asp Glu Val Leu Asp Asp Met Glu His Leu Ala Lys
800                 805                 810                 815

aag ttt ttt ggg aaa gtc aaa cgt gga aag aac tat gtt gag ata cca     2855
Lys Phe Phe Gly Lys Val Lys Arg Gly Lys Asn Tyr Val Glu Ile Pro
                820                 825                 830

aag aaa atg gct tat atc atc ttt gag agc ctt tgt ggg act ttg gca     2903
Lys Lys Met Ala Tyr Ile Ile Phe Glu Ser Leu Cys Gly Thr Leu Ala
            835                 840                 845

gaa aac aaa agg gtt cct gag gta atc ttt acc tca tca aag ggc gtt     2951
Glu Asn Lys Arg Val Pro Glu Val Ile Phe Thr Ser Ser Lys Gly Val
        850                 855                 860

aga tgg gcc ttc ctt gag ggt tat ttc atc ggc gat ggc gat gtt cac     2999
Arg Trp Ala Phe Leu Glu Gly Tyr Phe Ile Gly Asp Gly Asp Val His
    865                 870                 875

cca agc aag agg gtt cgc cta tca acg aag agc gag ctt tta gta aat     3047
Pro Ser Lys Arg Val Arg Leu Ser Thr Lys Ser Glu Leu Leu Val Asn
880                 885                 890                 895

ggc ctt gtt ctc cta ctt aac tcc ctt gga gta tct gcc att aag ctt     3095
Gly Leu Val Leu Leu Leu Asn Ser Leu Gly Val Ser Ala Ile Lys Leu
                900                 905                 910

gga tac gat agc gga gtc tac agg gtt tat gta aac gag gaa ctt aag     3143
Gly Tyr Asp Ser Gly Val Tyr Arg Val Tyr Val Asn Glu Glu Leu Lys
            915                 920                 925

ttt acg gaa tac aga aag aaa aag aat gta tat cac tct cac att gtt     3191
Phe Thr Glu Tyr Arg Lys Lys Lys Asn Val Tyr His Ser His Ile Val
        930                 935                 940

cca aag gat att ctc aaa gaa act ttt ggt aag gtc ttc cag aaa aat     3239
Pro Lys Asp Ile Leu Lys Glu Thr Phe Gly Lys Val Phe Gln Lys Asn
    945                 950                 955

ata agt tac aag aaa ttt aga gag ctt gta gaa aat gga aaa ctt gac     3287
Ile Ser Tyr Lys Lys Phe Arg Glu Leu Val Glu Asn Gly Lys Leu Asp
960                 965                 970                 975

agg gag aaa gcc aaa cgc att gag tgg tta ctt aac gga gat ata gtc     3335
Arg Glu Lys Ala Lys Arg Ile Glu Trp Leu Leu Asn Gly Asp Ile Val

                980                 985                 990

cta gat aga gtc gta gag att aag aga gag tac tat gat ggt tac gtt 3383
Leu Asp Arg Val Val Glu Ile Lys Arg Glu Tyr Tyr Asp Gly Tyr Val
            995                 1000                1005

tac gat cta agt gtc gat gaa gat gag aat ttc ctt gct ggc ttt 3428
Tyr Asp Leu Ser Val Asp Glu Asp Glu Asn Phe Leu Ala Gly Phe
        1010                1015                1020

gga ttc ctc tat gca cat aat agc tat tat ggg tat tat ggg tac 3473
Gly Phe Leu Tyr Ala His Asn Ser Tyr Tyr Gly Tyr Tyr Gly Tyr
        1025                1030                1035

gca aaa gcc cgt tgg tac tgt aag gag tgc gca gag agc gtt acg 3518
Ala Lys Ala Arg Trp Tyr Cys Lys Glu Cys Ala Glu Ser Val Thr
        1040                1045                1050

gcc tgg ggg agg gaa tat ata gag ttc gta agg aag gaa ctg gag 3563
Ala Trp Gly Arg Glu Tyr Ile Glu Phe Val Arg Lys Glu Leu Glu
        1055                1060                1065

gaa aag ttc ggg ttc aaa gtc tta tac ata gac aca gat gga ctc 3608
Glu Lys Phe Gly Phe Lys Val Leu Tyr Ile Asp Thr Asp Gly Leu
        1070                1075                1080

tac gcc aca att cct ggg gca aaa ccc gag gag ata aag aag aaa 3653
Tyr Ala Thr Ile Pro Gly Ala Lys Pro Glu Glu Ile Lys Lys Lys
        1085                1090                1095

gcc cta gag ttc gta gat tat ata aac gcc aag ctc cca ggg ctg 3698
Ala Leu Glu Phe Val Asp Tyr Ile Asn Ala Lys Leu Pro Gly Leu
        1100                1105                1110

ttg gag ctt gag tac gag ggc ttc tac gtg aga ggg ttc ttc gtg 3743
Leu Glu Leu Glu Tyr Glu Gly Phe Tyr Val Arg Gly Phe Phe Val
        1115                1120                1125

acg aag aag aag tat gcg ttg ata gat gag gaa ggg aag ata atc 3788
Thr Lys Lys Lys Tyr Ala Leu Ile Asp Glu Glu Gly Lys Ile Ile
        1130                1135                1140

act agg ggg ctt gaa ata gtc agg agg gac tgg agc gaa ata gcc 3833
Thr Arg Gly Leu Glu Ile Val Arg Arg Asp Trp Ser Glu Ile Ala
        1145                1150                1155

aaa gaa acc caa gca aaa gtc cta gag gct atc cta aag cat ggc 3878
Lys Glu Thr Gln Ala Lys Val Leu Glu Ala Ile Leu Lys His Gly
        1160                1165                1170

aac gtt gag gag gca gta aag ata gtt aag gag gta act gaa aag 3923
Asn Val Glu Glu Ala Val Lys Ile Val Lys Glu Val Thr Glu Lys
        1175                1180                1185

ctg agc aag tac gaa ata cct cca gaa aag cta gtt att tac gag 3968
Leu Ser Lys Tyr Glu Ile Pro Pro Glu Lys Leu Val Ile Tyr Glu
        1190                1195                1200

cag atc acg agg ccc ctt cac gag tac aag gct ata ggt ccg cac 4013
Gln Ile Thr Arg Pro Leu His Glu Tyr Lys Ala Ile Gly Pro His
        1205                1210                1215

gtt gcc gtg gca aaa agg tta gcc gct aga gga gta aag gtg agg 4058

Val Ala Val Ala Lys Arg Leu Ala Ala Arg Gly Val Lys Val Arg
        1220                1225                1230

cct ggc atg gtg ata ggg tac ata gtg ctg agg gga gac ggg cca      4103
Pro Gly Met Val Ile Gly Tyr Ile Val Leu Arg Gly Asp Gly Pro
        1235                1240                1245

ata agc aag agg gct atc ctt gca gag gag ttc gat ctc agg aag      4148
Ile Ser Lys Arg Ala Ile Leu Ala Glu Glu Phe Asp Leu Arg Lys
        1250                1255                1260

cat aag tat gac gct gag tat tac ata gaa aat cag gtt tta cct      4193
His Lys Tyr Asp Ala Glu Tyr Tyr Ile Glu Asn Gln Val Leu Pro
        1265                1270                1275

gcc gtt ctt aga ata tta gag gcc ttt ggg tac agg aaa gaa gac      4238
Ala Val Leu Arg Ile Leu Glu Ala Phe Gly Tyr Arg Lys Glu Asp
        1280                1285                1290

ctc agg tgg cag aag act aaa cag aca ggt ctt acg gca tgg ctt      4283
Leu Arg Trp Gln Lys Thr Lys Gln Thr Gly Leu Thr Ala Trp Leu
        1295                1300                1305

aac atc aag aag aag taatgtttat gtactcgtaa tgcgagtatt aagtgggtga      4338
Asn Ile Lys Lys Lys
        1310

tgagatggca gtattgagca taaggattcc ggatgatcta aaagagaaga tgaaggagtt      4398

tgacataaac tggagtgagg agatcaggaa gttcataaaa gagaggatag agtatgagga      4458

aaggaagaga acccttgaga aagctctaga acttctaaag aatactccag gatcagtcga      4518

gagaggattt tcagcaaggg cagtgaggga ggatcgtgat agtcattgat gcatcaatcc      4578

tagctaaaat aattctaaaa gaagagggct gggaacagat aactcttaca ccgagcacga      4638

taactttgga ctatgctttt gttgaatgta caaacgcaat atggaaggct gtcaggcgga      4698

acaggatcc                                                              4707

<210>  3
<211>  23
<212>  DNA
<213>  unknown

<220>
<223>  primer

<400>  3
agtgtctccg gagaaagtga gat                                              23

<210>  4
<211>  22
<212>  DNA
<213>  unknown

<220>
<223>  primer

<400>  4

agtattgtgt accaggatgt tg                                                22

<210>  5
<211>  24
<212>  DNA
<213>  unknown

<220>
<223>  primer

<400>  5
agcattttac cggaagaatg ggtt                                              24

<210>  6
<211>  25
<212>  DNA
<213>  unknown

<220>
<223>  primer

<400>  6
gctattatgt gcatagagga atcca                                             25

<210>  7
<211>  24
<212>  DNA
<213>  unknown

<220>
<223>  primer

<400>  7
agggtcgaca gatttgatcc agcg                                              24

<210>  8
<211>  20
<212>  DNA
<213>  unknown

<220>
<223>  primer

<400>  8
gagaactttg ttcgtacctg                                                   20

<210>  9
<211>  21
<212>  DNA
<213>  unknown

<220>
<223>  primer

<400>  9
ggtattattt cttctaaagc a                                                 21

<210> 10
<211> 20
<212> DNA
<213> unknown

<220>
<223> primer

<400> 10
gttgtttgtt ggttttacca 20


<210> 11
<211> 20
<212> DNA
<213> unknown

<220>
<223> primer

<400> 11
atggcaaatg ctgtatggat 20


<210> 12
<211> 23
<212> DNA
<213> unknown

<220>
<223> primer

<400> 12
agtgtctccg gagaaagtga gat 23


<210> 13
<211> 26
<212> DNA
<213> unknown

<220>
<223> primer

<400> 13
attgtgtact agtatgttgt ttgcaa 26


<210> 14
<211> 37
<212> DNA
<213> unknown

<220>
<223> primer

<400> 14
gcctccggag acactatcgc caaaatcacc gccgtaa 37


<210> 15
<211> 38
<212> DNA

<213>  unknown

<220>
<223>  primer

<400>  15
gccactagta cacaatacgc cgaacgatcg ccagttct                              38


<210>  16
<211>  26
<212>  DNA
<213>  unknown

<220>
<223>  primer

<400>  16
ccttctagac cggtgcagta tgaagg                                           26


<210>  17
<211>  36
<212>  DNA
<213>  unknown

<220>
<223>  primer

<400>  17
gccgtcgacc ctagtgtctc aggagaaagt gagatc                                36


<210>  18
<211>  33
<212>  DNA
<213>  unknown

<220>
<223>  primer

<400>  18
gcctctagaa ttgtgtacca ggatgttgtt tgc                                   33


<210>  19
<211>  23
<212>  DNA
<213>  unknown

<220>
<223>  oligonucleotide

<400>  19
gcaaagaacc ggtgcgtctc ttc                                              23


<210>  20
<211>  20
<212>  DNA
<213>  unknown

<220>

<223>  oligonucleotide

<400>  20
agcaacagag ttacctcttg                                                    20


<210>  21
<211>  33
<212>  DNA
<213>  unknown

<220>
<223>  oligonucleotide

<400>  21
cagtttccag ctcctacaat gagacctacg agc                                     33


<210>  22
<211>  20
<212>  DNA
<213>  unknown

<220>
<223>  oligonucleotide

<400>  22
gtagtgtcga ccccatgcgg                                                    20


<210>  23
<211>  28
<212>  DNA
<213>  unknown

<220>
<223>  oligonucleotide

<400>  23
cgttttgcct gattattatc tcactttc                                           28


<210>  24
<211>  22
<212>  DNA
<213>  unknown

<220>
<223>  oligonucleotide

<400>  24
gtccaccttc gaaaaagat cc                                                  22


<210>  25
<211>  21
<212>  DNA
<213>  unknown

<220>
<223>  oligonucleotide

<400>  25

ccgcataaag gaccttaaag c                                                21

<210>  26
<211>  23
<212>  DNA
<213>  unknown

<220>
<223>  oligonucleotide

<400>  26
gaggaagaga tcatcatcat agc                                              23

<210>  27
<211>  40
<212>  DNA
<213>  unknown

<220>
<223>  oligonucleotide

<400>  27
gtccttcgtg cggacagtgt ctcaggagaa agtgagataa                            40

<210>  28
<211>  40
<212>  DNA
<213>  unknown

<220>
<223>  oligonucleotide

<400>  28
gtcctttatg cggactaggt ctcaggagaa agtgagataa                            40

<210>  29
<211>  61
<212>  DNA
<213>  unknown

<220>
<223>  oligonucleotide

<400>  29
ccggttcttt gcaaacaaca tcctggtaca caattaagac ggcttttatg ccacaatacc      60

c                                                                      61

<210>  30
<211>  22
<212>  PRT
<213>  unknown

<220>
<223>  pyrococcus sp. IVPS 1

<400>  30

Ile Lys Ile Leu Ala Asn Ser Ile Leu Pro Glu Glu Trp Val Pro Leu
1               5                   10                  15

Ile Lys Asn Gly Lys Val
            20

<210>  31
<211>  22
<212>  PRT
<213>  unknown

<220>
<223>  T.litoralis IVPS 1

<400>  31

Ile Lys Leu Leu Ala Asn Ser Ile Leu Pro Asn Glu Trp Leu Pro Ile
1               5                   10                  15

Ile Glu Asn Gly Glu Ile
            20

<210>  32
<211>  22
<212>  PRT
<213>  unknown

<220>
<223>  T. litoralis IVPS2

<400>  32

Lys Val Leu Tyr Ala Asp Ser Val Ser Gly Glu Ser Glu Ile Ile Ile
1               5                   10                  15

Arg Gln Asn Gly Lys Ile
            20

<210>  33
<211>  22
<212>  PRT
<213>  unknown

<220>
<223>  yeast TFP1

<400>  33

Ala Ile Leu Tyr Val Gly Cys Phe Ala Lys Gly Thr Asn Val Leu Met
1               5                   10                  15

Ala Asp Gly Ser Ile Glu
            20

<210>  34

<211>   22
<212>   PRT
<213>   unknown

<220>
<223>   M. tuberculosis recA

<400>   34

Lys Val Val Lys Asn Lys Cys Leu Ala Glu Gly Thr Arg Ile Arg Asp
1               5                   10                  15

Pro Val Thr Gly Thr Thr
            20

<210>   35
<211>   23
<212>   PRT
<213>   unknown

<220>
<223>   Pyrococcus sp. IVPS1

<400>   35

Glu Asx Gly Lys Ala Gly Phe Gly Phe Leu Tyr Ala His Asn Ser Tyr
1               5                   10                  15

Tyr Gly Tyr Tyr Gly Tyr Ala
            20

<210>   36
<211>   23
<212>   PRT
<213>   unknown

<220>
<223>   T. litoralis IVPS1

<400>   36

Glu Asn Phe Leu Val Gly Phe Gly Leu Leu Tyr Ala His Asn Ser Tyr
1               5                   10                  15

Tyr Gly Tyr Met Gly Tyr Pro
            20

<210>   37
<211>   23
<212>   PRT
<213>   unknown

<220>
<223>   T. litoralis IVPS2

<400>   37

Glu Thr His Arg Phe Phe Ala Asn Asn Ile Leu Val His Asn Thr Asp
1               5                   10                  15

Gly Phe Tyr Ala Thr Ile Pro
            20

<210>  38
<211>  23
<212>  PRT
<213>  unknown

<220>
<223>  Yeast TFP1

<400>  38

Asp His Gln Phe Leu Leu Ala Asn Gln Val Val Val His Asn Cys Gly
1               5                   10                  15

Glu Arg Gly Asn Glu Met Ala
            20

<210>  39
<211>  23
<212>  PRT
<213>  unknown

<220>
<223>  M. tuberculosis recA

<400>  39

Glu Leu His Thr Leu Val Ala Glu Gly Val Val Val His Asn Cys Ser
1               5                   10                  15

Pro Pro Phe Lys Gln Ala Glu
            20

<210>  40
<211>  25
<212>  DNA
<213>  unknown

<220>
<223>  primer

<400>  40
gcaattatgt gcatagagga atcca                                          25

<210>  41
<211>  25
<212>  DNA
<213>  unknown

<220>
<223>  primer

<400>  41
ggtattatgt gcatagagga atcca                                          25


<210>  42
<211>  25
<212>  DNA
<213>  unknown

<220>
<223>  primer

<400>  42
attatgtgca tagaggaatc caaag                                          25


<210>  43
<211>  42
<212>  DNA
<213>  unknown

<220>
<223>  primer

<400>  43
ggtacccgtc gtgctagcat tttaccggaa gaatgggtac ca                       42


<210>  44
<211>  27
<212>  DNA
<213>  unknown

<220>
<223>  primer

<400>  44
cccgctatta tgtgcataga gggatcc                                        27


<210>  45
<211>  4
<212>  PRT
<213>  unknown

<220>
<223>  common subset of IVPSs


<220>
<221>  MISC_FEATURE
<222>  (1)..(1)
<223>  X= A or V

<220>
<221>  MISC_FEATURE
<222>  (4)..(4)
<223>  X= S or C or T

<400>  45

Xaa His Asn Xaa

1

<210> 46
<211> 29
<212> DNA
<213> unknown

<220>
<223> oligomer

<400> 46
gatccctcta tgcacataat tcaggcctc 29

<210> 47
<211> 29
<212> DNA
<213> unknown

<220>
<223> oligomer

<400> 47
aattgaggcc tgaattatgt gcatagagg 29

<210> 48
<211> 35
<212> DNA
<213> unknown

<220>
<223> oligomer

<400> 48
gctcgaggct agcattttac cggaagaatg ggtac 35

<210> 49
<211> 35
<212> DNA
<213> unknown

<220>
<223> oligomer

<400> 49
ccattcttcc ggtaaaatgc tagcctcgag cgtac 35

<210> 50
<211> 25
<212> DNA
<213> unknown

<220>
<223> oligomer

<400> 50
gatccctcta taagcataat tcagg 25

<210> 51
<211> 21
<212> DNA
<213> unknown

<220>
<223> oligomer

<400> 51
cctgaattat gcttatagag g 21


<210> 52
<211> 25
<212> DNA
<213> unknown

<220>
<223> oligomer

<400> 52
gatccctcta tgcactgaat tcagg 25


<210> 53
<211> 21
<212> DNA
<213> unknown

<220>
<223> oligomer

<400> 53
cctgaattca gtgcatagag g 21


<210> 54
<211> 32
<212> DNA
<213> unknown

<220>
<223> primer

<400> 54
gtcaggcctc tcagacagta cagctcgtac at 32


<210> 55
<211> 6
<212> DNA
<213> unknown

<220>
<223> StuI

<400> 55
aggcct 6


<210> 56
<211> 35
<212> DNA

<213>  unknown

<220>
<223>  primer

<400>  56
cccctgcagt taaaagtaat tgctttccaa ataag                                35


<210>  57
<211>  6
<212>  DNA
<213>  unknown

<220>
<223>  PstI

<400>  57
ctgcag                                                                 6


<210>  58
<211>  28
<212>  DNA
<213>  unknown

<220>
<223>  primer

<400>  58
ggaattccat atgaaaatcg aagaaggt                                        28


<210>  59
<211>  31
<212>  DNA
<213>  unknown

<220>
<223>  primer

<400>  59
cgggatcccg ttatagtgag ataacgtccc g                                    31


<210>  60
<211>  28
<212>  DNA
<213>  unknown

<220>
<223>  primer

<400>  60
ggaattccat atgccagagg aagaactg                                        28


<210>  61
<211>  34
<212>  DNA
<213>  unknown

<220>

<223>  primer

<400>  61
atagtttagc ggccgctcac gacgttgtaa aacg                                    34


<210>  62
<211>  33
<212>  DNA
<213>  unknown

<220>
<223>  oligomer

<400>  62
tcgaggctag caaattaccg gaagaatggg tac                                     33


<210>  63
<211>  25
<212>  DNA
<213>  unknown

<220>
<223>  oligomer

<400>  63
ccattcttcc ggtaatttgc tagcc                                              25


<210>  64
<211>  33
<212>  DNA
<213>  unknown

<220>
<223>  oligomer

<400>  64
tcgaggcttg cattttaccg gaagaatggg tac                                     33


<210>  65
<211>  25
<212>  DNA
<213>  unknown

<220>
<223>  oligomer

<400>  65
ccattcttcc ggtaaaatgc aagcc                                              25


<210>  66
<211>  34
<212>  DNA
<213>  unknown

<220>
<223>  oligomer

<400>  66

gatccctcta taagcataat attggcatgc agta 34

<210> 67
<211> 30
<212> DNA
<213> unknown

<220>
<223> oligomer

<400> 67
tactgcatgc caatattatg cttatagagg 30

<210> 68
<211> 29
<212> DNA
<213> unknown

<220>
<223> oligomer

<400> 68
gatccctcta tgcacataat taaggcatg 29

<210> 69
<211> 21
<212> DNA
<213> unknown

<220>
<223> oligomer

<400> 69
ccttaattat gtgcatagag g 21

<210> 70
<211> 33
<212> DNA
<213> unknown

<220>
<223> primer

<400> 70
gcgctcgagg ggtgctttgc caagggtacc aat 33

<210> 71
<211> 28
<212> DNA
<213> unknown

<220>
<223> primer

<400> 71
cctccgcaat tatggacgac aacctggt 28

<210> 72
<211> 24
<212> DNA
<213> unknown

<220>
<223> primer

<400> 72
gaatgcggaa ttcaggcctc cgca 24


<210> 73
<211> 39
<212> DNA
<213> unknown

<220>
<223> primer

<400> 73
atggacgaca acctgggatc caagcaaaaa ctgatgatc 39


<210> 74
<211> 35
<212> DNA
<213> unknown

<220>
<223> oligomer

<400> 74
gatcccaggt tgtcgtccat gcatgcggag gcctg 35


<210> 75
<211> 35
<212> DNA
<213> unknown

<220>
<223> oligomer

<400> 75
aattcaggcc tccgcatgca tggacgacaa cctgg 35


<210> 76
<211> 36
<212> DNA
<213> unknown

<220>
<223> oligomer

<400> 76
gatcccaggt tgtcgtccat gcatgcggtg gcctga 36


<210> 77
<211> 36
<212> DNA

<213>  unknown

<220>
<223>  oligomer

<400>  77
ccggtcaggc ctccgcatgc atggacgaca acctgg                                36